# EUROPEAN PATENT APPLICATION

(11) **EP 4 534 700 A2**
(43) Date of publication of application: **09.04.2025**
(21) Application number: 24214327.9
(22) Date of filing: 12.02.2021
(51) Int. Cl.: C12Q 1/689

(54) **REFERENCE LADDERS AND ADAPTORS**

(30) Priority: 13.02.2020 AU 2020900400; 13.02.2020 AU 2020900401
(62) Divisional of application: 21754464.2
(71) Applicant: Garvan Institute of Medical Research, Darlinghurst, NSW 2010 (AU)
(72) Inventor: MERCER, Timothy, Darlinghurst (AU)
(74) Representative: Mewburn Ellis LLP

(57) **Abstract**

The present disclosure generally relates to polynucleotides that are useful in calibrating methods of determining the identity and/or quantity of polynucleotides in a sample. In particular, the present disclosure relates to polunucleotides comprising calibration sequences which function as reference ladders or reference adaptors. The polynucleotides can be used to calibrate a wide variety of sequencing methods, including high throughput sequencing methods (for example, those referred to as next generation sequencing or NGS methods). The present disclosure also generally relates to the use of these polynucleotides in a wide variety of applications including, for example, in the calibration of a wide variety of sequencing methods.

## Description

### Cross-reference to Related Applications

The present application claims priority from Australian Provisional Patent Application No's 2020900400 and 2020900401 filed on 13 February 2020, the content of each of which is incorporated herein by reference in its entirety.

### Technical Field

The present disclosure generally relates to polynucleotides that are useful in calibrating methods of determining the identity and/or quantity of polynucleotides in a sample. The polynucleotides can be used to calibrate a wide variety of sequencing methods, including high throughput sequencing methods (for example, those referred to as next generation sequencing or NGS methods). The present disclosure also generally relates to the use of these polynucleotides in a wide variety of applications including, for example, in the calibration of a wide variety of sequencing methods.

### Background

NGS technologies (exemplified by services and products provided by companies such as Illumina, Oxford Nanopore, PacBio, Ion Torrent, Roche 454 Pyrosequencing (see, e.g., Bentley, D.R. *et al.*, 2008; Clarke, J. *et al.*, 2009; Ronaghi, M. *et al.*, 1998; Eid, J. *et al.*, 2009; Rothberg, J.M. *et al.*, 2011) and others) enable the high-throughput, massively parallel sequencing of nucleic acid molecules. These technologies have the capacity to determine the nucleotide base sequence of millions of RNA and DNA molecules within a single sample. Furthermore, the rate at which individual RNA or DNA sequences are determined is proportional to the relative abundance of that individual RNA or DNA sequence within the sample. Therefore, NGS can also be used to determine the quantity of one or more nucleic acid sequences within a sample.

NGS is widely used to determine the sequence and/or measure the quantities of nucleic acids found within samples taken from natural sources, such as animals, plants, microorganisms, or the diverse population of microbes within an environmental sample (Edwards, R.A. *et al.*, 2006). These uses include the determination of an organism's full genome sequence (see, e.g., Bentley, D.R. *et al.*, 2008), the determination of the sequence and abundance of messenger RNA present within a sample (see, e.g., Mortazavi, A. *et al.,* 2008), or the identification of genetic variants that are associated with disease, or the sequencing and measurement of a range of cellular features, such as epigenetic modifications (see, e.g., Bernstein, B.E. *et al.*, 2005), protein binding sites (see, e.g., Johnson, D.S., *et al.*, 2007), three-dimensional DNA structure (see, e.g., Lieberman-Aiden, E. *et al.*, 2009), and other features.

The millions of individual RNA or DNA sequences determined by NGS can be merged by *de novo* assembly into longer sequences (called contigs) or matched to a known reference sequence. *De novo* assembly of DNA sequences can be used to assemble an organism's genome; *de novo* assembly of RNA sequences can indicate gene sequence, length and isoforms. The matching or alignment of DNA sequences to a reference genome can identify the location of genetic differences or variation between individuals. The location of matches between DNA sequences and the reference genome can indicate locations of epigenetic features, such as histone modifications, or protein binding sites. Alignment of RNA sequences to a reference genome can indicate the existence of intron sequences that are excised during the process of gene splicing.

In some instances, during the operation of such sequencing methods, nucleic acids of known quantities or sequences, termed standards, have been added (or "spiked-in") to a natural sample of nucleic acids. The resultant combined mixture may then be analysed using a range of genetic technologies (such as NGS technologies), including microarray technologies, quantitative polymerase chain reaction methods, and others. The quantities or sequences of the sample nucleic acids can be compared to the known quantities or sequences of the added nucleic acid standards, in order to provide a reference scale that can be used to measure and determine the quantities or sequences of a natural sample of nucleic acids.

Previously used RNA and DNA standards have been derived from natural sources. For example, a DNA sequence extracted from the NA12878 cell line originally derived from a Caucasian female human has been extensively characterized and has been used to assess the performance of analytical tools to identify genetic variation (Zook, J.M. *et al.,* 2014). Ribonucleic-acid standards (known as ERCC Spike-Ins) containing sequences derived from the archaea *Methanocaldococcus jannaschii* were developed for microarrays and qRT-PCR technologies (Baker, S.C. *et al.,* 2005; Consortium, E.R.C., 2005) and have been used with RNA sequencing (Jiang, L. *et al.*, 2011). More recently, artificial calibration sequences have been devised in order to address one or more disadvantages resulting from the use of natural sequences as standards. Such artificial calibration sequences are described in International patent application number PCT/AU2015/050797, published as WO 2016/094947.

Adaptor (or adapter) sequences are typically known as DNA sequences that are used to prepare target polynucleotides for next-generation sequencing. The library preparation process during next generation sequencing generates DNA sequences that comprise the adaptor sequence upstream (i.e., in the 5' direction) and/or downstream (i.e., in the 3' direction) of the target DNA or RNA sequence. These generated DNA sequences are then sequenced using next-generation sequencing methods.

Despite these developments, the analysis of NGS data remains challenging. Sequencing errors can result in erroneously detected (false-positve) mutations that result in unnecessary and expensive treatment, and missed mutations (false-negatives) can result in a missed diagnosis and missed opportunity for patient treatment. Further improvements in the accuracy of NGS methods would therefore be beneficial.

### Summary

The present disclosure generally relates to polynucleotide sequences, which may be used to calibrate a wide variety of sequencing methods. These polynucleotide sequences are also referred to herein as "standards", "reference standards" or "controls", and these terms are used interchangeably herein. Thus, the present disclosure provides reference standards which can be used to calibrate a wide variety of high throughput sequencing methods such as NGS methods. The polynucleotide sequences disclosed herein comprise two or more calibration sequences, each independently representing a naturally occurring polynucleotide sequence and arranged consecutively in the polynucleotide sequence in an arrangement not found in any naturally occurring genome. The different calibration sequences may be present in single copies. Alternatively, one or more of the calibration sequences may be present in two or more copy numbers in the polynucleotide sequence. Thus, the present disclsosure provides polynucleotide "ladders" comprising multiple calibration sequences, each independently present in the same copy number or in varying copy numbers.

In one aspect, the present disclosure provides an isolated polynucleotide sequence comprising two or more calibration sequences, wherein the calibration sequences each independently represent a naturally occurring polynucleotide sequence, and wherein the calibration sequences are arranged consecutively within the polynucleotide sequence in an arrangement not found in any naturally occurring genome. One or more of the calibration sequences may be present in two or more copy numbers within the polynucleotide sequence.

A first calibration sequence may represent an exon, intron, gene, allele, chromosome or genome sequence, and a second calibration sequence may differ from the first calibration sequence and may represent a different exon, intron, gene, allele, chromosome or genome sequence. The second calibration sequence may represent a variant of the first calibration sequence.

One or more of the calibration sequences may be indicative of a feature of interest, such as any one or more of: cancer, inherited disease, a pathogen, drug-resistance, circulating tumor DNA, circulating fetal DNA, circulating maternal DNA, or other attribute of interest.

When the polynucleotide disclosed herein comprises a calibration sequence present in two or more copy numbers, those copy numbers may be selected to replicate naturally occurring frequencies of the naturally occurring sequences represented by the calibration sequences. For example but without limitation, the two or more copy numbers may be selected to replicate any one or more of homozygous, heterozygous and/or somatic allele frequencies of naturally occurring human polynucleotide sequences. Other copy number representations are described herein.

The calibration sequences may be independently between 18 nucleotides and 20,000 nucleotides in length. For example, the calibration sequences may be independently between 100 nucleotides and 2,000 nucleotides in length.

In another aspect, the present disclosure provides a vector comprising a DNA sequence encoding the polynucleotide sequence disclosed herein, operably linked to any one or more of the following: a 5' promoter; flanking (3' and 5') endonuclease sites; and a 3' poly-adenine repeat tract.

In another aspect, the present disclosure provides a method of making the polynucleotide sequence of the present disclosure, comprising providing and ligating the two or more calibration sequences; optionally further comprising providing and ligating flanking or terminal sequences and/or a spacer sequence, if present.

In another aspect, the present disclosure provides a composition comprising any two or more polynucleotide sequences disclosed herein.

In another aspect, the present disclosure provides a method of providing a polynucleotide for calibrating a circulating DNA detection method, the method comprising fragmenting the polynucleotide sequence of the present disclosure to produce one or more polynucleotide fragments, which one or more polynucleotide fragments are suitable for calibrating a circulating DNA detection method.

In another aspect, the present disclosure provides a method of providing a polynucleotide for calibrating an RNA sequencing method, the method comprising performing *in-vitro* transcription of the polynucleotide sequence of the present disclosure, which polynucleotide sequence is a DNA polynucleotide sequence, to produce one or more RNA fragments, which one or more RNA fragments are suitable for calibrating an RNA sequencing method.

In another aspect, the present disclosure provides a method of calibrating a polynucleotide sequencing process, comprising:
i) subjecting the polynucleotide sequence of the present disclosure to a polynucleotide sequencing process under a given set of parameters;
ii) recording the sequence of the calibration sequences as determined by the sequencing process performed in i);
iii) comparing the sequence of the calibration sequences recorded in ii) with the known sequence of respective calibration sequences in the polynucleotide sequence of the present disclosure; and
iv) subjecting a sample comprising a polynucleotide of interest to the same sequencing process performed in i) under the same set of parameters;
wherein the accuracy of the sequence determination in iii) is used to calibrate the sequence determination in iv). The quantitation process in iv) may be performed in the absence of the polynucleotide sequence of the present disclosure.

In another aspect, the present disclosure provides a method of calibrating a polynucleotide quantitation process, comprising:
i) subjecting the polynucleotide sequence of the present disclosure to a polynucleotide quantitation process under a given set of parameters;
ii) recording the quantity of the calibration sequences as determined by the quantitation process performed in i);
iii) comparing the quantity of the calibration sequences recorded in ii) with the known quantities of respective calibration sequences in the polynucleotide sequence of the present disclosure; and
iv) subjecting a sample comprising a polynucleotide of interest to the same quantitation process performed in i) under the same set of parameters;
wherein the accuracy of the quantity determination in iii) is used to calibrate the quantity determination in iv). Again, the quantitation process in iv) may be performed in the absence of the polynucleotide sequence of the present disclosure.

As is apparent from the above, any of the methods disclosed herein can be performed separately from equivalent methods having the same parameters, performed on a test sample. For example, the methods disclosed herein can be performed in parallel to an equivalent method performed on a test sample. This reduces the risk of the calibration sequences interfering with the results of the methods performed on a test sample, e.g. by altering the amount of a target polynucleotide sequence of interest in a test sample through the addition of a calibration sequence representing the same target sequence.

In another aspect, the present disclosure provides a method of calibrating a target DNA enrichment process, the method comprising:
i) combining the polynucleotide sequence of the present disclosure with an oligonucleotide of generally complementary sequence under conditions allowing hybridization;
ii) enriching an oligonucleotide hybridized to a polynucleotide sequence;
iii) sequencing the polynucleotide enriched in ii) under a given set of parameters;
iv) comparing the polynucleotide sequence determined in iii) with the known sequence of the polynucleotide sequence; and
v) sequencing the oligonucleotide enriched in ii) under the same set of parameters in iii);
wherein the accuracy of the sequence determination in iv) is used to calibrate the sequence determination in v).

In another aspect, the present disclosure provides a method of calibrating a target DNA enrichment process, the method comprising:
i) combining a known quantity of the polynucleotide sequence of the present disclosure with an oligonucleotide of generally complementary sequence under conditions allowing hybridization;
ii) enriching an oligonucleotide hybridized to a polynucleotide sequence;
iii) quantifying the polynucleotide enriched in ii) under a given set of parameters;
iv) comparing the polynucleotide quantity determined in iii) with the known quantity of the polynucleotide sequence in i); and
v) quantifying the oligonucleotide enriched in ii) under the same set of parameters in iii);
wherein the accuracy of the quantity determination in iv) is used to calibrate the quantity determination in v).

In another aspect, the present disclosure provides the use of the polynucleotide sequence of the present disclosure to calibrate a polynucleotide sequencing and/or quantitation process.

In another aspect, the present disclosure provides a kit comprising a polynucleotide sequence of the present disclosure and one or more nuclease enzymes capable of fragmenting the polynucleotide sequence.

The present disclosure also provides polynucleotide adaptor sequences, each comprising a calibration sequence (such as described herein), which are useful in calibrating methods of determining the identity and/or quantity of polynucleotides in a sample. The incorporation of the calibration sequence within the adaptor sequence improves the efficiency of calibrating those methods, for example, compared to the performance of a separate calibration method performed in parallel to a method performed on a sample. The calibration sequence provides a reference sequence, against which errors and technical bias occurring during performance of the sequencing methods can be determined. Thus, the adaptor sequences disclosed herein can be used during polynucleotide library preparation methods (such as NGS library preparation methods), to measure the sequencing and/or quantitative performance of those library preparation methods.

Accordingly, the present disclosure provides an isolated polynucleotide adaptor sequence comprising a calibration sequence and a primer sequence.

The primer sequence may be complementary (or generally complementary) to a separate primer sequence that is capable of being added to a sample containing a polynucleotide to be replicated. Thus, the adaptor sequence can be added to a sample containing a polynucleotide to be replicated, ligated to a polynucleotide to be replicated, and the resulting, ligated sequence can be bound to by a primer that is added in order to facilitate synthesis of a complementary polynucleotide to that resulting, ligated sequence and/or transcription of that resulting, ligated sequence.

Alternatively, the primer sequence may be complementary (or generally complementary) to a portion of a polynucleotide to be replicated. Thus, the adaptor sequence can be added to a sample containing a polynucleotide to be replicated, and the primer sequence within the adaptor sequence can bind to a complementary sequence within a polynucleotide to be replicated in order to facilitate synthesis of the polynucleotide to be replicated.

The present disclosure also provides an isolated polynucleotide adaptor sequence comprising a calibration sequence and a sequence that is complementary to a target sequence (a "complementary sequence"). The complementary sequence may bind to a target polynucleotide sequence in the same manner as a primer, but without necessarily performing the function of a primer in facilitating replication or synthesis of the target polynucleotide. Thus, the isolated polynucleotide adaptor sequence comprising a calibration sequence and a complementary sequence may not include a primer sequence. Such adaptor polynucleotides comprising a polynucleotide a calibration sequence and a complementary sequence may be particularly useful in calibrating library preparation methods which do not involve poynucleotide replication. For example, such adaptor polynucleotides may be particularly useful in library preparation methods for sequencing with a protein nanopore.

The adaptor sequence may further comprise any one or more of: a sequence complementary to a flow-cell DNA sequence; an index sequence that indicates the identity of a sample containing a polynucleotide to be replicated; and a unique molecular identifier (UMI) sequence that indicates the identity of an individual adaptor sequence.

The calibration sequence may represent a naturally occurring polynucleotide sequence. This may be achieved by the calibration sequence comprising or consisting of a naturally occurring polynucleotide sequence, or by the calibration sequence comprising or consisting of an artificial polynucleotide sequence which nevertheless retains certain higher order features of a naturally occurring polynucleotide sequence (e.g., as described in WO 2016/094947).

The adaptor sequence may be a single stranded or double stranded polynucleotide sequence, and may be a DNA or a RNA polynucleotide sequence.

Any adaptor sequence disclosed herein may comprise a sequence which forms a terminal nucleotide overhang when the adaptor sequence binds to a complementary target sequence. The nucleotide overhang may be a thymine (T). The terminal T nucleotide may be joined by a phosphorothioate bond to prevent nuclease digestion. Alternatively, any adaptor sequence disclosed herein may comprise a sequence which forms a sticky overhang when the adaptor sequence binds to a complementary target sequence. The sticky overhang comprises a sequence that is complementary to a sticky overhang on another polynucleotide, to facilitate annealing of the two polynucleotides.

Two or more calibration sequences may be present in a single adaptor sequence, in different quantities (i.e. copy numbers). In addition, two or more adaptor sequences may be combined in a mixture in known quantities. Thus, the mixture can provide known relative quantities of two or more different calibration sequences. This can be achieved by including in the mixture, adaptors having multiple copies of each calibration sequence. This can also be achieved by including in the mixture, known, different quantities of different adaptors, each adaptor having a single calibration sequence. This can also be achieved through a combination of these approaches. The provision of two or more calibration sequences in different quantities allows the measurement of a quantity of a target polynucleotide to be calibrated.

The present disclosure also provides a composition comprising one or more adaptor sequence as disclosed herein.

The present disclosure also provides a container comprising the composition disclosed herein.

The present disclosure also provides a vector comprising the adaptor sequence disclosed herein.

The present disclosure also provides a kit comprising one or more adaptor sequence disclosed herein, or the composition disclosed herein, and a ligase enzyme and/or a transposase enzyme and/or one or more separate oligonucleotide primer.

The present disclosure also provides a method of making the adaptor sequence disclosed herein, the method comprising excising the adaptor sequence from the vector disclosed herein by endonuclease digestion, or by amplifying the adaptor sequence from the vector disclosed herein, or by transcribing the adaptor sequence comprised within the vector disclosed herein.

The present disclosure also provides a method of preparing a sample comprising a target polynucleotide for a sequencing or quantification process, the method comprising a step of attaching the polynucleotide adaptor sequence disclosed herein to one or both of the termini (i.e. the 5' and/oror 3' ends) of the target polynucleotide.

The present disclosure also provides a method of preparing a sample comprising a target polynucleotide for a sequencing or quantification process, the method comprising contacting the target polynucleotide with an adaptor sequence disclosed herein under conditions suitable to allow the primer sequence in the polynucleotide adaptor sequence to bind to a complementary portion of the target polynucleotide.

The present disclosure also provides the use of the polynucleotide adaptor sequence disclosed herein or the composition disclosed herein, to calibrate a polynucleotide sequencing and/or quantitation process.

Accordingly, the present disclosure also provides a method of calibrating a polynucleotide sequencing process, comprising:
i) adding the adaptor sequence or composition disclosed herein to a sample comprising a target polynucleotide whose sequence is to be determined, under conditions allowing attachment of the polynucleotide adaptor sequence to the 5' and/or 3' termini of the target polynucleotide;
ii) amplifying the target polynucleotide;
iii) determining the sequence of the target polynucleotide;
iv) determining the sequence of the calibration sequence in the adaptor sequence; and
v) comparing the sequence determined in iv) to the original calibration sequence;
wherein the accuracy of the sequence determination in iv) is used to calibrate the sequence determination in iii).

The present disclosure also provides a method of calibrating a polynucleotide sequencing process, comprising:
i) adding the adaptor sequence or composition disclosed herein to a sample comprising a target polynucleotide whose sequence is to be determined, under conditions allowing the primer sequence in the polynucleotide adaptor sequence to bind to the complementary portion of the target polynucleotide;
ii) amplifying the target polynucleotide;
iii) determining the sequence of the target polynucleotide;
iv) determining the sequence of the calibration sequence in the adaptor sequence; and
v) comparing the sequence determined in iv) to the original calibration sequence;
wherein the accuracy of the sequence determination in iv) is used to calibrate the sequence determination in iii).

The present disclosure also provides a method of calibrating a polynucleotide quantitation process, comprising:
i) adding a known amount of an adaptor sequence or composition disclosed herein to a sample comprising a target polynucleotide whose quantity is to be determined, under conditions allowing attachment of the adaptor sequence to the 5' and/or 3' termini of the target polynucleotide;
ii) amplifying the target polynucleotide;
iii) determining the quantity of the target polynucleotide;
iv) determining the quantity of the calibration sequence in the adaptor sequence; and
v) comparing the quantity determined in iv) to the known amount of the calibration sequence in the adaptor sequence in i);
wherein the accuracy of the quantity determination in iv) is used to calibrate the quantity determination in iii).

The present disclosure also provides a method of calibrating a polynucleotide quantitation process, comprising:
i) adding a known amount of an adaptor sequence or composition disclosed herein to a sample comprising a target polynucleotide whose quantity is to be determined, under conditions allowing the primer sequence in the polynucleotide adaptor sequence to bind to the complementary portion of the target polynucleotide;
ii) amplifying the target polynucleotide;
iii) determining the quantity of the target polynucleotide;
iv) determining the quantity of the calibration sequence in the adaptor sequence; and
v) comparing the quantity determined in iv) to the known amount of the calibration sequence in the adaptor sequence in i);
wherein the accuracy of the quantity determination in iv) is used to calibrate the quantity determination in iii).

Each feature of any particular aspect or embodiment or example of the present disclosure may be applied *mutatis mutandis* to any other aspect or embodiment or example of the present disclosure.

### Brief Description of Drawings

The following figures further demonstrate certain aspects of the present disclosure. The disclosure may be better understood by reference to one or more of these figures in combination with the detailed description of specific embodiments presented herein.
**Figure 1**. Schematic illustrating the design and use of a polynucleotide sequence comprising ERBB2 gene exon sequences repeated 1-, 2- and 3- copy number(s).
**Figure 2**. Schematic illustrating the design and use of a polynucleotide sequence comprising TP53 gene exon sequences repeated 1-copy number.
**Figure 3****.** Schematic illustrating the design and use of a polynucleotide sequence comprising bacterial genome sequences repeated 1-, 2- and 4-copy number.
**Figure 4****.** Schematic illustrating the design and use of a polynucleotide sequence comprising calibration sequences that represent genetic variation at somatic allele frequencies in the BRAF gene.
**Figure 5****.** Schematic illustrating the design and use of a polynucleotide sequence comprising calibration sequences that represent genetic variation at heterozygous allele frequencies in the BRCA2 gene.
**Figure 6****.** Size profile of DNA fragments from enzymatic shearing of a polynucleotide sequence for use with circulating DNA sequencing methods.
**Figure 7****.** Schematic illustrating a suitable computer system 3800 for calibrating a polynucleotide sequencing process. The computer system 3800 comprises a processor 3802 connected to a program memory 3804, a data memory 3806, a communication port 3808 and a user port 3810.

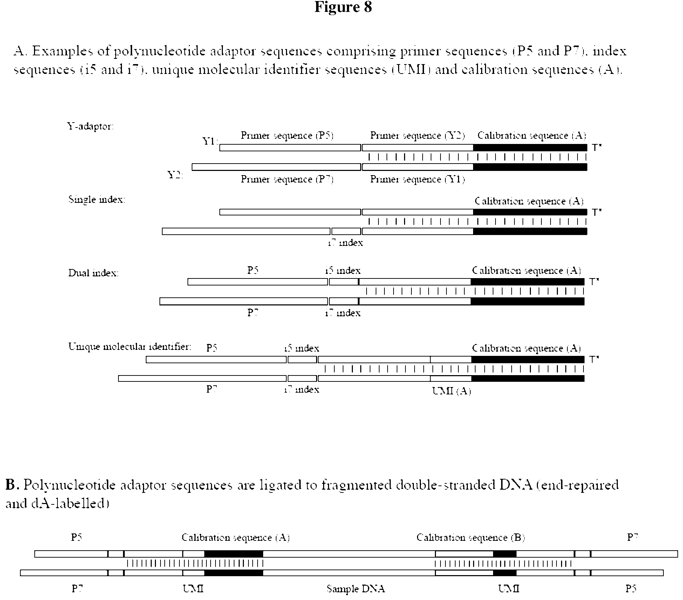

**Figure 8****.** Design of polynucleotide adaptor sequences comprising calibration sequences during library preparation and sequencing. (A) Schematic diagram showing the location of calibration sequences within a Y-adaptor, and the position relative to P5 and P7 primer sequences, index sequences, and unique molecular identifiers (UMI), and T-nucleotide overhang. (B) Schematic diagram showing the polynucleotide adaptor sequence ligated to the termini of test sample DNA fragments during the library preparation steps for next-generation sequencing.

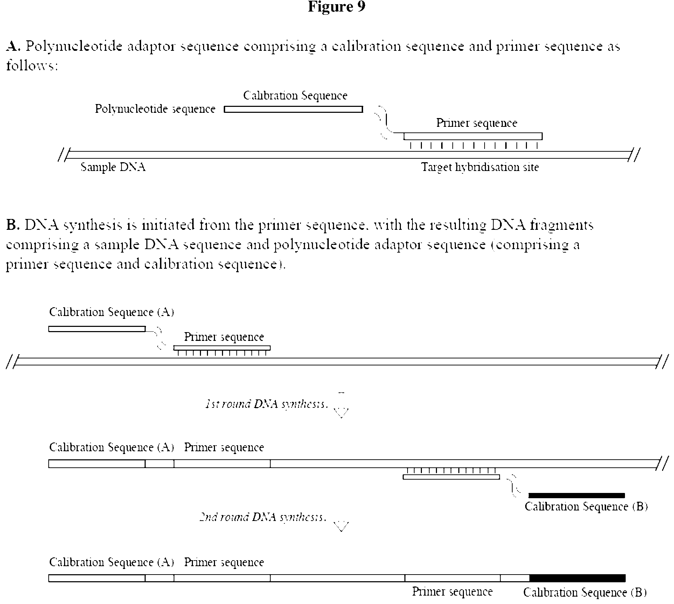

**Figure 9****.** Incorporation of polynucleotide adaptor sequences comprising calibration sequences during DNA synthesis. (A) The polynucleotide adaptor sequences comprise a calibration sequence and a primer sequence that is complementary to a target site in sample DNA. The polynucleotide adaptor sequence is hybridized to target DNA, and DNA synthesis is initiated from the primer sequence. (B) The first and second round of DNA synthesis incorporates the polynucleotide adaptor sequence, comprising the primer sequence and the calibration sequence, with the test sample DNA fragment to form single continuous DNA sequence.
**Figure 10****.** Use of polynucleotide adaptor sequences during long read (nanopore) sequencing. (A) Two polynucleotide adaptor sequences comprising two calibration sequences, with the second calibration sequence (G) being a genetic variant sequence of the first calibration sequence (WT). (B) Polynucleotide sequences are ligated to 5' and 3' termini of long test sample DNA fragments. (C) Long sample DNA fragments and polynucleotide adaptor sequences (including calibration sequences) are sequenced using nanopore technology.

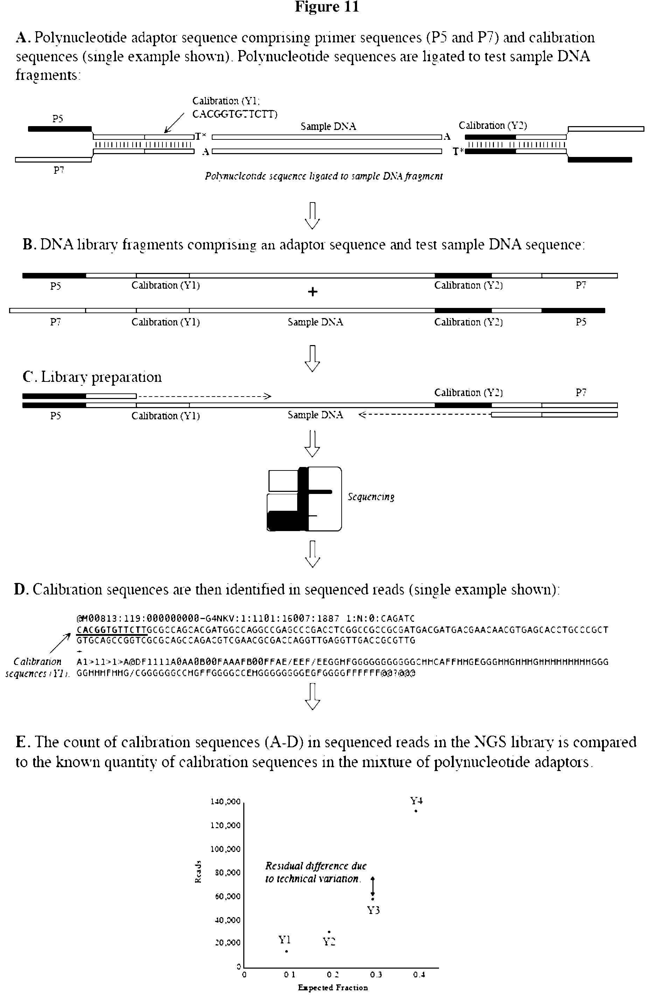

**Figure 11****.** Use of polynucleotide adaptor sequences during library preparation and next-generation sequencing. (A) The polynucleotide adaptor sequences are ligated to test sample DNA. (B) Library fragments comprise sample DNA sequence and polynucleotide adaptor sequences that include calibration sequences. (C) During library preparation, DNA synthesis incorporates calibration and adaptor sequences at end regions. (D) Calibration sequences are identified within the sequenced read. (E) Scatter-plot indicates the observed count of calibration sequences within reads relative to the expected fractional abundance of calibration sequences within the adaptor mixture.
**Figure 12****.** (A) Genome-browser view shows sequenced long-read alignments corresponding to the calibration sequence within the polynucleotide adaptor. Sequencing errors are indicated. Lower panel shows an example sequenced read, with calibration sequence indicated. (B) Scatter-plot shows the observed fractional abundance of different calibration sequences (Y5-Y8) relative to their expected fractional abundance in the NGS library.
**Figure 13****.** (A) Genome-browser view shows sequenced long-read alignments corresponding to the calibration sequences within the polynucleotide adaptor. Lower panel shows an example sequenced read, with calibration sequence indicated. The presence of the genetic variant sequence (C) is indicated.
**Figure 14****.** (A) Genome-browser view shows sequenced long-read alignments corresponding to the calibration sequences within the polynucleotide adaptor. The calibration sequence represents the human reference genome sequence corresponding to the BRAF gene.
**Figure 15****.** (A) Genome browser view shows the region of the human EFGR gene that is targeted by primer sequences within polynucleotide adaptor sequences. (B) Schematic diagram shows polynucleotide adaptor sequence, comprising calibration sequence and primer sequence that targets human EFGFR gene. (C) Example sequenced read shows the identification of calibration sequences in sequenced reads in output library (FASTQ).

### Key to the Sequence Listing

| | |
|---|---|
| SEQ ID NO: 1 | Nucleotide sequence of a linear polynucleotide sequence comprising three calibration sequences from the ERBB2 gene (EG1_ERBB2). |
| SEQ ID NO: 2 | Nucleotide sequence of EG1_EGFR. |
| SEQ ID NO: 3 | Nucleotide sequence of EG1_MET. |
| SEQ ID NO: 4 | Nucleotide sequence of EG1_BRAF. |
| SEQ ID NO: 5 | Nucleotide sequence of EG1_FGFR1. |
| SEQ ID NO: 6 | Nucleotide sequence of a linear polynucleotide sequence comprising five calibration sequences from the TP53 gene (EG2_TP53). |
| SEQ ID NO: 7 | Nucleotide sequence of a linear polynucleotide sequence comprising calibration sequences from Pseudomonas aeruginosa PAO1, Haemophilus influenzae Rd KW20 and Escherichia coli K-12 (EG3_16S). |
| SEQ ID NO: 8 | Nucleotide sequence of a BRAF gene calibration sequence (EG4_BRAF). |
| SEQ ID NO: 9 | Nucleotide sequence of two calibration sequences from the BRCA2 gene joined together (EG4_BRCA2). |
| SEQ ID NO: 10 | Nucleotide sequence of three calibration sequences from the BCR, ABL and ALK genes joined together (EG6_GENES). |
| SEQ ID NO: 11 | Nucleotide sequence of a calibration sequence from an exon from the ERBB2 gene (EG1_CNV_ERBB2_A). |
| SEQ ID NO: 12 | Nucleotide sequence of a calibration sequence from an exon from the ERBB2 gene (EG1_CNV_ERBB2_B). |
| SEQ ID NO: 13 | Nucleotide sequence of a calibration sequence from an exon from the ERBB2 gene (EG1_CNV_ERBB2_C). |
| SEQ ID NO: 14 | Nucleotide sequence of a calibration sequence from the EGFR gene (EG1_CNV_EGFR_A). |
| SEQ ID NO: 15 | Nucleotide sequence of a calibration sequence from the EGFR gene (EG1_CNV_EGFR_B). |
| SEQ ID NO: 16 | Nucleotide sequence of a calibration sequence from the EGFR gene (EG1_CNV_EGFR_C). |
| SEQ ID NO: 17 | Nucleotide sequence of a calibration sequence from the MET gene (EG1_CNV_MET_A). |
| SEQ ID NO: 18 | Nucleotide sequence of a calibration sequence from the MET gene (EG1_CNV_MET_B). |
| SEQ ID NO: 19 | Nucleotide sequence of a calibration sequence from the MET gene (EG1_CNV_MET_C). |
| SEQ ID NO: 20 | Nucleotide sequence of a calibration sequence from the BRAF gene (EG1_CNV_BRAF_A). |
| SEQ ID NO: 21 | Nucleotide sequence of a calibration sequence from the BRAF gene (EG1_CNV_BRAF_B). |
| SEQ ID NO: 22 | Nucleotide sequence of a calibration sequence from the BRAF gene (EG1_CNV_BRAF_C). |
| SEQ ID NO: 23 | Nucleotide sequence of a calibration sequence from the FGFR1 gene (EG1_CNV_FGFR1_A). |
| SEQ ID NO: 24 | Nucleotide sequence of a calibration sequence from the FGFR1 gene (EG1_CNV_FGFR1_B). |
| SEQ ID NO: 25 | Nucleotide sequence of a calibration sequence from the FGFR1 gene (EG1_CNV_FGFR1_C). |
| SEQ ID NO: 26 | Nucleotide sequence of a calibration sequence that overlaps with the TP53 gene exons (EG2_TP53_A). |
| SEQ ID NO: 27 | Nucleotide sequence of a calibration sequence that overlaps with the TP53 gene exons (EG2_TP53_B). |
| SEQ ID NO: 28 | Nucleotide sequence of a calibration sequence that overlaps with the TP53 gene exons (EG2_TP53_C). |
| SEQ ID NO: 29 | Nucleotide sequence of a calibration sequence that overlaps with the TP53 gene exons (EG2_TP53_D). |
| SEQ ID NO: 30 | Nucleotide sequence of a calibration sequence that overlaps with the TP53 gene exons (EG2_TP53_E). |
| SEQ ID NO: 31 | Nucleotide sequence of a polynucleotide sequence comprising a calibration sequence from Pseudomonas aeruginosa PAO1 (EG3_16S_pseuAeru_C). |
| SEQ ID NO: 32 | Nucleotide sequence of a polynucleotide sequence comprising a calibration sequence from Haemophilus influenzae Rd KW20 (EG3_16S_haemInfl_B). |
| SEQ ID NO: 33 | Nucleotide sequence of a polynucleotide sequence comprising a calibration sequence from Escherichia coli K-12 (EG3_16S_eschColi_A). |
| SEQ ID NO: 34 | Nucleotide sequence of a polynucleotide sequence comprising a calibration sequence that represents a different genetic variant of BRAF (EG4_BRAF_WT). |
| SEQ ID NO: 35 | Nucleotide sequence of a polynucleotide sequence comprising a calibration sequence that represents a different genetic variant of BRAF (EG4_BRAF_V600E). |
| SEQ ID NO: 36 | Nucleotide sequence of a calibration sequence from the BRCA2 gene (EG5_BRCA2_WT). |
| SEQ ID NO: 37 | Nucleotide sequence of a calibration sequence from the BRCA2 with a single nucleotide deletion (EG5_BRCA2_DEL). |
| SEQ ID NO: 38 | Nucleotide sequence of a synthetic sequence to measure gene expression of the BCR gene (EG6_GENE_BCR_A). |
| SEQ ID NO: 39 | Nucleotide sequence of a synthetic sequence to measure gene expression of the ABL gene (EG6_GENE_ABL_B). |
| SEQ ID NO: 40 | Nucleotide sequence of a synthetic sequence to measure gene expression of the ALK gene (EG6_GENE_ALK_C). |
| SEQ ID NO: 41 | Nucleotide sequence of an artificial spacer sequence used in assembly of ERBB2_SPCR. |
| SEQ ID NO: 42 | Nucleotide sequence of an artificial spacer sequence used in assembly of EGFR_SPCR. |
| SEQ ID NO: 43 | Nucleotide sequence of an artificial spacer sequence used in assembly of MET_SPCR. |
| SEQ ID NO: 44 | Nucleotide sequence of an artificial spacer sequence used in assembly of BRAF_SPCR. |
| SEQ ID NO: 45 | Nucleotide sequence of an artificial spacer sequence used in assembly of FGFR1_SPCR. |
| SEQ ID NO: 46 | Nucleotide sequence of Y-shaped adaptor sequence designated Y1A. |
| SEQ ID NO: 47 | Nucleotide sequence of Y1A primer. |
| SEQ ID NO: 48 | Nucleotide sequence of Y1A calibration sequence. |
| SEQ ID NO: 49 | Nucleotide sequence of Y1A Y sequence. |
| SEQ ID NO: 50 | Nucleotide sequence of Y-shaped adaptor sequence designated Y2A. |
| SEQ ID NO: 51 | Nucleotide sequence of Y2A primer. |
| SEQ ID NO: 52 | Nucleotide sequence of Y2A calibration sequence. |
| SEQ ID NO: 53 | Nucleotide sequence of Y2A Y sequence. |
| SEQ ID NO: 54 | Nucleotide sequence of Y-shaped adaptor sequence designated Y3A. |
| SEQ ID NO: 55 | Nucleotide sequence of Y3A primer. |
| SEQ ID NO: 56 | Nucleotide sequence of Y3A calibration sequence. |
| SEQ ID NO: 57 | Nucleotide sequence of Y3A Y sequence. |
| SEQ ID NO: 58 | Nucleotide sequence of Y-shaped adaptor sequence designated Y4A. |
| SEQ ID NO: 59 | Nucleotide sequence of Y4A primer. |
| SEQ ID NO: 60 | Nucleotide sequence of Y4A calibration sequence. |
| SEQ ID NO: 61 | Nucleotide sequence of Y4A Y sequence. |
| SEQ ID NO: 62 | Nucleotide sequence of Y-shaped adaptor sequence designated Y1B. |
| SEQ ID NO: 63 | Nucleotide sequence of Y1B primer. |
| SEQ ID NO: 64 | Nucleotide sequence of Y1B calibration sequence. |
| SEQ ID NO: 65 | Nucleotide sequence of Y1B Y sequence. |
| SEQ ID NO: 66 | Nucleotide sequence of Y-shaped adaptor sequence designated Y2A. |
| SEQ ID NO: 67 | Nucleotide sequence of Y2B primer. |
| SEQ ID NO: 68 | Nucleotide sequence of Y2B calibration sequence. |
| SEQ ID NO: 69 | Nucleotide sequence of Y2B Y sequence. |
| SEQ ID NO: 70 | Nucleotide sequence of Y-shaped adaptor sequence designated Y3B. |
| SEQ ID NO: 71 | Nucleotide sequence of Y3B primer. |
| SEQ ID NO: 72 | Nucleotide sequence of Y3B calibration sequence. |
| SEQ ID NO: 73 | Nucleotide sequence of Y3B Y sequence. |
| SEQ ID NO: 74 | Nucleotide sequence of Y-shaped adaptor sequence designated Y4B. |
| SEQ ID NO: 75 | Nucleotide sequence of Y4B primer. |
| SEQ ID NO: 76 | Nucleotide sequence of Y4B calibration sequence. |
| SEQ ID NO: 77 | Nucleotide sequence of Y4B Y sequence. |
| SEQ ID NO: 78 | Nucleotide sequence of Y-shaped adaptor sequence designated Y5A. |
| SEQ ID NO: 79 | Nucleotide sequence of Y5A calibration sequence. |
| SEQ ID NO: 80 | Nucleotide sequence of Y5A Y sequence. |
| SEQ ID NO: 81 | Nucleotide sequence of Y-shaped adaptor sequence designated Y6A. |
| SEQ ID NO: 82 | Nucleotide sequence of Y6A calibration sequence. |
| SEQ ID NO: 83 | Nucleotide sequence of Y6A Y sequence. |
| SEQ ID NO: 84 | Nucleotide sequence of Y-shaped adaptor sequence designated Y7A. |
| SEQ ID NO: 85 | Nucleotide sequence of Y7A calibration sequence. |
| SEQ ID NO: 86 | Nucleotide sequence of Y7A Y sequence. |
| SEQ ID NO: 87 | Nucleotide sequence of Y-shaped adaptor sequence designated Y8A. |
| SEQ ID NO: 88 | Nucleotide sequence of Y8A calibration sequence. |
| SEQ ID NO: 89 | Nucleotide sequence of Y8A Y sequence. |
| SEQ ID NO: 90 | Nucleotide sequence of Y-shaped adaptor sequence designated Y5B. |
| SEQ ID NO: 91 | Nucleotide sequence of Y5B calibration sequence. |
| SEQ ID NO: 92 | Nucleotide sequence of Y5B Y sequence. |
| SEQ ID NO: 93 | Nucleotide sequence of Y-shaped adaptor sequence designated Y6B. |
| SEQ ID NO: 94 | Nucleotide sequence of Y6B calibration sequence. |
| SEQ ID NO: 95 | Nucleotide sequence of Y6B Y sequence. |
| SEQ ID NO: 96 | Nucleotide sequence of Y-shaped adaptor sequence designated Y7B. |
| SEQ ID NO: 97 | Nucleotide sequence of Y7B calibration sequence. |
| SEQ ID NO: 98 | Nucleotide sequence of Y7B Y sequence. |
| SEQ ID NO: 99 | Nucleotide sequence of Y-shaped adaptor sequence designated Y8B. |
| SEQ ID NO: 100 | Nucleotide sequence of Y8B calibration sequence. |
| SEQ ID NO: 101 | Nucleotide sequence of Y8B Y sequence. |
| SEQ ID NO: 102 | Nucleotide sequence of Y-shaped adaptor sequence designated Y9A. |
| SEQ ID NO: 103 | Nucleotide sequence of Y9A calibration sequence. |
| SEQ ID NO: 104 | Nucleotide sequence of Y9A Y sequence. |
| SEQ ID NO: 105 | Nucleotide sequence of Y-shaped adaptor sequence designated Y9B |
| SEQ ID NO: 106 | Nucleotide sequence of Y9B calibration sequence. |
| SEQ ID NO: 107 | Nucleotide sequence of Y9B Y sequence. |
| SEQ ID NO: 108 | Nucleotide sequence of Y-shaped adaptor sequence designated Y10A. |
| SEQ ID NO: 109 | Nucleotide sequence of Y10A calibration sequence. |
| SEQ ID NO: 110 | Nucleotide sequence of Y10A Y sequence. |
| SEQ ID NO: 111 | Nucleotide sequence of Y-shaped adaptor sequence designated Y11A. |
| SEQ ID NO: 112 | Nucleotide sequence of Y11A calibration sequence. |
| SEQ ID NO: 113 | Nucleotide sequence of Y11A Y sequence. |
| SEQ ID NO: 114 | Nucleotide sequence of Y-shaped adaptor sequence designated Y10B. |
| SEQ ID NO: 115 | Nucleotide sequence of Y10B calibration sequence. |
| SEQ ID NO: 116 | Nucleotide sequence of Y10B Y sequence. |
| SEQ ID NO: 117 | Nucleotide sequence of Y-shaped adaptor sequence designated Y11B. |
| SEQ ID NO: 118 | Nucleotide sequence of Y11B calibration sequence. |
| SEQ ID NO: 119 | Nucleotide sequence of Y11B Y sequence. |
| SEQ ID NO: 120 | Nucleotide sequence of Y-shaped adaptor sequence designated Y12F. |
| SEQ ID NO: 121 | Nucleotide sequence of Y12F primer. |
| SEQ ID NO: 122 | Nucleotide sequence of Y12F calibration sequence. |
| SEQ ID NO: 123 | Nucleotide sequence of Y-shaped adaptor sequence designated Y13F. |
| SEQ ID NO: 124 | Nucleotide sequence of Y13F primer. |
| SEQ ID NO: 125 | Nucleotide sequence of Y13F calibration sequence. |
| SEQ ID NO: 126 | Nucleotide sequence of Y-shaped adaptor sequence designated Y14F. |
| SEQ ID NO: 127 | Nucleotide sequence of Y14F primer. |
| SEQ ID NO: 128 | Nucleotide sequence of Y14F calibration sequence. |
| SEQ ID NO: 129 | Nucleotide sequence of Y-shaped adaptor sequence designated Y12R. |
| SEQ ID NO: 130 | Nucleotide sequence of Y12R primer |
| SEQ ID NO: 131 | Nucleotide sequence of Y12R calibration sequence. |
| SEQ ID NO: 132 | Nucleotide sequence of Y-shaped adaptor sequence designated Y13R. |
| SEQ ID NO: 133 | Nucleotide sequence of Y13R primer. |
| SEQ ID NO: 134 | Nucleotide sequence of Y13R calibration sequence. |
| SEQ ID NO: 135 | Nucleotide sequence of Y-shaped adapter sequence designated Y14R. |
| SEQ ID NO: 136 | Nucleotide sequence of Y14R primer. |
| SEQ ID NO: 137 | Nucleotide sequence of Y14R calibration sequence. |
| SEQ ID NO: 138 | Nucleotide sequence of polyT mRNA adaptor sequence. |
| SEQ ID NO: 139 | Nucleotide sequence of polyT mRNA adaptor primer sequence. |
| SEQ ID NO: 140 | Nucleotide sequence of polyT mRNA adaptor calibration sequence. |
| SEQ ID NO: 141 | Nucleotide sequence of polyT mRNA adaptor. |
| SEQ ID NO: 142 | Nucleotide sequence of polyT mRNA adaptor primer sequence. |
| SEQ ID NO: 143 | Nucleotide sequence of polyT mRNA adaptor calibration sequence. |
| SEQ ID NO: 144 | Nucleotide sequence of natural Human immunodeficiency virus (HIV) sequence adaptors. |
| SEQ ID NO: 145 | Nucleotide sequence of natural HIV sequence adaptors primer sequence. |
| SEQ ID NO: 146 | Nucleotide sequence of natural HIV sequence adaptors calibration sequence. |
| SEQ ID NO: 147 | Nucleotide sequence of natural Human immunodeficiency virus (HIV) sequence adaptors. |
| SEQ ID NO: 148 | Nucleotide sequence of natural HIV sequence adaptors primer sequence. |
| SEQ ID NO: 149 | Nucleotide sequence of natural HIV sequence adaptors calibration sequence. |
| SEQ ID NO: 150 | Nucleotide sequence of PCR Primer 1.0 (P5). |
| SEQ ID NO: 151 | Nucleotide sequence of PCR Primer 2.0 (P7). |
| SEQ ID NO: 152 | Nucleotide sequence of TruSeq Universal Adapter. |
| SEQ ID NO: 153 | Nucleotide sequence of TruSeq Adapter, Index 1. |
| SEQ ID NO: 154 | Nucleotide sequence of TruSeq Adapter, Index 2. |
| SEQ ID NO: 155 | Nucleotide sequence of TruSeq Adapter, Index 3. |
| SEQ ID NO: 156 | Nucleotide sequence of TruSeq Adapter, Index 4. |
| SEQ ID NO: 157 | Nucleotide sequence of TruSeq Adapter, Index 5. |
| SEQ ID NO: 158 | Nucleotide sequence of TruSeq Adapter, Index 6. |
| SEQ ID NO: 159 | Nucleotide sequence of TruSeq Adapter, Index 7. |
| SEQ ID NO: 160 | Nucleotide sequence of TruSeq Adapter, Index 8. |
| SEQ ID NO: 161 | Nucleotide sequence of TruSeq Adapter, Index 9. |
| SEQ ID NO: 162 | Nucleotide sequence of TruSeq Adapter, Index 10. |
| SEQ ID NO: 163 | Nucleotide sequence of TruSeq Adapter, Index 11. |
| SEQ ID NO: 164 | Nucleotide sequence of TruSeq Adapter, Index 12. |
| SEQ ID NO: 165 | Nucleotide sequence of TruSeq Adapter, Index 13. |
| SEQ ID NO: 166 | Nucleotide sequence of TruSeq Adapter, Index 14. |
| SEQ ID NO: 167 | Nucleotide sequence of TruSeq Adapter, Index 15. |
| SEQ ID NO: 168 | Nucleotide sequence of TruSeq Adapter, Index 16. |
| SEQ ID NO: 169 | Nucleotide sequence of TruSeq Adapter, Index 18. |
| SEQ ID NO: 170 | Nucleotide sequence of TruSeq Adapter, Index 19. |
| SEQ ID NO: 171 | Nucleotide sequence of TruSeq Adapter, Index 20. |
| SEQ ID NO: 172 | Nucleotide sequence of TruSeq Adapter, Index 21. |
| SEQ ID NO: 173 | Nucleotide sequence of TruSeq Adapter, Index 22. |
| SEQ ID NO: 174 | Nucleotide sequence of TruSeq Adapter, Index 23. |
| SEQ ID NO: 175 | Nucleotide sequence of TruSeq Adapter, Index 25. |
| SEQ ID NO: 176 | Nucleotide sequence of TruSeq Adapter, Index 27. |
| SEQ ID NO: 177 | Nucleotide sequence of NEB Index 1. |
| SEQ ID NO: 178 | Nucleotide sequence of NEB Index 2. |
| SEQ ID NO: 179 | Nucleotide sequence of NEB Index 3. |
| SEQ ID NO: 180 | Nucleotide sequence of NEB Index 4. |
| SEQ ID NO: 181 | Nucleotide sequence of NEB Index 5. |
| SEQ ID NO: 182 | Nucleotide sequence of NEB Index 6. |
| SEQ ID NO: 183 | Nucleotide sequence of NEB Index 7. |
| SEQ ID NO: 184 | Nucleotide sequence of NEB Index 8. |
| SEQ ID NO: 185 | Nucleotide sequence of NEB Index 9. |
| SEQ ID NO: 186 | Nucleotide sequence of NEB Index 10. |
| SEQ ID NO: 187 | Nucleotide sequence of NEB Index 11. |
| SEQ ID NO: 188 | Nucleotide sequence of NEB Index 12. |
| SEQ ID NO: 189 | Nucleotide sequence of NEBnext Universal primer. |
| SEQ ID NO: 190 | Nucleotide sequence of NEBNext adaptor for Illumina. |
| SEQ ID NO: 191 | Nucleotide sequence of 16S Forward primer. |
| SEQ ID NO: 192 | Nucleotide sequence of 23S Reverse primer. |
| SEQ ID NO: 193 | Nucleotide sequence of TruSeq Adapter, Index 1. |
| SEQ ID NO: 194 | Nucleotide sequence of TruSeq Adapter, Index 2. |
| SEQ ID NO: 195 | Nucleotide sequence of TruSeq Adapter, Index 3. |
| SEQ ID NO: 196 | Nucleotide sequence of TruSeq Adapter, Index 4. |
| SEQ ID NO: 197 | Nucleotide sequence of TruSeq Adapter, Index 5. |
| SEQ ID NO: 198 | Nucleotide sequence of TruSeq Adapter, Index 6. |
| SEQ ID NO: 199 | Nucleotide sequence of TruSeq Adapter, Index 7. |
| SEQ ID NO: 200 | Nucleotide sequence of TruSeq Adapter, Index 8. |
| SEQ ID NO: 201 | Nucleotide sequence of TruSeq Adapter, Index 9. |
| SEQ ID NO: 202 | Nucleotide sequence of TruSeq Adapter, Index 10. |
| SEQ ID NO: 203 | Nucleotide sequence of TruSeq Adapter, Index 11. |
| SEQ ID NO: 204 | Nucleotide sequence of TruSeq Adapter, Index 12. |
| SEQ ID NO: 205 | Nucleotide sequence of TruSeq Adapter, Index 13. |
| SEQ ID NO: 206 | Nucleotide sequence of TruSeq Adapter, Index 14. |
| SEQ ID NO: 207 | Nucleotide sequence of TruSeq Adapter, Index 15. |
| SEQ ID NO: 208 | Nucleotide sequence of TruSeq Adapter, Index 16. |
| SEQ ID NO: 209 | Nucleotide sequence of TruSeq Adapter, Index 18. |
| SEQ ID NO: 210 | Nucleotide sequence of TruSeq Adapter, Index 19. |
| SEQ ID NO: 211 | Nucleotide sequence of TruSeq Adapter, Index 20. |
| SEQ ID NO: 212 | Nucleotide sequence of TruSeq Adapter, Index 21. |
| SEQ ID NO: 213 | Nucleotide sequence of TruSeq Adapter, Index 22. |
| SEQ ID NO: 214 | Nucleotide sequence of TruSeq Adapter, Index 23. |
| SEQ ID NO: 215 | Nucleotide sequence of TruSeq Adapter, Index 25. |
| SEQ ID NO: 216 | Nucleotide sequence of TruSeq Adapter, Index 27. |
| SEQ ID NO: 217 | UMI sequence. |
| SEQ ID NO: 218 | PolyT sequence. |
| SEQ ID NO: 219 | Nucleotide sequence of Experimental read sequence 1. |
| SEQ ID NO: 220 | Nucleotide sequence of Experimental read sequence 2. |
| SEQ ID NO: 221 | Nucleotide sequence of Experimental read sequence 3. |
| SEQ ID NO: 222 | Nucleotide sequence of Experimental read sequence 4. |
| SEQ ID NO: 223 | Nucleotide sequence of Experimental read sequence 5. |
| SEQ ID NO: 224 | Nucleotide sequence of Experimental read sequence 6. |
| SEQ ID NO: 225 | Nucleotide sequence of IDT Dual Index UMI Adapter 1. |
| SEQ ID NO: 226 | Nucleotide sequence of IDT Dual Index UMI Adapter 2. |
| SEQ ID NO: 227 | Nucleotide sequence of IDT Dual Index UMI Adapter 3. |
| SEQ ID NO: 228 | Nucleotide sequence of IDT Dual Index UMI Adapter 4. |
| SEQ ID NO: 229 | Nucleotide sequence of IDT Dual Index UMI Adapter 5. |
| SEQ ID NO: 230 | Nucleotide sequence of IDT Dual Index UMI Adapter 6. |

### Detailed Description

### General

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

As used herein, the singular forms of "a", "and" and "the" include plural forms of these words, unless the context clearly dictates otherwise.

The term "and/or", e.g., "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The term "about" as used herein refers to a range of +/-10*%* of the specified value and is taken to include an explicit reference to the stated value itself (e.g., the term "about 10" includes an explicit reference to 10).

The term "generally complementary" shall be understood to mean having sufficient sequence identity to allow binding of one polynucleotide to another. For example, any sequence described herein may be at least 50*%*, at least 60*%*, at least 70*%*, at least 80*%*, at least 85*%*, at least 90*%*, at least 95*%*, at least 97*%*, at least 98*%*, or at least 99*%* identical to a target sequence but may still be capable of hybridizing to that target sequence. The extent of the hybridization may be sufficient to achieve any given function or methodological processing step of a polynucleotide, for example, to facilitate the initiation of transcription or to facilitate enrichment or selection or processing of a double stranded polynucleotide within a sample.

### Polynucleotide sequence

The polynucleotide sequence disclosed herein can be a single continuous RNA or DNA sequence that encodes two or more calibration sequences. The polynucleotide sequence may be a recombinant polynucleotide sequence. Th polynucleotide sequences described herein may also be an adaptor polynucleotide sequence as described herein.

### Polynucleotide adaptor sequence

A polynucleotide adaptor sequence (referred to herein interchangeably as "the adaptor sequence" or "the adaptor" or "the adaptor molecule") disclosed herein can be a single continuous RNA or DNA sequence. Preferably, the adaptor sequence is a DNA sequence. The adaptor sequence may be a recombinant polynucleotide sequence.

The adaptor sequence is capable of being used in a polynucleotide sequencing and/or quantitation method. The function imparted by the adaptor sequence is to facilitate synthesis (or replication) of a target polynucleotide sequence of interest during a polynucleotide sequencing and/or quantitation method. This is achieved by the inclusion of a primer sequence within the adaptor sequence. The primer sequence facilitates specific binding of DNA synthesis protein molecules at the site of the primer, allowing initiation of synthesis (or replication). Any known primer can be included in the adapter sequence disclosed herein.

For example, primer sequences can be generally complementary to (or complementary to) any commercially available primers. Examples of commercially available primers include P5 and P7 primers (Illumina). These and other examples of commercially available primers are included in the following Table 1.

**Table 1: Non-limiting examples of commercially available primers, adaptors, index sequences and UMI sequences.**

| **Primer** | **Sequence** | **SEQ ID NO** |
|---|---|---|
| PCR Primer 1.0 (P5) | AATGATACGGCGACCACCGAGATCTACACTCTTTC CCTACACGA | 150 |
| PCR Primer 2.0 (P7) | CAAGCAGAAGACGGCATACGAGAT | 151 |
| TruSeq Universal Adapter | AATGATACGGCGACCACCGAGATCTACACTCTTTC CCTACACGACGCTCTTCCGATC | 152 |
| TruSeq Adapter, Index 1 | GATCGGAAGAGCACACGTCTGAACTCCAGTCACAT CACGATCTCGTATGCCGTCTTCTGCTTG | 153 |
| TruSeq Adapter, Index 2 | GATCGGAAGAGCACACGTCTGAACTCCAGTCACCG ATGTATCTCGTATGCCGTCTTCTGCTTG | 154 |
| TruSeq Adapter, Index 3 | GATCGGAAGAGCACACGTCTGAACTCCAGTCACTT AGGCATCTCGTATGCCGTCTTCTGCTTG | 155 |
| TruSeq Adapter, Index 4 | GATCGGAAGAGCACACGTCTGAACTCCAGTCACTG ACCAATCTCGTATGCCGTCTTCTGCTTG | 156 |
| TruSeq Adapter, Index 5 | GATCGGAAGAGCACACGTCTGAACTCCAGTCACAC AGTGATCTCGTATGCCGTCTTCTGCTTG | 157 |
| TruSeq Adapter, Index 6 | GATCGGAAGAGCACACGTCTGAACTCCAGTCACGC CAATATCTCGTATGCCGTCTTCTGCTTG | 158 |
| TruSeq Adapter, Index 7 | GATCGGAAGAGCACACGTCTGAACTCCAGTCACCA GATCATCTCGTATGCCGTCTTCTGCTTG | 159 |
| TruSeq Adapter, Index 8 | GATCGGAAGAGCACACGTCTGAACTCCAGTCACAC TTGAATCTCGTATGCCGTCTTCTGCTTG | 160 |
| TruSeq Adapter, Index 9 | GATCGGAAGAGCACACGTCTGAACTCCAGTCACGA TCAGATCTCGTATGCCGTCTTCTGCTTG | 161 |
| TruSeq Adapter, Index 10 | GATCGGAAGAGCACACGTCTGAACTCCAGTCACTA GCTTATCTCGTATGCCGTCTTCTGCTTG | 162 |
| TruSeq Adapter, Index 11 | GATCGGAAGAGCACACGTCTGAACTCCAGTCACGG CTACATCTCGTATGCCGTCTTCTGCTTG | 163 |
| TruSeq Adapter, Index 12 | GATCGGAAGAGCACACGTCTGAACTCCAGTCACCT TGTAATCTCGTATGCCGTCTTCTGCTTG | 164 |
| TruSeq Adapter, Index 13 | GATCGGAAGAGCACACGTCTGAACTCCAGTCACAG TCAACAATCTCGTATGCCGTCTTCTGCTTG | 165 |
| TruSeq Adapter, Index 14 | GATCGGAAGAGCACACGTCTGAACTCCAGTCACAG TTCCGTATCTCGTATGCCGTCTTCTGCTTG | 166 |
| TruSeq Adapter, Index 15 | GATCGGAAGAGCACACGTCTGAACTCCAGTCACAT GTCAGAATCTCGTATGCCGTCTTCTGCTTG | 167 |
| TruSeq Adapter, Index 16 | GATCGGAAGAGCACACGTCTGAACTCCAGTCACCC GTCCCGATCTCGTATGCCGTCTTCTGCTTG | 168 |
| TruSeq Adapter, Index 18 | GATCGGAAGAGCACACGTCTGAACTCCAGTCACGT CCGCACATCTCGTATGCCGTCTTCTGCTTG | 169 |
| TruSeq Adapter, Index 19 | GATCGGAAGAGCACACGTCTGAACTCCAGTCACGT GAAACGATCTCGTATGCCGTCTTCTGCTTG | 170 |
| TruSeq Adapter, Index 20 | GATCGGAAGAGCACACGTCTGAACTCCAGTCACGT GGCCTTATCTCGTATGCCGTCTTCTGCTTG | 171 |
| TruSeq Adapter, Index 21 | GATCGGAAGAGCACACGTCTGAACTCCAGTCACGT TTCGGAATCTCGTATGCCGTCTTCTGCTTG | 172 |
| TruSeq Adapter, Index 22 | GATCGGAAGAGCACACGTCTGAACTCCAGTCACCG TACGTAATCTCGTATGCCGTCTTCTGCTTG | 173 |
| TruSeq Adapter, Index 23 | GATCGGAAGAGCACACGTCTGAACTCCAGTCACGA GTGGATATCTCGTATGCCGTCTTCTGCTTG | 174 |
| TruSeq Adapter, Index 25 | GATCGGAAGAGCACACGTCTGAACTCCAGTCACAC TGATATATCTCGTATGCCGTCTTCTGCTTG | 175 |
| TruSeq Adapter, Index 27 | GATCGGAAGAGCACACGTCTGAACTCCAGTCACAT TCCTTTATCTCGTATGCCGTCTTCTGCTTG | 176 |
| NEB Adaptor, Index 1 | CAAGCAGAAGACGGCATACGAGATCGTGATGTGAC TGGAGTTCAGACGTGTGCTCTTCCGATC*T | 177 |
| NEB Adaptor, Index 2 | CAAGCAGAAGACGGCATACGAGATACATCGGTGAC TGGAGTTCAGACGTGTGCTCTTCCGATC*T | 178 |
| NEB Adaptor, Index 3 | CAAGCAGAAGACGGCATACGAGATGCCTAAGTGAC TGGAGTTCAGACGTGTGCTCTTCCGATC*T | 179 |
| NEB Adaptor, Index 4 | CAAGCAGAAGACGGCATACGAGATTGGTCAGTGAC TGGAGTTCAGACGTGTGCTCTTCCGATC*T | 180 |
| NEB Adaptor, Index 5 | CAAGCAGAAGACGGCATACGAGATCACTGTGTGAC TGGAGTTCAGACGTGTGCTCTTCCGATC*T | 181 |
| NEB Adaptor, Index 6 | CAAGCAGAAGACGGCATACGAGATATTGGCGTGAC TGGAGTTCAGACGTGTGCTCTTCCGATC*T | 182 |
| NEB Adaptor, Index 7 | CAAGCAGAAGACGGCATACGAGATGATCTGGTGAC TGGAGTTCAGACGTGTGCTCTTCCGATC*T | 183 |
| NEB Adaptor, Index 8 | CAAGCAGAAGACGGCATACGAGATTCAAGTGTGAC TGGAGTTCAGACGTGTGCTCTTCCGATC*T | 184 |
| NEB Adaptor, Index 9 | CAAGCAGAAGACGGCATACGAGATCTGATCGTGAC TGGAGTTCAGACGTGTGCTCTTCCGATC*T | 185 |
| NEB Adaptor, Index 10 | CAAGCAGAAGACGGCATACGAGATAAGCTAGTGA CTGGAGTTCAGACGTGTGCTCTTCCGATC*T | 186 |
| NEB Adaptor, Index 11 | CAAGCAGAAGACGGCATACGAGATGTAGCCGTGAC TGGAGTTCAGACGTGTGCTCTTCCGATC*T | 187 |
| NEB Adaptor, Index 12 | CAAGCAGAAGACGGCATACGAGATTACAAGGTGA CTGGAGTTCAGACGTGTGCTCTTCCGATC*T | 188 |
| NEBnext Universal primer | AATGATACGGCGACCACCGAGATCTACACTCTTTC CCTACACGACGCTCTTCCGATC*T | 189 |
| NEBNext adaptor for Illumina | GATCGGAAGAGCACACGTCTGAACTCCAGTC-U- ACACTCTTTCCCTACACGACGCTCTTCCGATC*T | 190 |
| 16S Fwd Primer | AGRGTTYGATYMTGGCTCAG | 191 |
| 23S Rev Primer | CGACATCGAGGTGCCAAAC | 192 |
| TruSeq Index 1 | ATCACG | 193 |
| TruSeq Index 2 | CGATGT | 194 |
| TruSeq Index 3 | TTAGGC | 195 |
| TruSeq Index 4 | TGACCA | 196 |
| TruSeq Index 5 | ACAGTG | 197 |
| TruSeq Index 6 | GCCAAT | 198 |
| TruSeq Index 7 | CAGATC | 199 |
| TruSeq Index 8 | ACTTGA | 200 |
| TruSeq Index 9 | GATCAG | 201 |
| TruSeq Index 10 | TAGCTT | 202 |
| TruSeq Index 11 | GGCTAC | 203 |
| TruSeq Index 12 | CTTGTA | 204 |
| TruSeq Index 13 | AGTCAACA | 205 |
| TruSeq Index 14 | AGTTCCGT | 206 |
| TruSeq Index 15 | ATGTCAGA | 207 |
| TruSeq Index 16 | CCGTCCCG | 208 |
| TruSeq Index 18 | GTCCGCAC | 209 |
| TruSeq Index 19 | GTGAAACG | 210 |
| TruSeq Index 20 | GTGGCCTT | 211 |
| TruSeq Index 21 | GTTTCGGA | 212 |
| TruSeq Index 22 | CGTACGTA | 213 |
| TruSeq Index 23 | GAGTGGAT | 214 |
| TruSeq Index 25 | ACTGATAT | 215 |
| TruSeq Index 27 | ATTCCTTT | 216 |
| IDT Dual Index UMI Adapter 1, i7 index and UMI | CTGATCGTNNNNNNNNN | 225 |
| IDT Dual Index UMI Adapter 2, i7 index and UMI | ACTCTCGANNNNNNNNN | 226 |
| IDT Dual Index UMI Adapter 3, i7 index and UMI | TGAGCTAGNNNNNNNNN | 227 |
| IDT Dual Index UMI Adapter 4, i7 index and UMI | GAGACGATNNNNNNNNN | 228 |
| IDT Dual Index UMI Adapter 5, i7 index and UMI | CTTGTCGANNNNNNNNN | 229 |
| IDT Dual Index UMI Adapter 6, i7 index and UMI | TTCCAAGGNNNNNNNNN | 230 |

The primer sequences may be generally complementary to (or complementary to): universal sequencing primers (such as TruSeq Universal Adapter, NEBnext Univeral primer); sequences within a test DNA sample; regions of the human genome; mRNA 3' poly-A sequences, or any known primers. For example, the primer sequences may be generally complementary to (or complementary to): 16S and 23S rRNA genes; immunoglobulin and T-cell receptor genes; coding exons; or sequences flanking and within exons in genes causatively associated with human diseases such as cancer.

The adaptor sequence may also be provided so as to incorporate one or more specific sequences into the synthesised polynucleotide. This is typically performed when preparing a target polynucleotide for sequencing. This can be achieved, for example, by the positioning of one or more such specific sequences downstream (i.e., in the 3' direction) from a primer sequence. Alternatively or in addition, one or more such specific sequences can be positioned upstream (i.e., in the 5' direction) from a primer sequence. For example, the adaptor sequence may be a double stranded polynucleotide wherein a first strand comprises a primer sequence positioned upstream from a specific sequence to be incorporated into the synthesised polynucleotide, and wherein a second strand which is complementary to (or generally complementary to) the first strand comprises a primer sequence positioned downstream from a specific sequence to be incorporated into the synthesised polynucleotide.

### Calibration sequences

The polynucleotide sequence may comprise any number of calibration sequences. In one example, the polynucleotide sequence may contain no more than 200,000, or no more than 100,000, or no more than 10,000, or no more than 1,000, or no more than 900, or no more than 800, or no more than 700, or no more than 600, or no more than 500, or no more than 400, or no more than 300, or no more than 200, or no more than 100, or no more than 90, or no more than 80, or no more than 70, or no more than 60, or no more than 50, or no more than 40, or no more than 30, or no more than 20, or no more than 10, or no more than 9, or no more than 8, or no more than 7, or no more than 6, or no more than 5, or no more than 4, or no more than 3, or no more than 2 calibration sequences.

Alternatively, the polynucleotide sequence may comprise more than 2, more than 3, more than 4, more than 5, more than 6, more than 7, more than 8, more than 9, more than 10, more than 20, more than 30, more than 40, more than 50, more than 60, more than 70, more than 80, more than 90, more than 100, more than 200, more than 300, more than 400, more than 500, more than 600, more than 700, more than 800, more than 900, more than 1000, more than 5000, more than 10,000, or more than 20,000 calibration sequences

The polynucleotide sequence may comprise between 2 and 20,000 calibration sequences. For example, the polynucleotide sequence may comprise between 2 and 10,000 calibration sequences, or between 2 and 5,000 calibration sequences, or between 2 and 1,000 calibration sequences, or between 2 and 500 calibration sequences, or between 2 and 100, or between 2 and 10 calibration sequences, or between 2 and 5 calibration sequences, or between 2 and 4 calibration sequences, or between 2 and 3 calibration sequences, or another similar range.

Each calibration sequence may be of a polynucleotide length that is independent of the length of the other calibration sequences. The calibration sequences may be of differing lengths between 21 nucleotides to 20,000 nucleotides in length. The calibration sequences may independently be no more than 20,000, or no more than 10,000, or no more than 1,000, or no more than 900, or no more than 800, or no more than 700, or no more than 600, or no more than 500, or no more than 400, or no more than 300, or no more than 200, or no more than 100, or no more than 90, or no more than 80, or no more than 70, or no more than 60, or no more than 50, or no more than 40, or no more than 30, or no more than 21, or no more than 21, or no more than 18, or no more than 15 nucleotides in length. The calibration sequences may independently be at least 20,000, or at least 10,000, or at least 1,000, or at least 900, or at least 800, or at least 700, or at least 600, or at least 500, or at least 400, or at least 300, or at least 200, or at least 100, or at least 90, or at least 80, or at least 70, or at least 60, or at least 50, or at least 40, or at least 30, or at least 21 nucleotides, or at least 18 nucleotides, or at least 15 nucleotides in length. The length of the calibration sequences may be within a range defined by any combination of the minimum and maximum values stated above. For example, the calibration sequences may be between 21 and 10,000 nucleotides in length, or between 21 and 2,000 nucleotides in length, or between 21 and 1,000 nucleotides in length, or between 21 and 900 nucleotides in length, or between 21 and 800 nucleotides in length, or between 21 and 700 nucleotides in length, or between 21 and 600 nucleotides in length, or between 21 and 500 nucleotides in length, or between 21 and 400 nucleotides in length, or between 21 and 300 nucleotides in length, or between 21 and 200 nucleotides in length, or between 21 and 100 nucleotides in length. In one example, the calibration sequences may be between about 200 and about 1600 nucleotides in length. In another example, the calibration sequences may be between about 600 and about 1600 nucleotides in length.

Any calibration sequence present in the polynucleotide sequence may be present in only one copy. In one example, all calibration sequences present in the polynucleotide sequence may be present in only one copy. Alternatively, any one or more calibration sequences present in the polynucleotide sequence may be present in two or more copy numbers. For example, the polynucleotide may contain one or more calibration sequences independently present in any of 2, 3, 4, 5, 6, 7, 8, 9, 10, 12, 14, 16, 32, 64, 100, 128, 256, 512, 1000, 1024, 2048, 4096, 8192 or 10,000 copies, in any permutation.

In another example, the polynucleotide may contain a first calibration sequence that is present in one or more copies, and a second calibration sequence that is present in any of 2, 3, 4, 5, 6, 7, 8, 9, 10, 20, 50, 100, 1,000 or 10,000 times greater copy number than the first calibration sequence. In another example, one or more calibration sequence may be present in the polynucleotide sequence in more than 2, 3, 4 or 5 copies. Alternatively or in addition, any calibration sequence may be present in the polynucleotide sequence in not more than 5, 10, 20, 50, 100 or 100,000 copies. In a further example, the polynucleotide may comprise calibration sequences that are present at equal copy-number. Any two, or more than two, or all of the calibration sequences may be present at equal copy-number.

The polynucleotide sequences disclosed herein may comprise one or more calibration sequences that are repeated at a copy-number that is in known, logarithmic, integer, sequential and constant ratio to the copy number of a second calibration sequence within the polynucleotide sequence. Alternatively, the polynucleotide sequences disclosed herein may include two or more calibration sequences that are repeated at different copy-numbers within the polynucleotide sequence. In one example, the polynucleotide sequence includes calibration sequences repeated at one, two, and three copy-numbers within the polynucleotide sequence.

In one particular example, the polynucleotide sequence comprises calibration sequences, which can be denominated A, B, C, and D, which are present at 2, 2, 1 and 3 copies, respectively. Thus, in one example, the polynucleotide may comprise or consist of four calibration sequences, one of which is present only in one copy, two of which are present in two copies, and one of which is present in three copies.

The copies of a calibration sequence are identical to the original calibration sequence. It will be appreciated that a "variant" of a given calibration sequence is considered to be a different calibration sequence. Thus, for example, a polynucleotide sequence disclosed herein may comprise a first calibration sequence representing a genomic sequence of interest, and a second calibration sequence representing a variant of the first calibration sequence. The polynucleotide sequence may comprise multiple calibration sequences, each representing a variant of a genomic sequence of interest. The variants may result from any genetic change relative to a particular genomic sequence of interest.

The calibration sequences may be combined at predetermined copy-numbers into a single contiguous polynucleotide sequence.

The polynucleotides disclosed herein comprise two or more calibration sequences that are provided in an arrangement not found in any naturally occurring genome. Thus, the calibration sequences are artificially arranged within the single polynucleotide sequence. Accordingly, while the calibration sequences may be identical to naturally occurring genomic sequences, they are provided in an unnatural form through their artificial arrangement in the polynucleotides disclosed herein. This unnatural form may simply be the presentation of the calibration sequences within the polynucleotide sequence disclosed herein. It will be appreciated that the preparation of the polynucleotide sequences disclosed herein requires the generation of a polynucleotide sequence that is not found in nature.

The polynucleotide sequences disclosed herein contain two or more calibration sequences that are artificially arranged to form a linear sequence that is not otherwise present in natural genomes or transcriptomes. Thus, the two or more calibration sequences may be joined or ligated together to form a single polynucleotide sequence. The polynucleotide sequence may comprise calibration sequences that are arranged consecutively and linearly, with intervening spacer sequences. Alternatively or in addition, the polynucleotide sequence may comprise calibration sequences that are contiguously arranged in a linear order, (i.e., without intervening spacer sequences).

The polynucleotide sequences disclosed herein may comprise at least two copies of a single calibration sequence, which copies are arranged within the polynucleotide sequence so that the two copies are separated by other calibration sequences, and/or by multiple copies of other calibration sequences.

The polynucleotide sequences disclosed herein may comprise additional sequence elements. These additional sequence elements may be artificial (i.e., not present in a naturally occurring genome). For example, the polynucleotide sequences may comprise spacer and/or flanking sequences that occur before, and/or between, and/or after any one or more calibration sequences. The spacer sequence may be more than 5, more than 6, more than 7, more than 8, more than 9, more than 10, more than 12, more than 15, more than 18, more than 21, more than 30, more than 40, more than 50, more than 100, more than 200, more than 500, more than 600, more than 1000, more than 2000, more than 10,000 nucleotides in length. The spacer may be no more than 5, no more than 6, no more than 7, no more than 8, no more than 9, no more than 10, no more than 12, no more than 15, no more than 15, no more than 18, no more than 21, no more than 30, no more than 40, no more than 50, no more than 100, no more than 200, no more than 500, no more than 600, no more than 1000, no more than 2000, no more than 10,000 nucleotides in length. Non-limiting examples of spacer sequences are set out in SEQ ID NOs: 41-45. It will be appreciated that many alternative spacer sequences can be used.

The flanking sequences may be more than 5, more than 6, more than 7, more than 8, more than 9, more than 10, more than 12, more than 15, more than 18, more than 21, more than 30, more than 40, more than 50, more than 100, more than 200, more than 500, more than 600, more than 1000, more than 2000, more than 10,000 nucleotides in length. The spacer may be no more than 5, no more than 6, no more than 7, no more than 8, no more than 9, no more than 10, no more than 12, no more than 15, no more than 15, no more than 18, no more than 21, no more than 30, no more than 40, no more than 50, no more than 100, no more than 200, no more than 500, no more than 600, no more than 1000, no more than 2000, no more than 10,000 nucleotides in length. In one example, the polynucleotide sequence comprises spacer and flanking sequences that occur before, between and after one or more calibration sequences, or each calibration sequence.

In another example, the polynucleotide sequence comprises two or more copies of a calibration sequence that are separated by a spacer sequence. The spacer sequence may be artificial (i.e., not present in a naturally occurring genome). In one example, the spacer sequence is a randomized genetic sequence, which may be curated to remove or reduce sequence homology with any publicly available, naturally occurring genome sequence. The polynucleotide sequences disclosed herein may comprise one or more spacer sequences that are contiguous with one or more calibration sequences. Alternatively or in addition, the polynucleotide sequences may include spacer and flanking sequences that mitigate edge-effects that may occur during library preparation.

The polynucleotide sequences may include spacer and/or flanking sequences that can be computationally identified, filtered and/or removed from a next-generation sequencing library. In one example, the polynucleotide sequence comprises spacer and flanking sequences that, when sequenced, can be computationally aligned to a synthetic sequence or reference synthetic chromosome, or to an artificial sequence or artificial chromosome (e.g., as described in WO 2016/094947).

The polynucleotide sequences disclosed herein may include one or more spacer and/or flanking sequences that are wholly or partially complementary to primer sequences used during polymerase chain amplification. For example, the polynucleotide sequences can include spacer and/or flanking sequences that contain recognition site sequences for restriction endonuclease digestion. Alternatively or in addition, the polynucleotide sequences can include spacer and flanking sequences that contain any one or more: promoter sequences, transcription initiation sites, or poly-A tract. The polynucleotide sequence may include calibration sequences that are operably linked to one or more primer sites, restriction sites, promoter sequences, transcription initiation sites, and/or poly-A tracts. In one example, the polynucleotide sequence includes a first calibration sequence that is operably linked to one or more primer sites, restriction sites, promoter sequences, transcription initiation sites, and/or poly-A tracts, and a second calibration sequences that is operably linked to one or more different primer sites, restriction sites, promoter sequences, transcription initiation sites, and/or poly-A tracts.

It will be appreciated that two or more polynucleotide sequences as disclosed herein can be combined to form a composition. The composition can comprise two or more different polynucleotide sequences. The two or more different polynucleotide sequences may each comprise two or more different calibration sequences. Alternatively, the composition may comprise two or more different polynucleotide sequences, each comprising an identical set of calibration sequences.

In the compositions disclosed herein, one or more calibration sequences may be present in two or more polynucleotide sequences in the composition. In one example, a single calibration sequence is present in two or more polynucleotide sequences at a different copy number.

In another example, two or more polynucleotide sequences may be combined at different concentrations to form a mixture. The polynucleotide sequences may be combined at predetermined concentrations to form a mixture. In some compositions, two or more polynucleotide sequences are combined at known absolute concentrations to form a mixture. In some compositions, two or more polynucleotide sequences are combined at known relative concentrations to form a mixture. In some compositions, two or more polynucleotide sequences are combined at equimolar concentrations to form a mixture. The relative concentrations may be predetermined so as to provide relative amounts of each calibration sequence in the composition at any of the relative fold differences described herein in respect of the copy numbers of each calibration sequence.

As described herein, the adaptor sequences of the disclosure comprise a calibration sequence, which is to be incorporated into the transcribed polynucleotide. The calibration sequence (also referred to herein as a "standard", "reference standard" or "control") can be of any suitable length and/or nucleotide composition to serve as a reference standard against which the accuracy of the polynucleotide sequencing and/or quantitation method can be measured. The calibration sequence is a ground-truth control of known sequence and quantity against which the accuracy and/or quantitation of polynucleotide sequencing can be evaluated. The calibration sequence can be of any suitable length and/or nucleotide composition such that it does not impede the performance of synthesis, sequencing and/or alignment during the method.

In the context of adaptors of the disclosure, the calibration sequence may be, for example, at least 8, 9, 10, 11, 12, 13, 14, 15, 16, 17, 18, 19, 20, 30, 40, 50, 60, 70, 80, 90, 100, 200, 500, 1,000 nucleotides in length. Thus, the calibration sequence may be, for example, at least about 8, at least about 10, about 15, about 20, about 25, about 30, about 35, about 40, about 45, about 50, about 60, about 70, about 80, about 90, about 100, about 150, about 200, about 300, about 400, about 500, or about 1,000 nucleotides in length. In another example, the calibration sequence may be about 10, about 15, about 20, about 25, about 30, about 35, about 40, about 45, about 50, about 60, about 70, about 80, about 90, about 100, about 150, about 200, about 300, about 400, about 500, or about 1,000 nucleotides in length. Alternatively or in addition, the calibration sequence may be, for example, less than 20,000 10,000, 5,000, 2,000, 1,000, 900, 800, 700, 600, 500, 400, 300, 200, or 100 nucleotides in length. Thus, the calibration sequence may be, for example, between 8 and 10,000 nucleotides long. Shorter calibration sequences (e.g., between 8 and 100 nucleotides in length, such as between 8 and 50 nucleotides in length, such as between 8 and 20 nucleotides in length, such as about 20 nucleotides in length) may be preferred for flow cell-based sequencing methods. Longer calibration sequences (e.g., between 8 and 10,000 nucleotides in length, such as between 12 and 10,000 nucleotides in length, such as between 20 and 10,000 nucleotides in length, such as between 50 and 10,000 nucleotides in length, such as between 100 and 10,000 nucleotides in length) may be preferred for nanopore-based sequencing methods, which typically comprise the sequencing of longer polynucleotides.

The calibration sequence may constitute any proportion of the adaptor sequence. For example, the calibration sequence may constitute at least 0.0001, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 0.95, 0.99, 0.999, 0.9999 fraction of the length of the adaptor sequence. In another example, the calibration sequence may constitute about 0.1*%*, about 1*%*, about 2*%*, about 3*%*, about 4*%*, about 5*%*, about 6*%*, about 7*%*, about 8*%*, about 9*%*, about 10*%*, about 11*%*, about 12*%*, about 13*%*, about 14*%*, about 15*%*, about 16*%*, about 17*%*, about 18*%*, about 19*%*, about 20*%*, about 30*%*, about 40*%*, about 50*%*, about 60*%*, about 70*%*, about 80*%*, about 90*%*, about 95*%*, about 99*%*, about 99.9*%* of the polynucleotide adaptor sequence length. In another example, the calibration sequence may constitute no more than 1*%*, 2*%*, 3*%*, 4*%*, 5%, 6*%*, 7*%*, 8*%*, 9*%*, 10*%*, 11*%*, 12*%*, 13*%*, 14*%*, 15*%*, 16*%*, 17*%*, 18*%*, 19*%*, 20*%*, 30*%*, 40*%*, 50*%*, 60*%*, 70*%*, 80*%*, 90*%*, 95*%*, 99*%* or 99.9*%* of the polynucleotide adaptor sequence length.

The adaptor sequence may comprise any number of calibration sequences. In one example, the adaptor sequence may contain no more than 1,000, or no more than 900, or no more than 800, or no more than 700, or no more than 600, or no more than 500, or no more than 400, or no more than 300, or no more than 200, or no more than 100, or no more than 90, or no more than 80, or no more than 70, or no more than 60, or no more than 50, or no more than 40, or no more than 30, or no more than 20, or no more than 10, or no more than 9, or no more than 8, or no more than 7, or no more than 6, or no more than 5, or no more than 4, or no more than 3, or no more than 2, or no more than 1 calibration sequences.

The polynucleotide sequence may comprise between 2 and 1,000 calibration sequences, or between 2 and 500 calibration sequences, or between 2 and 100 calibration sequences, or between 2 and 50 calibration sequences, or between 2 and 10 calibration sequences, or between 2 and 8 calibration sequences, or another similar range.

In an adaptor sequence comprising two or more calibration sequences, or in a composition of two or more adaptor sequences comprising two or more calibration sequences (whether in the same adaptor sequence or not), each calibration sequence may be of a polynucleotide length that is independent of the length of the other calibration sequences. The calibration sequences may be of differing lengths between 8 nucleotides to 10,000 nucleotides in length. The calibration sequences may independently be no more than 10,000, or no more than 1,000, or no more than 900, or no more than 800, or no more than 700, or no more than 600, or no more than 500, or no more than 400, or no more than 300, or no more than 200, or no more than 100, or no more than 90, or no more than 80, or no more than 70, or no more than 60, or no more than 50, or no more than 40, or no more than 30, or no more than 21 nucleotides in length. Alternatively or in addition, the calibration sequences may independently be at least 9,900, or at least 1,000, or at least 900, or at least 800, or at least 700, or at least 600, or at least 500, or at least 400, or at least 300, or at least 200, or at least 100, or at least 90, or at least 80, or at least 70, or at least 60, or at least 50, or at least 40, or at least 30, or at least 21, or at least 20, or at least 15, or at least 10, or at least 8 nucleotides in length. The length of the calibration sequences may be within a range defined by any combination of the minimum and maximum values stated above. For example, the calibration sequences may be between 8 and 10,000 nucleotides in length, or between 21 and 2,000 nucleotides in length, or between 21 and 1,000 nucleotides in length, or between 21 and 900 nucleotides in length, or between 21 and 800 nucleotides in length, or between 21 and 700 nucleotides in length, or between 21 and 600 nucleotides in length, or between 21 and 500 nucleotides in length, or between 21 and 400 nucleotides in length, or between 21 and 300 nucleotides in length, or between 21 and 200 nucleotides in length, or between 21 and 100 nucleotides in length. In one example, the calibration sequences may be between about 8 and about 10,000 nucleotides in length. In one example, the calibration sequences may be between about 8 and about 10,000 nucleotides in length. In one example, the calibration sequences may be between about 10 and about 10,000 nucleotides in length. In another example, the calibration sequences may be between about 20 and about 1000 nucleotides in length.

Any calibration sequence present in the adaptor may be present in only one copy. In one example, all calibration sequences present in the adaptor may be present in only one copy. Alternatively, any one or more calibration sequences present in the adaptor may be present in two or more copy numbers. For example, the adaptor may contain one or more calibration sequences independently present in any of 2, 3, 4, 5, 6, 7, 8, 9, 10, 12, 14, 16, 32, 64, 100, 128, 256, 512, 1000, 1024, 2048, 4096, 8192 or 10,000 copies, in any permutation.

In another example, the adaptor may contain a first calibration sequence that is present in one or more copies, and a second calibration sequence that is present in any of 2, 3, 4, 5, 6, 7, 8, 9, 10, 20, 50, 100, 1,000 or 10,000 times greater copy number than the first calibration sequence. In another example, one or more calibration sequence may be present in the adaptor in more than 2, 3, 4 or 5 copies. Alternatively or in addition, any calibration sequence may be present in the adaptor in not more than 5, 10, 20, 50, 100 or 100,000 copies. In a further example, the polynucleotide may comprise calibration sequences that are present at equal copy-number. Any two, or more than two, or all of the calibration sequences may be present at equal copy-number.

In another example, one adaptor may contain a first calibration sequence that is present in one or more copies, and a second adaptor may contain a second calibration sequence that is present in any of 2, 3, 4, 5, 6, 7, 8, 9, 10, 20, 50, 100, 1,000 or 10,000 times greater copy number than the first calibration sequence.

The adaptor sequences disclosed herein (which may be provided in a composition and/or mixture comprising one or more adaptors, each having one or more calibration sequences present in one or more copy numbers, independently), may comprise one or more calibration sequences that are repeated at a copy-number that is in known, logarithmic, integer, sequential and constant ratio to the copy number of a second calibration sequence within the adaptor sequence, or within a different adaptor in a composition of multiple adaptors. In one example, the adaptor sequence includes calibration sequences repeated at one, two, and three copy-numbers within the adaptor sequence, or in the aggregate across multiple adaptors present in a mixture. Thus, a composition or a mixture of adaptors disclosed herein may comprise one or more calibration sequences that are repeated at a copy-number that is in known, logarithmic, integer, sequential and constant ratio to the copy number of a second calibration sequence within a different adaptor in the composition or mixture.

In one particular example, the adaptor sequence comprises calibration sequences, which can be denominated A, B, C, and D, which are present at 2, 2, 1 and 3 copies, respectively. Thus, in one example, the adaptor may comprise or consist of four calibration sequences, one of which is present only in one copy, two of which are present in two copies, and one of which is present in three copies.

The copies of a calibration sequence are identical to the original calibration sequence. It will be appreciated that a "variant" of a given calibration sequence is considered to be a different calibration sequence. Thus, for example, an adaptor sequence (or composition of adaptor sequences, or mixture) disclosed herein may comprise a first calibration sequence representing a genomic sequence of interest, and a second calibration sequence representing a variant of the first calibration sequence. The adaptor sequence may comprise multiple calibration sequences, each representing a variant of a genomic sequence of interest. The variants may result from any genetic change relative to a particular genomic sequence of interest.

The calibration sequences may be combined at predetermined copy-numbers into a single contiguous polynucleotide sequence. That single contiguous polynucleotide sequence may constitute the adaptor sequence.

The adaptor sequence disclosed herein may comprise a calibration sequence which is a naturally occurring polynucleotide sequence. In addition, the adaptor sequence disclosed herein may comprise two or more calibration sequences that are provided in an arrangement not found in any naturally occurring genome. Thus, the calibration sequences may be artificially arranged within the adaptor sequence. Accordingly, while the calibration sequences may be identical to naturally occurring genomic sequences, they may be provided in an unnatural form through their artificial arrangement in the adaptors disclosed herein. This unnatural form may simply be the presentation of the calibration sequences within the adaptor sequence disclosed herein. It will be appreciated that the preparation of the adaptor sequences disclosed herein requires the generation of a polynucleotide sequence that is not found in nature.

The adaptor sequences disclosed herein may contain two or more calibration sequences that are artificially arranged to form a linear sequence that is not otherwise present in natural genomes or transcriptomes. Thus, the two or more calibration sequences may be joined or ligated together to form a single polynucleotide sequence. The adaptor sequence may comprise calibration sequences that are arranged consecutively and linearly, with intervening spacer sequences. Alternatively or in addition, the adaptor sequence may comprise calibration sequences that are contiguously arranged in a linear order, (i.e., without intervening spacer sequences).

The adaptor sequences disclosed herein may comprise at least two copies of a single calibration sequence, which copies are arranged within the adaptor sequence so that the two copies are separated by other calibration sequences, and/or by multiple copies of other calibration sequences.

Alternatively or in addition, the adaptor sequences disclosed herein may comprise an artificial calibration sequence. The artificial calibration sequence may have less than 100*%*, less than 99.99*%*, less than 99.9*%*, less than 99*%*, less than 90*%*, less than 80*%*, less than 70*%*, less than 60*%*, less than 50*%*, less than 40*%*, less than 30*%*, less than 20*%*, less than 10*%*, or less than 1*%* sequence identity with sequences selected from natural exons, genes, alleles, genome or transcriptome sequences. In one example, the calibration sequence is selected from naturally occurring genetic sequences that are modified with one or more genetic variants, nucleotide transversions, transitions, insertions and deletions that enable the calibration sequence to be distinguished from the original genetic sequences. In one example, the calibration sequence is selected from naturally occurring genetic sequences that are modified with one or more artificial nucleotide transversions, transitions, insertions and deletions that enable the calibration sequence to be distinguished from the original genetic sequences. In another example, the calibration sequence may be selected from naturally occurring genetic sequences which are then is reversed, shuffled, and/or modified to enable the calibration sequence to be distinguished from the original genetic sequence. The calibration sequences can therefore comprise artificial sequence representations of one or more natural genetic sequences, wherein natural genetic sequences include genetic variation, genes or repeat sequences.

The calibration sequence disclosed herein may comprise guanine or cytosine nucleotides for greater than 10*%*, 20*%*, 30*%*, 40*%*, 50*%*, 60*%*, 70*%*, 80*%* or 90*%* of its length. Alternatively or in addition, the calibration sequence may comprise adenine or thymine nucleotides for greater than 10*%*, 20*%*, 30*%*, 40*%*, 50*%*, 60*%*, 70*%*, 80*%* or 90*%* of its length. Alternatively or in addition, the calibration sequence may include a single-nucleotide repeat sequence greater than 2, 3, 4, 5, 6, 7, 8, 9, 10, 11, 12, 13, 14, 15, 16, 17, 18, 19 or 20 nucleotides in length. Alternatively or in addition, the calibration sequence may include a dinucleotide repeat sequence greater than 2, 4, 6, 8, 10, 12, 14, 16, 18, or 20 nucleotides in length. Alternatively or in addition, the calibration sequence may include a tri-nucleotide repeat sequence greater than 3, 6, 9, 12, 15, 18, or 21 nucleotides in length.

The adaptor sequences disclosed herein may comprise additional sequence elements. These additional sequence elements may be artificial (i.e., not present in a naturally occurring genome). For example, the polynucleotide sequences may comprise spacer sequences that occur before, and/or between, and/or after any one or more calibration sequences. The spacer sequence may be at least 1, at least 2, at least 3, at least 4, at least 5, at least 6, at least 7, at least 8, at least 9, at least 10, at least 20, at least 50, at least 100 nucleotides in length. Alternatively or in addition, the spacer sequence may be no more than 6, no more than 8, no more than 10, no more than 20, no more than 50, no more than 100 nucleotides in length. In one example, the adaptor sequence comprises spacer sequences that occur before, between and after one or more calibration sequences, or each calibration sequence.

In another example, the adaptor sequence comprises two or more copies of a calibration sequence that are separated by a spacer sequence. The spacer sequence may be artificial (i.e., not present in a naturally occurring genome). In one example, the spacer sequence is a randomized genetic sequence, which may be curated to remove or reduce sequence homology with any publicly available, naturally occurring genome sequence. The adaptor sequences disclosed herein may comprise one or more spacer sequences that are contiguous with one or more calibration sequences. Alternatively or in addition, the adaptor sequences may include spacer sequences that mitigate edge-effects that may occur during library preparation.

The adaptor sequences may include spacer and/or flanking sequences that can be computationally identified, filtered and/or removed from a next-generation sequencing library. In one example, the adaptor sequence comprises spacer and flanking sequences that, when sequenced, can be computationally aligned to a synthetic sequence or reference synthetic chromosome, or to an artificial sequence or artificial chromosome (e.g., as described in WO 2016/094947).

The adaptor sequences disclosed herein may include spacer and/or flanking sequences that contain recognition site sequences for restriction endonuclease digestion, or ligation. Alternatively or in addition, the adaptor sequences can include spacer and flanking sequences that contain any one or more: promoter sequences, transcription initiation sites, or poly-A tract. The adaptor sequence may include calibration sequences that are operably linked to one or more primer sites, restriction sites, promoter sequences, transcription initiation sites, and/or poly-A tracts. In one example, the adaptor sequence includes a first calibration sequence that is operably linked to one or more primer sites, restriction sites, promoter sequences, transcription initiation sites, and/or poly-A tracts, and a second calibration sequences that is operably linked to one or more different primer sites, restriction sites, promoter sequences, transcription initiation sites, and/or poly-A tracts.

The adaptor sequence may also comprise any one or more of a UMI, a sequence complementary to a flow-cell DNA sequence, a cell barcode sequence, and an index sequence. Alternatively, the adaptor sequence may not include any one or more of a UMI, a sequence complementary to a flow-cell DNA sequence, a cell barcode sequence, and an index sequence.

A unique molecular identifier (UMI) sequence is a unique artificial sequence that indicates whether a sequence read is a duplication of another sequence read (which can occur during PCR amplification during library preparation). A UMI sequence is typcially not known prior to sequencing, but is made by the incorporation of random nucleotides during the synthesis of the adaptor (the random and unknown UMI sequence is typically indicated by a NNNNN annotation). As a result of their random synthesis, the UMIs are present uniquely within a composition, and are not at known quantities within the mixture. UMIs do not share any homology with other UMI sequences. A UMI is typically a short sequence between 4-12 nt (for short-read Illumina sequencing) and up to 36 nt (for long-read Oxford Nanopore sequencing). The UMI sequence is typically a random and artificial sequence that does not include nucleotide repeat sequences. Any single test sample DNA sequence will likely be associated with 2 identified UMIs, with the chance of a second target sample of the same sequence and associating with the same 2 identified UMIs being very improbable, and indicating that the read is a duplicate clone of the first read, typically due to PCR amplification. Each adaptor disclosed herein may include a single UMI.

The UMI sequence may include any one or more randomly-incoporated nucleotides, and may include any one or more modified nucleotides selected from the group consisting of R, Y, S, W, K, M, B, D, H, V or N nucleotides (IUPAC nucleotide code). Thus, the calibration sequence may, in alternative embodiments, not include such a UMI sequence. Accordingly, the calibration sequence may comprise only A, T, C or G nucleotides, and may not include any one or more modified nucleotides selected from the group consisting of R, Y, S, W, K, M, B, D, H, V or N nucleotides (IUPAC nucleotide code).

An index sequence is a unique artificial sequence that indicates which library a sequenced read belongs to. Each different library will include a different index sequence within the adaptor sequences used. The libraries can then be mixed together for sequencing on a single flow cell (termed "multiplexing"), with the output sequenced reads assigned to different libraries according to the presence of the index sequence. An index sequence is typically a between 6-8 nucleotides (e.g., for short-read Illumina sequencing) and 36 nucleotides (e.g., for long-read Oxford Nanopore sequencing) and a random and artificial sequence that does not include nucleotide repeat sequences. The adaptor sequence may also include a 'cell-barcode' sequence for single cell sequencing applications. This comprises a short (e.g. 26 nucleotide) sequence that indicates the association of a sequenced read with an individual cell, well or droplet. Typically, index and cell barcode sequences are random sequences.

The adaptor sequence may form a bridge between a target polynucleotide and a polynucleotide that is anchored to a surface, such as the surface of a flow-cell, or a polynucleotide that is attached to a protein. The flow-cell may comprise a solid surface with attached oligonucleotides. The adaptor sequences may contain primer sequences that are complementary to the oligonucleotide sequences that are attached to the surface. DNA fragments that comprise the test sample DNA sequence and the adaptor sequence can thereby hybridise to the oligonucleotide sequences that are attached to the surface.

### Y-shaped adaptors

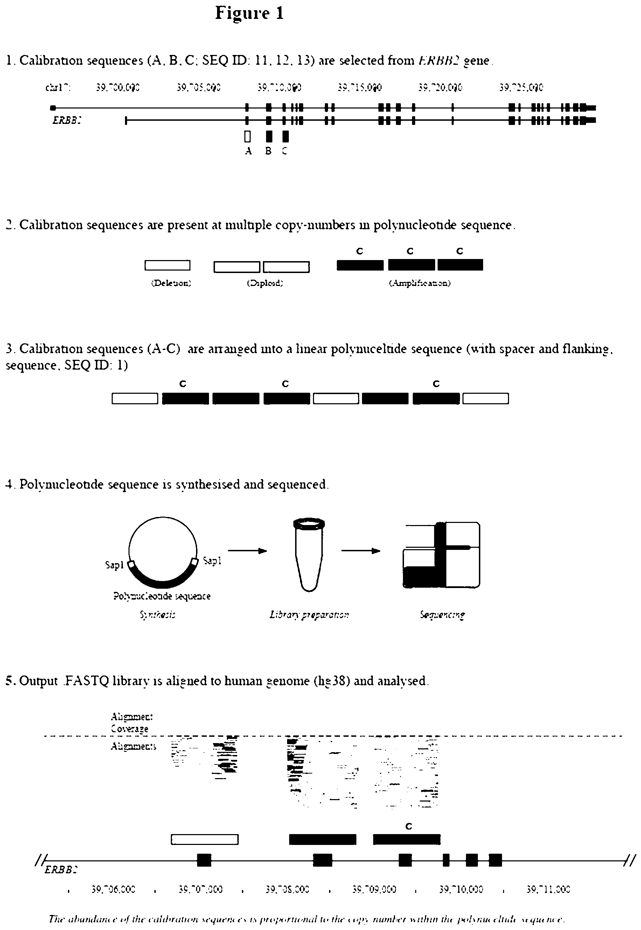

The adaptor sequences disclosed herein may be Y-shaped adaptors. Y-shaped adaptors comprise two generally complementary polynucleotide sequences annealed together, wherein one end of the annealed molecule comprises complementary polynucleotides and the other end of the annealed molecule comprises non-complementary polynucleotides. Thus, for example, a Y-shaped adaptor can be formed by annealing two polynucleotide adaptor sequences, wherein the 3' end of the first polynucleotide adaptor sequence is complementary to the 5' end of the second polynucleotide sequence, whilst the 5' end of the first polynucleotide adaptor sequence is non-complementary to the 3' end of the second polynucleotide adaptor sequence. When the two polynucleotide sequences hybridize and anneal, they form a Y-shaped molecular structure. Figure 1 illustrates non-limiting examples of Y-shaped adaptors. In one example, the Y-shaped adaptors disclosed herein may comprise two complementary primer sequences in the annealed portion of the Y (the stem of the Y), and the two non-annealed portions (the prongs of the Y) comprise non-complementary sequences. Those non-complementary sequences may be primer sequences or may not be primer sequences.

### Relative position of primer and calibration sequences within adaptors

The calibration sequence may be located at the 5' end of the polynucleotide adaptor sequence. Alternatively, the calibration sequence may be located at the 3' end of the polynucleotide adaptor sequence. In one example, one polynucleotide includes a calibration sequence that ends at the 3' end of the polynucleotide adaptor sequence, whilst a second polynucleotide adaptor sequence includes a second calibration sequence that starts at the 5' end of the polynucleotide adaptor sequence. In such an example, the second calibration sequence can be the reverse complement of the first calibration sequence. This results in two calibration sequences hybridizing at the same end of a double-stranded polynucleotide.

In any of the adaptors disclosed herein, the calibration sequence in one adaptor may be the reverse complement of a second calibration sequence in a second adaptor.

In one example, the calibration sequence is located 3' downstream from complementary sequences between two polynucleotide adaptor sequences that anneal to form a Y-shaped adaptor. In another example, the calibration sequence is located 5' upstream to complementary sequences between the two polynucleotide adaptor sequences that anneal to form a Y-shaped adaptor.

In one example, a polynucleotide adaptor sequence comprises one or more calibration sequences 3' downstream from one or more primer sequence. In another example, the calibration sequence is located 3' downstream from complementary sequences to synthetic, universal, P5 or P7 oligonucleotide sequences (see Table 1). In another example, the calibration sequence is located 3' downstream from sequences that bind complementary DNA oligonucleotides that initiate DNA synthesis (see Table 1).

In one example, a polynucleotide adaptor sequence comprises one or more calibration sequences upstream from one or more primer sequence. In one example, the polynucleotide adaptor sequence includes a primer sequence that hybridizes to a target DNA site to initiate DNA synthesis, wherein the resulting amplified DNA fragments will comprise single polynucleotide sequences that comprise the polynucleotide adaptor sequence upstream from the target DNA sequence. In one example, the calibration sequence is located 3' downstream of a primer sequence that is complementary to an oligonucleotide attached to the surface of a flow cell.

In another example, the calibration sequence is located 5' upstream of a complementary sequence to a synthetic, universal, oligonucleotide sequence (see Table 1). In another example, the calibration sequence is located 3' downstream from a sequence that binds complementary DNA oligonucleotides that initiate DNA synthesis (see Table 1).

In another example, the calibration sequence is located 5' upstream from a sequence that binds a complementary target polynucleotide sequence within a test DNA sample. In another example, the calibration sequence is located 5' upstream from a sequence that binds a complementary target polynucleotide sequence within the human genome sequence. In another example, the calibration sequence is located 5' upstream from a sequence that binds a complementary target polynucleotide sequence within a human gene exon and/or intron.

Reverse transcription requires a short DNA oligonucleotide primer that hybridizes to complementary target RNA sequences, and initiates complementary DNA (cDNA) synthesis. In one example of the present disclosure, the adaptor sequence comprises one or more calibration sequences and one or more primer sequences that are complementary to target RNA sequences. Such adaptors can be used in a method of reverse transcription. In this example, the polynucleotide adaptor sequence used in the method of reverse transcription includes a calibration sequence and a sequence complementary to the RNA target. The hybridization of the polynucleotide adaptor sequence to the target RNA site will initiate synthesis of a cDNA molecule comprising the polynucleotide adaptor sequence at the 5' end of the cDNA molecule, followed by the target RNA sequence. Thus, reverse transcription synthesis can generate a cDNA fragment that comprises the polynucleotide adaptor sequence and the target RNA sequence.

In one example, a polynucleotide adaptor sequence includes a calibration sequence wherein the calibration sequence is upstream from a primer sequence that contains more than 3, more than 8, more than 10, more than 15, or more than 20 contiguous thymine nucleotides. In one example, a polynucleotide adaptor sequence includes a calibration sequence wherein the calibration sequence is upstream from a primer sequence that contains more than 3, more than 8, more than 10, more than 15, or more than 20 contiguous uracil nucleotides. In one example, a polynucleotide adaptor sequence includes a calibration sequence wherein the calibration sequence is downstream from a sequence comprising more than 3, 8, 10, 15, or 20 contiguous thymine nucleotides.

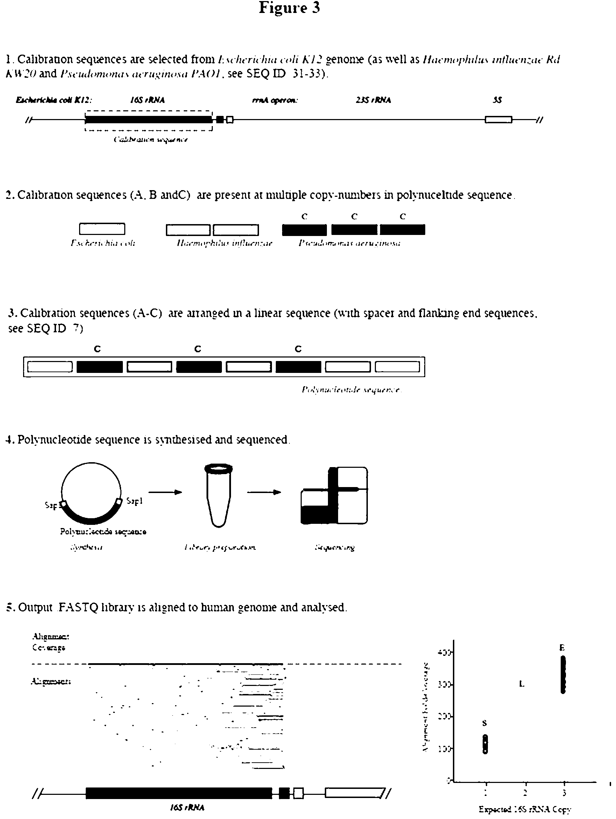

Nanopore sequencing can determine nucleotide identity by changes to electrical current as an RNA or DNA sequence passes though a nanopore. In one example of the present disclosure, the adaptor sequence comprises one or more calibration sequences. Such adaptors can be used in a method of nanopore sequencing. In this example, the adaptor sequence may be ligated to a target polynucleotide sequence (illustrated in an non-limiting example in Figure 3). In one example, the adaptor sequence may have a protein attached. In one example, the adaptor sequence may be ligated to a second DNA molecule with an attached protein. The attached protein may be an enzyme motor that initiates and controls the translocation of the DNA or RNA molecule through the nanopore. The target polynucleotide sequence and the adaptor sequence can then be loaded by the protein into the nanopore, and subsequently pass through the nanopore, with the determination of the adaptor and polynucleotide target sequence.

In one example, a polynucleotide adaptor sequence includes a calibration sequence wherein the calibration sequence ends at the 3' end of the polynucleotide adaptor sequence, and wherein the calibration sequence ends with a thymine nucleotide. In another example, a polynucleotide adaptor sequence includes a calibration sequence wherein the calibration sequence starts at the 5' start of the polynucleotide adaptor sequence, and wherein the calibration sequence starts with a thymine nucleotide. In one example, the adaptor sequence has a thymine nucleotide at the 3' termini. In another example, the adaptor comprises a 5' phosphate at the 5' end of the adaptor molecule.

### Compositions

It will be appreciated that two or more adaptor sequences as disclosed herein can be combined to form a composition. The composition can comprise two or more different adaptor sequences. The two or more different adaptor sequences may each comprise two or more different calibration sequences. Alternatively, the composition may comprise two or more different adaptor sequences, each comprising an identical set of calibration sequences.

In the compositions disclosed herein, one or more calibration sequences may be present in two or more adaptor sequences in the composition. In one example, a single calibration sequence is present in two or more adaptor sequences at a different copy number.

In another example, two or more adaptor sequences may be combined at different concentrations to form a mixture. The adaptor sequences may be combined at predetermined concentrations to form a mixture. In some compositions, two or more adaptor sequences are combined at known absolute concentrations to form a mixture. In some compositions, two or more adaptor sequences are combined at known relative concentrations to form a mixture. In some compositions, two or more adaptor sequences are combined at equimolar concentrations to form a mixture. The relative concentrations may be predetermined so as to provide relative amounts of each calibration sequence in the composition at any of the relative fold differences described herein in respect of the copy numbers of each calibration sequence. For example, the adaptor may contain one or more calibration sequences present in concentrations that are 2, 3, 4, 5, 6, 7, 8, 9, 10, 12, 14, 16, 32, 64, 100, 128, 256, 512, 1000, 1024, 2048, 4096, 8192 or 10,000 times greater than the concentration of one or more other calibration sequences in the composition.

### Representation of natural genetic features.

The calibration sequences described herein can be based on any particular sequence of interest present in a naturally occurring genome. For example, the calibration sequences can be based on any particular sequence of interest present in the latest publicly available human genome sequence (currently GRCh38.p13 (Genome Reference Consortium Human Build 38 patch release 13, Homo sapiens (human), submitted to the NCBI on 28 February 2019 by the Genome Reference Consortium and identified by the GenBank assembly accession number GCA_000001405.28 (latest))). Thus, the calibration sequences can be based on any particular sequence of interest present in the human genome sequence GRCh38.p13. The calibration sequences can be based on any particular sequence of interest present in any other publicly available genome sequence, such as any mammalian, vertebrate, animal, microbial, bacterial, viral, phage, organelle, prokaryotic or eukaryotic genome. Two non-limiting examples of such publicly available genome sequences include the hepatitis B genome (NC_003977.2) and the HIV genome (NC_001802.1). It will be readily apparent that many such publicly available genome sequences exist and can be used to generate calibration sequences as described herein.

The polynucleotide sequences disclosed herein may comprise at least one calibration sequence that is a naturally occurring polynucleotide sequence. The calibration sequence can be based in part or whole on a naturally occurring sequence. Thus, the calibration sequences may be based on one or more sequences observed in a natural genome or transcriptome sequence. Thus, the polynucleotide sequences disclosed herein may comprise calibration sequences that replicate genetic sequences found in nature.

The polynucleotide sequences may comprise calibration sequences that represent genetic sequences from two or more different exons, genes, introns, chromosomes or genomes.

The calibration sequences may be 100*%* identical to a naturally occurring genomic sequence, across the entire length of the calibration sequence. However, in other examples, the calibration sequences comprised within adaptors of the disclosure may have less than 100*%* identity (or homology) to naturally occurring genetic sequences. For example, the adaptor sequence may have more than 1*%*, 10*%*, 20*%*, 30*%*, 40*%*, 50*%*, 60*%*, 70*%*, 80*%*, 90*%*, 99*%*, 99.99*%*, 99.9999*%*, or 99.999999*%* identity (or homology) to sequences observed in a natural genome or transcriptome sequences. In one embodiment, the adaptor sequence comprises one or more calibration sequence with more than 1*%*, 10*%*, 20*%*, 30*%*, 40*%*, 50*%*, 60*%*, 70*%*, 80*%*, 90*%*, 99*%*, 99.99*%*, 99.9999*%*, or 99.999999*%* identity (homology) to sequences observed in a natural genome or transcriptome sequences. In one embodiment, the adaptor sequence comprises two or more calibration sequence with more than 1*%*, 10*%*, 20*%*, 30*%*, 40*%*, 50*%*, 60*%*, 70*%*, 80*%*, 90*%*, 99*%*, 99.99*%*, 99.9999*%*, or 99.999999*%* homology to sequences observed in a natural genome or transcriptome sequences.

The polynucleotide sequences disclosed herein can comprise two or more calibration sequences that replicate two or more sequences observed in natural genome or transcriptome sequences that are joined into a single contiguous RNA or DNA sequence.

In one example, the polynucleotide sequence described herein (including the ladders and adaptors of the disclosure) may include a first calibration sequence that replicates a genetic sequence selected from a first genome, and a second calibration sequence that replicates a genetic sequence selected from a second genome. In another example, the polynucleotide sequence described herein (including the ladders and adaptors of the disclosure) may include a first calibration sequence that replicates a genetic sequence selected from a first chromosome sequence, and a second calibration sequence that replicates a genetic sequence selected from a second chromosome sequence. In other examples, the polynucleotide sequences described herein (including the ladders and adaptors of the disclosure) may comprise calibration sequences that replicate sequences selected from any one or more of: eukaryotic, prokaryotic or viral genomes, vector, organelle or plasmid genome or transcriptome sequences.

The polynucleotide sequence may comprise calibration sequences representing features of naturally occurring genomic sequences. Such features may be selected from, for example and without limitation, human microsatellites, T-cell and immunoglobulin gene sequences, and CpG methylated sequences. The polynucleotide sequence may comprise calibration sequences that are selected from sequences within genetic variants, wherein genetic variants include, for example, nucleotide transversions, nucleotide transitions, insertions, deletions, inversions, repeat expansions and/or structural variants. In another example, the polynucleotide sequence may comprise a calibration sequence that is selected from genetic sequences with copy-number variation, amplifications, deletions, microdeletions, or chromosomal aneuploidy. The polynucleotide sequences disclosed herein may comprise one or more calibration sequences that are selected from coding, intronic and noncoding sequences selected from a genome. In one example, the polynucleotide sequence comprises calibration sequences that are selected from gene sequences. In another example, the polynucleotide sequence comprises calibration sequences that are selected from messenger RNA sequences, fusion-gene sequences, microRNAs, long noncoding RNAs, and other species of RNA transcripts. Such features may be selected from, for example and without limitation, human microsatellites, methylated sequences, and T-cell and immunoglobulin gene sequences. In another example, the polynucleotide sequence comprises calibration sequences that are selected from common, difficult, pathogenic and/or benign genetic variant sequences. In another example, the polynucleotide sequence comprises calibration sequences that are selected from a publicly available reference genome assembly sequence. In another example, the polynucleotide sequence comprises calibration sequences that are selected from a human genome assembly sequence. In another example, the polynucleotide sequence comprises calibration sequences that are selected from human genetic sequences. In another example, the polynucleotide sequence comprises calibration sequences that are selected from genetic sequences with copy-number variation, amplifications, deletions, microdeletions, or chromosomal aneuploidy. The adaptor sequences disclosed herein may comprise at least one calibration sequence that is a naturally occurring polynucleotide sequence. The calibration sequence can be based in part or whole on a naturally occurring sequence. Thus, the calibration sequences may be based on one or more sequences observed in a natural genome or transcriptome sequence. Thus, the polynucleotide sequences disclosed herein may comprise calibration sequences that replicate genetic sequences found in nature. The adaptor sequences may comprise calibration sequences that represent genetic sequences from exons, genes, introns, chromosomes or genomes. The adaptor sequences may comprise calibration sequences that represent genetic sequences from two or more different exons, genes, introns, chromosomes or genomes.

In another example, the polynucleotide sequence (e.g., adaptor) comprises a calibration sequence that is selected from a publicly available reference genome assembly sequence. In another example, the polynucleotide sequence (e.g., adaptor) comprises a calibration sequence that is selected from a human genome assembly sequence. In another example, the polynucleotide sequence (e.g., adaptor) comprises a calibration sequence that is selected from human genetic sequences.

In one example, the polynucleotide sequence (e.g., adaptor) comprises at least one calibration sequence that represents a naturally occurring first polynucleotide sequence and at least one other calibration sequence that represents a variant sequence of the first calibration sequence.

Thus, the polynucleotide sequences disclosed herein may comprise calibration sequences that replicate genetic sequences containing genetic variants. In one example, the polynucleotide sequence includes a first calibration sequence selected from a genome sequence, and a second calibration sequence that represents a variant sequence to the first sequence. In one example, the polynucleotide sequence includes two or more calibration sequences, wherein the first calibration sequence is selected from a human genome reference assembly sequence, and a second calibration sequence represents a variant of the first calibration sequence. In one example, the polynucleotide sequence comprises first and second calibration sequences that represent different allele sequences selected from a diploid pair of human chromosomes, respectively. Thus, one calibration sequence may represent a maternal-derived sequence and another calibration sequence may represent a paternal-derived sequence.

The polynucleotide sequence may include a first calibration sequence selected from a genome sequence, and a second calibration sequence that represents a variant sequence to the first sequence, wherein the second calibration sequence is repeated at greater, lesser or the same copy-number as the first calibration sequence. For example, the polynucleotide sequence may include two or more calibration sequences that represent two or more allele sequences that are repeated at a copy-number that replicates the ratio of copy-numbers observed for a heterozygous genotype, homozygous genotype, copy-number amplifications, deletion, insertion, and/or loss-of-heterozygosity. In one example, the polynucleotide sequence comprises a first calibration sequence that represents a maternal inherited chromosome sequence, and a second calibration sequence that represents a paternal inherited chromosome sequence. In another example, the polynucleotide sequence may include a first calibration sequence that represents a first allele sequence, and a second calibration sequence that represents a second allele sequence. It will be appreciated that additional calibration sequences can be included, representing additional allele sequences.

The polynucleotide sequences disclosed herein may include a first calibration sequence that represents a first allele sequence, and a second calibration sequence that represents a second allele sequence, wherein both calibration sequences are repeated at different copy-numbers and wherein the ratio of copy-numbers replicates somatic allele frequencies observed in the sequencing of a biological sample. Any biological sample may be replicated in terms of the allele frequencies present therein, by using calibration sequences whose copy numbers are tailored accordingly. One non-limiting example of a suitable biological sample is a tumour biopsy. For example, the polynucleotide sequences may include two or more calibration sequences that are repeated at copy-numbers, wherein the ratio of copy numbers replicates 50%, 25%, 12.5%, 6.25%, 10%, 1% or 0.1% allele frequency. In addition, the polynucleotide sequences may include two or more calibration sequences that are repeated at copy-numbers, wherein the ratio of copy numbers replicates copy-number variation, amplifications, deletions, microdeletions, trisomy, and/or chromosomal aneuploidy. In one example, the polynucleotide sequence includes two or more calibration sequences that are repeated at copy-numbers, wherein the ratio of copy numbers replicates foetal and/or maternal and/or paternal genotypes observed in non-invasive prenatal sequencing.

The polynucleotide sequences disclosed herein may comprise one or calibration sequences that are selected from genetic sequences identified during the clinical diagnosis of human disease. For example, the polynucleotide sequence may comprise one or more calibration sequences that are selected from human genome sequences that are causally associated with human disease. For example, the polynucleotide sequences disclosed herein may comprise calibration sequences that are selected from genetic sequences identified during the diagnosis of cancer, inherited disease, pathogen detection and/or drug resistance. In one example, the polynucleotide sequence comprises calibration sequences that are selected from human sequences used for clinical diagnosis.

### Cancer

The sequencing of a tumor sample may detect the presence of genetic mutations which can be used to diagnose cancer. This may inform patient prognosis and treatment. However, the complexity of a tumor sample, the presence of somatic mutations at often low allele frequency, and additional technical variables during library preparation and sequencing may affect the accurate diagnosis of mutations.

The polynucleotide sequences disclosed herein may comprise calibration sequences that are selected from genetic sequences that are known to cause cancer. For example, the polynucleotide sequences may comprise calibration sequences that are selected from one or more gene or exon sequences that are causally associated with cancer. In another example, the polynucleotide sequences may comprise calibration sequences that are selected from genetic sequences from two or more exons from a single gene that is causally associated with cancer. Thus, the polynucleotide sequences disclosed herein can be used to interpret the detection of variants within the sequences of genes causally associated with cancer. Accordingly, the present disclosure provides the use of a polynucleotide disclosed herein to calibrate a method of detecting cancer. For example, the polynucleotides disclosed herein can be used to calibrate a method of detecting one or more genetic sequences which is indicative of the presence of cancer, or predisposition to developing cancer, or the prognosis of a subject suffering from cancer. Such methods may be performed on an animal subject, such as a mammalian subject, and more particularly, a human subject.

In another example, the polynucleotide sequences disclosed herein may comprise two or more calibration sequences that are selected from different exon sequences from a single gene. Such calibration sequences can be repeated at copy-numbers that replicate the amplification and deletion of gene exons in human disease. Thus, in one example, the polynucleotide sequences disclosed herein may comprise calibration sequences that are selected from different exon sequences from the same gene. In another example, the polynuleootide sequences disclosed herein may comprise calibration sequences that are selected from different exon sequences from the same gene, which calibration sequences are provided at 2, 3, 4, 5, 6, 7, 8, 9, 10, 20, 100, 1,000 and/or 10,000 copies. Thus, the polynucleotide sequences disclosed herein can replicate mutational burden typically present within tumour samples. Accordingly, the calibration sequences may be provided in copy numbers which replicate mutational burden typically present within a tumour sample. Accordingly, the calibration sequences may be provided in copy numbers which replicate mutational burden typically present within a tumour sample.

The polynucleotide sequences disclosed herein may comprise one or more calibration sequences that have been selected from the following gene sequences, in any combination or permutation (which are publicly available on databases such as the NIH's genetic sequence database GenBank^{®}): *ABL1, ABO, ACADM, ACTA2, ACTC1, AFF2, AKT1, AKT2, AKT3, ALK, APC, APOA1, APOB, APOE, AR, ARAF, AREG, ARID1A, ATM, ATN1, ATP7B, ATR, ATXN1, ATXN3, ATXN7, AURKA, AXIN2, BAP1, BCL2, BCR, BCR-ABL1, BCR-JAK2, BLM, BMPR1A, BMPR2, BRAF, BRCA1, BRCA2, BRD4, BTD, BTK, C9orf72, CACNA1A, CACNA1S, CAS9, CASR, CBL, CCND1, CCND2, CCND3, CCNE1, CD19, CD274, CDH1, CDK4, CDK6, CDKN1A, CDKN1B, CDKN2A, CDKN2B, CDKN2C, CFTR, CHEK2, chrM, COL1A1, COL1A1-PDGFRB, COL3A1, CRLF2, CSF1R, CSF3R, CTNNB1, CYP1A2, CYP2C19, CYP2C9, CYP2D6, CYP2D7, CYP3A5, DDR2, DMD, DMPK, DNMT3A, DSC2, DSG2, DSP, EFla, EGFR, EPHA2, EPHA3, ERBB, ERBB2, ERBB3, ERBB4, ERCC1, ERCC2, ERCC4, ERCC6, EREG, ESR1, EZH2, F2, F5, F8, F9, FANCA, FANCC, FBNl, FBXW7, FGA, FGF3, FGF4, FGFR1, FGFR2, FGFR3, FGFR4, FH, FLCN, FLT3, FMR1, FRS2, FXN, G6PD, GALT, GATA3, GBA, GLA, GNA11, GNAQ, GNAS, HBA1, HBA2, HBB, HBD, HBG1, HBG2, HDAC2, HEXA, HFE, HGF, HLA-A, HLA-B, HLA-C, HLA-DM, HLA-DO, HLA-DP, HLA-DQ, HLA-DR, HRAS, HTT, IDH1, IDH2, IGF1R, IGF2, IKBKAP, IL7R, INPP4B, JAK1, JAK2, JAK3, KCNH2, KCNQ1, KDR, KIT, KMT2A, KRAS, LDLR, LMNA, LRP1B, LYZ, MAP2K1, MAP2K2, MAP3K1, MCL1, MCOLNl, MDM2, MDM4, MEK1, MENl, MET, MGMT, MITF, MLH1, MLL, MLL2, MLL3, MPL, MSH2, MSH3, MSH6, MTHFR, MTOR, MUTYH, MYBPC3, MYC, MYCN, MYD88, MYH11, MYH7, MYL2, MYL3, MYLK, NAT2, NF1, NF2, NKX2, NOTCH1, NOTCH2, NPM1, NRAS, NRG1, NTRK1, NTRK3, OTC, PAH, PAK1, PALB2, PAX8, PBRM1, PCSK9, PDGFRA, PDGFRB, PDK1, PDPK1, PIK3CA, PIK3CB, PIK3R1, PIK3R2, PKD1, PKD2, PKHD1, PKP2, PLCG2, PML, PML-RARA, PMS2, PPARG, PPP2R2B, PRKAG2, PRKCH, PRSS1, PTCH1, PTEN, PTPN11, RAC1, RAD50, RAF1, RARA, RB1, RET, RET-PTCH1, RHCE, RHD, RICTOR, RNF43, ROS1, RYR1, RYR2, SCNSA, SDHAF2, SDHB, SDHC, SDHD, SERPINA1, SETD2, SF3B1, SH2B3, SLCO1B1, SMAD3, SMAD4, SMARCA1, SMARCA4, SMARCB1, SMN1, SMN2, SMO, SMPD1, SOCS1, SPINK1, STAG2, STK11, SUZ12, SYK, TBP, TGFBR1, TGFBR2, TMEM43, TMPRSS2, TNNI3, TNNT2, TOP2A, TP53, TPM1, TPMT, TSC1, TSC2, TTR, UGT1A1, VEGFA, VHL, VKORC1, WT1, ZNRF3.* In one example, the polynucleotide sequence comprises calibration sequences that have been selected from a reference, wild-type, mutated or variant sequence of any one or more of the genes described herein.

### ctDNA

Fragments of circulating tumor DNA (ctDNA) may be detected in the bloodstream of cancer patients. These fragments (or ctDNA) may indicate the presence and evolution of a tumor. Detecting ctDNA may be used as a non-invasive method for detecting and diagnosing a tumor.

The polynucleotide sequences disclosed herein may comprise calibration sequences that are selected from ctDNA sequences. For example, the polynucleotide sequences may comprise calibration sequences that are selected from genetic sequences that are associated with cancer, such as somatic, driver, clinically actionable or benign mutations or DNA modifications, which are present within ctDNA.

In one example, the polynucleotide sequences disclosed herein are used to identify and analyze ctDNA fragments. The polynucleotide sequences can be fragmented using enzymatic shearing to replicate the length of ctDNA. In one example, the polynucleotide sequences described herein are fragmented using mechanical or enzymatic shearing to generate fragments that match the length of ctDNA fragments. Such fragments may be, for example, from about 10 to about 400 nucleotides in length. For example, such fragments may be from about 15 to about 350 nucleotides in length, or from about 50 to about 300 nucleotides in length, or from about 100 to about 200 nucleotides in length, or from about 130 to about 170 nucleotides in length, or from about 140 to about 160 nucleotides in length, or about 150 nucleotides in length. Again, the calibration sequences can be repeated at a copy-number in the polynucleotide sequence that reflects the frequency of somatic mutations present within ctDNA.

The copy-number or ratio between calibration sequences in the polynucleotide sequences disclosed herein can emulate tumor mutational burden as reflected in ctDNA.

The calibration sequences can be repeated at a copy-number in the polynucleotide sequence that reflects the frequency of somatic mutations present within ctDNA. The copy-number or ratio between calibration sequences in the adaptor sequences disclosed herein (e.g., within a single adaptor sequence or within a composition of adaptor sequences) can emulate tumour mutational burden as reflected in ctDNA.

The polynucleotide sequences disclosed herein may comprise calibration sequences that contain modified nucleotides. For example, the polynucleotide sequences may comprise calibration sequences that include one or more methylated DNA sequences. In addition, the polynucleotide sequences disclosed herein may comprise calibration sequences that contain modified nucleotides at different and known abundance. In one example, the polynucleotide sequence comprises calibration sequences which are present at different copy-numbers that reflects the frequency of somatic mutations present within ctDNA, such that these polynucleotide sequences can advantageously be used in calibrating methods of detecting somatic mutations in a ctDNA sample.

### Karyotype

Aneuploidy, and large chromosomal deletions and amplifications may cause a range of human diseases. NGS may be used to detect such changes in large chromosomal regions or numbers, however, alignment errors, insufficient sequencing depth and low complexity and repetitive sequences may confound the analysis.

The polynucleotide sequences disclosed herein may comprise a first calibration sequence that represents a first allele sequence, and a second calibration sequence that represents a second allele sequence. The copy-number ratio between two calibration sequences representing different alleles can be determined so as to match the copy number ratio of chromosomal regions in a human karyotype. Thus, in one example, the polynucleotide sequences disclosed herein may comprise one or more calibration sequences that are selected paternal and maternal allele sequences from one or more regions of a human chromosome. The ratio of calibration sequences in the polynucleotide sequence can be predetermined to match the ratio of chromosomes in a normal or abnormal human karyotype. To provide one example of such a scenario, the polynucleotide sequence may comprise calibration sequences that are repeated at 3 copies, relative to at least one other calibration sequence that is repeated at 2 copies. The polynucleotide sequences may comprise calibration sequences that are selected from any one or more of human chromosome 1, 2, 3, 4, 5, 6, 7, 8, 9, 10, 11, 12, 13, 14, 15, 16, 17, 18, 19, 20, 21, 22, X, Y, or mitochondrial chromosome (chrM) sequences. In one example, the polynucleotide sequence comprises calibration sequences repeated at different copy-numbers, as described herein, to establish a quantitative reference ladder to measure and assess the quantity of copy-number variation in a DNA sample. In one example, the polynucleotide sequence comprises calibration sequences that are repeated at the same copy-number as sequences in the human genome.

### NIPT

DNA fragments derived from a fetus genome may be detected circulating within a pregnant mother's blood. The isolation and sequencing of circulating fetal DNA fragments may provide non-invasive methods to detect the presence of chromosomal aneuploidy, copy-number variants, structural variants and small mutations within the fetal genome. However, due to the small fragment size, variable fetal DNA load, sparse genome coverage, and the overlapping presence of the circulating maternal DNA, analysis with NGS methods has limited sensitivity.

The polynucleotide sequences disclosed herein may comprise calibration sequences that are selected from circulating maternal or fetal DNA sequences. Thus, the polynucleotide sequence may comprise one or more calibration sequences that are selected from paternal, maternal or fetal chromosome genetic sequences. In one example, the polynucleotide sequence comprises a first calibration sequence that is selected from maternal chromosome genetic sequences, and a second calibration sequence that is selected from fetal chromosome genetic sequences. The one or calibration sequences may be repeated at copy-numbers that replicate the ratio of maternal and fetal circulating DNA sequences. In one example, the polynucleotide sequences disclosed herein are used to measure fetal circulating DNA in the maternal blood.

The polynucleotide sequences disclosed herein may comprise one or more calibration sequences, wherein the ratio of copy-numbers between two or more calibration sequence matches the ratio of heterozygous, homozygous, fetal and maternal genetic variation, fetal chromosomal aneuploidy, copy number variation, deletions and/or amplifications. In one example, the polynucleotide sequence comprises calibration sequences that are selected from genetic sequences identified with non-invasive prenatal testing (NIPT). Additionally, the polynucleotide sequence may comprise calibration sequences that are selected from genetic sequences with chromosomal aneuploidy, large copy-number variants, micro-amplifications, micro-deletions and/or micro-duplications. In one example, the polynucleotide sequence is fragmented to generate DNA fragments that match the length of circulating fetal DNA and circulating maternal DNA fragments. Such fragments may be, for example, from about 10 to about 400 nucleotides in length. For example, such fragments may be from about 15 to about 350 nucleotides in length, or from about 50 to about 250 nucleotides in length, or from about 100 to about 200 nucleotides in length, or from about 130 to about 170 nucleotides in length, or from about 140 to about 160 nucleotides in length, or may be about 150 nucleotides in length.

### RNA

The genome is transcribed into a range of coding and noncoding RNAs that are collectively termed the transcriptome. RNA sequencing has become a common tool in biological research and is increasingly applied in clinical diagnosis. However, the sheer size and complexity of gene expression, a pervasive expression dependent bias, and additional technical variables during library preparation, sequencing and analysis affects analysis of the transcriptome.

The polynucleotide sequences disclosed herein may comprise or consist of a ribonucleotide (RNA) sequence. In one example, the polynucleotide sequence comprises calibration sequences that are selected from one or more gene exons. In another example, the polynucleotide sequence comprises two or more calibration sequences that are each selected from different exon sequences from a single gene. In a further example, the polynucleotide sequence comprises two or more calibration sequences that are each selected from different exon sequences from different genes. The ratio between the number of copy numbers by which a calibration sequences is repeated can be provided so as to match the gene expression abundance, alternative-splicing frequency and isoform expression of the naturally occurring sequence represented by the calibration sequence.

The polynucleotide sequences disclosed herein may be reverse transcribed into a complementary DNA (cDNA) sequence. Accordingly, the polynucleotide sequences disclosed herein can be used in RNA sequencing methods. Thus, the present disclosure provides the use of a polynucleotide sequence as disclosed herein, in the calibration of a RNA sequencing method.

The polynucleotide sequences disclosed herein can also be used in the targeted-enrichment of RNA sequences using PCR-based amplification methods. For example, the polynucleotide sequences disclosed herein can be used in methods of targeted enrichment of RNA sequences using hybridization to a complementary oligonucleotide probe. Thus, the present disclosure provides the use of a polynucleotide sequence as disclosed herein in the targeted-enrichment of RNA sequences using PCR-based amplification methods and/or using hybridization to a complementary oligonucleotide probe.

The ratio of calibration sequence copy-number present in the polynucleotides disclosed herein can be determined so as to correspond to gene expression abundance. For example, the polynucleotide sequences dislcosed herein can be used with single-cell RNA sequencing methods. In one example, the polynucleotide sequence comprises calibration sequences that are selected from a gene-signature used in the clinical diagnosis of human disease. In another example, the polynucleotide sequence comprises one or more calibration sequences that are selected from fusion gene sequences that occur in cancer. In another example, the polynucleotide sequence comprises one or more calibration sequences that are selected from one or more exons from two distinct genes, as typically occurs in a fusion gene.

The polynucleotide sequences disclosed herein may comprise, for example, one or more calibration sequences that are selected from the following gene list: *ABL1, ABO, ACADM, ACTA2, ACTC1, AFF2, AKT1, AKT2, AKT3, ALK, APC, APOA1, APOB, APOE, AR, ARAF, AREG, ARID1A, ATM, ATN1, ATP7B, ATR, ATXN1, ATXN3, ATXN7, AURKA, AXIN2, BAP1, BCL2, BCR, BCR-ABL1, BCR-JAK2, BLM, BMPR1A, BMPR2, BRAF, BRCA1, BRCA2, BRD4, BTD, BTK, C9orf72, CACNA1A, CACNA1S, CAS9, CASR, CBL, CCND1, CCND2, CCND3, CCNE1, CD19, CD274, CDH1, CDK4, CDK6, CDKN1A, CDKN1B, CDKN2A, CDKN2B, CDKN2C, CFTR, CHEK2, chrM, COL1A1, COL1A1-PDGFRB, COL3A1, CRLF2, CSF1R, CSF3R, CTNNB1, CYP1A2, CYP2C19, CYP2C9, CYP2D6, CYP2D7, CYP3A5, DDR2, DMD, DMPK, DNMT3A, DSC2, DSG2, DSP, EFla, EGFR, EPHA2, EPHA3, ERBB, ERBB2, ERBB3, ERBB4, ERCC1, ERCC2, ERCC4, ERCC6, EREG, ESR1, EZH2, F2, F5, F8, F9, FANCA, FANCC, FBNl, FBXW7, FGA, FGF3, FGF4, FGFR1, FGFR2, FGFR3, FGFR4, FH, FLCN, FLT3, FMR1, FRS2, FXN, G6PD, GALT, GATA3, GBA, GLA, GNA11, GNAQ, GNAS, HBA1, HBA2, HBB, HBD, HBG1, HBG2, HDAC2, HEXA, HFE, HGF, HLA-A, HLA-B, HLA-C, HLA-DM, HLA-DO, HLA-DP, HLA-DQ, HLA-DR, HRAS, HTT, IDH1, IDH2, IGF1R, IGF2, IKBKAP, IL7R, INPP4B, JAK1, JAK2, JAK3, KCNH2, KCNQ1, KDR, KIT, KMT2A, KRAS, LDLR, LMNA, LRP1B, LYZ, MAP2K1, MAP2K2, MAP3K1, MCL1, MCOLN1, MDM2, MDM4, MEK1, MENl, MET, MGMT, MITF, MLH1, MLL, MLL2, MLL3, MPL, MSH2, MSH3, MSH6, MTHFR, MTOR, MUTYH, MYBPC3, MYC, MYCN, MYD88, MYH11, MYH7, MYL2, MYL3, MYLK, NAT2, NF1, NF2, NKX2, NOTCH1, NOTCH2, NPM1, NRAS, NRG1, NTRK1, NTRK3, OTC, PAH, PAK1, PALB2, PAX8, PBRM1, PCSK9, PDGFRA, PDGFRB, PDK1, PDPK1, PIK3CA, PIK3CB, PIK3R1, PIK3R2, PKD1, PKD2, PKHD1, PKP2, PLCG2, PML, PML-RARA, PMS2, PPARG, PPP2R2B, PRKAG2, PRKCH, PRSS1, PTCH1, PTEN, PTPN11, RAC1, RAD50, RAF1, RARA, RB1, RET, RET-PTCH1, RHCE, RHD, RICTOR, RNF43, ROS1, RYR1, RYR2, SCNSA, SDHAF2, SDHB, SDHC, SDHD, SERPINA1, SETD2, SF3B1, SH2B3, SLCO1B1, SMAD3, SMAD4, SMARCA1, SMARCA4, SMARCB1, SMN1, SMN2, SMO, SMPD1, SOCS1, SPINK1, STAG2, STK11, SUZ12, SYK, TBP, TGFBR1, TGFBR2, TMEM43, TMPRSS2, TNNI3, TNNT2, TOP2A, TP53, TPM1, TPMT, TSC1, TSC2, TTR, UGT1A1, VEGFA, VHL, VKORC1, WTl, ZNRF3,* in any combination or permutation.

### Metagenomics

Metagenomics provides a global profile of the microbe genomes present within an environmental sample. However, a lack of microbe reference genome sequences, the presence of complex, variable and repetitive genetic sequences and technical variation due to library preparation and sequencing can confound analysis.

The polynucleotide sequences disclosed herein may contain one or more calibration sequences that are selected microbial genome sequences. For example, the polynucleotide sequences may comprise calibration sequences that are selected from bacterial, archaeal, viral, plasmid and/or baceriophage RNA or DNA sequences. In one example, the polynucleotide sequence comprises two or more calibration sequences that are repeated at copy numbers that match the abundance of two or more microbes. Alternatively or in addition, the polynucleotide sequences may comprise one or more calibration sequences that are selected from horizontally-acquired genes, plasmids, transposons, or integrons, viral or bacteriophage sequences.

For example, the polynucleotide sequences disclosed herein may comprise one or more calibration sequences that are selected from viral genome sequences, including RNA or DNA viral genome sequences, retro-transcribing viruses, double-stranded and single-stranded RNA and/or DNA viruses. In one example, the polynucleotide sequence comprises one or more calibration sequences that are selected from sequences of viral integration into the human genome. In another example, the polynucleotide sequence may comprise calibration sequences that are selected from variant sequences to a viral genome sequence. In another example, the polynucleotide sequence may comprise one or more calibration sequences that are selected from genetic variants sequences that confer drug resistance (such as antibiotic resistance). In one example, the polynucleotide sequence comprises one or more calibration sequences that are selected HIV genome sequences that confer resistance to non-nucleoside reverse-transcriptase inhibitors, nucleoside reverse-transcriptase inhibitors and protease inhibitors. In another example, the polynucleotide sequence comprises calibration sequences that are selected from a viral vector sequence used in gene therapy applications.

The polynucleotide sequences disclosed herein may comprise one or more calibration sequences that are selected, for example, from the following list of genome sequences: *Astrovirus, Candida Albicans, Candida glabrata, Cryptococcus neoformans, Cryptococcus neoformans (SOD1), Dengue virus, Ebola virus, Epstei Barr Virus, Helicobacter pylori, Hepatitis A virus, Hepatitis B virus, Hepatitis C virus, Hepatitis D virus, Hepatitis E virus, Hepatitis E virus, Hepatitus C, Human alphaherpesvirus, Human coronavirus, Human enterovirus A, Human enterovirus B, Human enterovirus D, Human herpesvirus, Human immunodeficiency virus, Human metapneumovirus, Human papillomavirus, Human parainfluenza virus 1, Human parainfluenza virus 3, Human parvovirus, Human rhinovirus, Infleunza, Influenza A, Influenza B., Norovirus GI, Parechovirus, Poliovirus, Rabies virus, Cryptosporidium parvum (Hsp70), Respiratory syncytial virus, Rotavirus, Sapovirus, Staphylococcus aureus, West Nile virus, Yellow fever virus, Zika virus, Escherichia coli.*

### Modified nucleotides

The polynucleotide sequences disclosed herein may include one or more modified nucleotides. In one embodiment, the polynucleotide sequence comprises one or more calibration sequences that include modified nucleotides, and one or more calibration sequences that do not include modified nucleotides. In one embodiment, the polynucleotide sequence includes modified nucleotides that are present at known sites in the polynucleotide sequence. In one embodiment, the polynucleotide sequence can be used to calibrate the detection of modified nucleotides using sequencing, hybridization, antibody or fluorescence technology.

The polynucleotide sequences disclosed herein may include one or more modified nucleotides that include one or more of 5-mehtly-uridine, 2'O-merthly-RNA, 2'-flurouracil, 2'-fluorocytosine, 5-propynyl-2'deoxycytidine, 5-propynyl-2'deoxyuridine, 5-methylcytosine, 5-hydroxymethylcytosine ionisin, 7-deazaguanine, 2,6-diaminopurine, 5'nitroindole, 3-nitropyrrol and 2'-O -methylated ribose. In addition, the polynucleotide sequences disclosed herein may include modified nucleotides including any one or more of 2-Amino-ATP, 2-Amino-6-Cl-RTP, 8-Aza-ATP, 6-Cl-RTP, 2'-Fluoro-dATP, 2'-Fluoro-dCTP, 2'-Fluoro-dGTP, 2'-Fluoro-dUTP, 5-Iodo-CTP, 5-Iodo-UTP, N6-Methyl-ATP, 5-Methyl-CTP, 2'-O-Methyl-ATP, 2'-O-Methyl-CTP, 2'-O-Methyl-GTP, 2'-O-Methyl-UTP, Pseudo-UTP, ITP, 2'-O-Methyl-ITP, Puromycin-TP, Xanthosine-TP, 5-Methyl-UTP, 4-Thio-UTP, 2'-Amino-dCTP, 2'-Amino-dUTP, 2'-Azido-dCTP, 2'-Azido-dUTP, O6-Methyl-GTP, 2-Thio-UTP, Ara-CTP, Ara-UTP, 5,6-Dihydro-UTP, 2-Thio-CTP, 6-Aza-CTP, 6-Aza-UTP, N1-Methyl-GTP, 2'-O-Methyl-2-Amino-ATP, 2'-O-Methylpseudo-UTP, N1-Methyl-ATP, 2'-O-Methyl-5-methyl-UTP, 7-Deaza-GTP, 2'-Azido-dATP, 2'-Amino-dATP, Ara-ATP, 8-Azido-ATP, 5-Bromo-CTP, 5-Bromo-UTP, 2'-Fluoro-dTTP, 3'-O-Methyl-ATP, 3'-O-Methyl-CTP, 3'-O-Methyl-GTP, 3'-O-Methyl-UTP, 7-Deaza-ATP, 5-AA-UTP, 2'-Azido-dGTP, 2'-Amino-dGTP, 5-AA-CTP, 8-Oxo-GTP, 2-Amino-RTP, Pseudoiso-CTP, N4-Methyl-CTP, N1-Methylpseudo-UTP, 5,6-Dihydro-5-Methyl-UTP, N6-Methyl-Amino-ATP, 5-Carboxy-CTP, 5-Formyl-CTP, 5-Hydroxymethyl-UTP, 5-Hydroxymethyl-CTP, Thieno-GTP, 5-Hydroxy-CTP, 5-Formyl-UTP, Thieno-UTP, 2-Amino-6-Cl-dRTP, 2-Amino-dATP, 2-Amino-dRTP, 5-Bromo-dCTP, 5-Bromo-dUTP, 6-Chloro-dRTP, 7-Deaza-dATP, 7-Deaza-dGTP, dITP, 5-Propynyl-dCTP, 5-Propynyl-dUTP, 2'-dUTP, 5-Fluoro-dUTP, 5-Iodo-dCTP, 5-Iodo-dUTP, N6-Methyl-dATP, 5-Methyl-dCTP, O6-Methyl-dGTP, N2-Methyl-dGTP, 5-Nitro-1-Indolyl-dRTP, 8-Oxo-dATP, 8-Oxo-dGTP, 2-Thio-dTTP, 2'-dPTP, 5-Hydroxy-dCTP, 4-Thio-dTTP, 2-Thio-dCTP, 6-Aza-dUTP, 6-Thio-dGTP, 8-Chloro-dATP, 5-AA-dCTP, 5-AA-dUTP, N4-Methyl-dCTP, 2'-deoxyzebularine-TP, 5-Hydroxymethyl-dUTP, 5-Hydroxymethyl-dCTP, 5-Propargylamino-dCTP, 5-Propargylamino-dUTP, 5-Carboxy-dCTP, 5-Formyl-dCTP, 5-Indolyl-AA-dUTP, 5-Carboxy-dUTP, 5-Formyl-dUTP, 3'-dATP, 3'-dGTP, 3'-dCTP, 5-Methyl-3'-dUTP, 3'-dUTP, ddATP, ddGTP, ddUTP, ddTTP, ddCTP, 3'-Azido-ddATP, 3'-Azido-ddGTP, 3'-Azido-ddTTP, 3'-Amino-ddATP, 3'-Amino-ddCTP, 3'-Amino-ddGTP, 3'-Amino-ddTTP, 3'-Azido-ddCTP, 3'-Azido-ddUTP, 5-Bromo-ddUTP, ddITP, Biotin-16-dUTP, Biotin-16-dCTP, Biotin-16-CTP, N4-Biotin-dCTP, Biotin-16-UTP, Dabcyl-dUTPN/AN/AN/A, Desthiobiotin-6-dCTP, Desthiobiotin-16-UTP, ppGpp, pGp, 5'-Biotin-GMP, 5'-Biotin-AMP, 5'-Biotin-dGMP, 5'-Biotin-dAMP, 5'-Amino-GMP, mCAP, CAP, ARCA, (1-Thio)-dATP, (1-Thio)-dCTP, (1-Thio)-dGTP, (1-Thio)-dTTP, (1-Thio)-ATP, (1-Thio)-CTP, (1-Thio)-GTP, (1-Thio)-UTP, (1-Thio)-ddATP, (1-Thio)-ddCTP, (1-Thio)-ddGTP, (1-Thio)-ddTTP, (1-Thio)-3'-Azido-ddTTP, (1-Thio)-ddUTP, (1-Borano)-dATP, (1-Borano)-dCTP, (1-Borano)-dGTP, (1-Borano)-dTTP, Ganciclovir-TP, Cidofovir-DP.

### Methods of producing reference standards and adaptors

In one embodiment, the present disclosure provides a method of generating a polynucleotide sequence that comprises calibration sequences. The polynucleotide sequence may be a ladder or adaptor as described herein. The method may comprise first selecting the calibration sequences, as described herein. In some embodiments, each calibration sequence is repeated at one or more copy-numbers. In some embodiments each calibration copy sequence is then arranged into a linear consecutive order to form a single continuous polynucleotide sequence. Due to this arrangement, the calibration sequences are arranged in an artificial linear order that is not otherwise observed in nature. In some embodiments, each calibration sequence is interspersed with 'spacer' sequences and the termini of the polynucleotide sequence comprise flanking terminal sequences. In some embodiments, the spacer and termini sequence described herein, may include primer-binding sites, restriction digestion sites, promoter and transcription initiation sites, and other sequence elements.

In some embodiments, the polynucleotide sequence is physically synthesized as a DNA or RNA molecule. In some embodiments, the polynucleotide sequence is flanked by two restriction digestion sites that allow excision and purification of the polynucleotide sequence from a host vector sequence. In some embodiments, the polynucleotide sequence comprises calibration sequences that are flanked by sequences that enable excision and purification of the calibration sequence by restriction endonuclease.

In some embodiments, the polynucleotide sequence, as described herein, is proceeded in the 5' direction by a promoter sequence and/or followed by a poly-A tail and/or a restriction site to enable in vitro transcription of the polynucleotide sequence or calibration sequences. In some embodiments, the calibration sequence, as described herein, is proceeded in the 5' direction by a promoter sequence and/or followed by a poly-A tail and/or a restriction site to enable in vitro transcription of the polynucleotide sequence or calibration sequences. In some embodiments, both forward and reverse strands of the polynucleotide sequence are in vitro transcribed to form two RNA fragments.

In one embodiment, the polynucleotide sequence is synthesized into a RNA or DNA molecule. In one embodiment, the polynucleotide sequence is hosted within a vector. In one aspect, the polynucleotide or calibration sequence, described herein, is excised from a vector. In one embodiment, the polynucleotide or calibration sequence, described herein, is excised from the vector by restriction digestion. In one embodiment, the polynucleotide or calibration sequence, described herein, is amplified from the vector by polymerase chain reaction. In one embodiment, the polynucleotide or calibration sequence, described herein, is amplified from the vector by polymerase chain reaction. In one embodiment, the polynucleotide sequence is transcribed from the vector by undergoing in vitro transcription. In one embodiment, two or more polynucleotide sequence are combined together to formulate a mixture.

In one embodiment, the polynucleotide sequence, described herein, is fragmented to generate derivative pools of fragmented DNA molecules. In one embodiment, the polynucleotide sequence, described herein, is mechanically or enzymatically sheared to generate derivative pools of fragmented DNA molecules. In one embodiment, the polynucleotide sequence, described herein, is digested to generate derivative pools of smaller DNA fragments. In one embodiment, the polynucleotide sequence, described herein, is digested to generate DNA fragments of same length as normal, tumor, fetal, and maternal circulating DNA molecules.

### Ligating adaptor sequences

In accordance with examples in which the polynucleotide sequence of the disclosure is an adaptor sequence, the adaptor sequence may be ligated to other polynucleotides. The adaptor sequences disclosed herein can be ligated to polynucleotides in a test sample by any means known in the art. For example and without limitation, the ligation may be effected using a ligase or transposase enzyme. One example of a suitable ligase enzyme is T4 DNA ligase.

The polynucleotide adaptor sequence can be ligated to the 3' and/or 5' termini of a target polynucleotide sequence to form a single continuous polynucleotide sequence. Thus, the methods disclosed herein may comprise ligating the adaptor sequence to a target polynucleotide. In one example, one or more polynucleotide adaptor sequence can be ligated to a naturally occurring RNA or DNA fragment.

In one example, the polynucleotide adaptor sequence can be ligated to DNA fragments, wherein the DNA fragments have been end-repaired and dA-tailed.

In another example, one or more polynucleotide adaptor sequences can be attached to a target polynucleotide during DNA synthesis, polymerase-chain reaction amplification or reverse transcription to generate a single continuous polynucleotide sequence. For example, the initiation of DNA synthesis from the 3' end of the polynucleotide adaptor sequence can generate a single continuous polynucleotide sequence that comprises the polynucleotide adaptor sequence followed by the target DNA sequence.

Preferably, the polynucleotide adaptor sequences are ligated to natural DNA fragments during the preparation of a library for the sequencing process.

In one example, the 3' end of the polynucleotide adaptor sequence is ligated to the 5' start of a target DNA fragment sequence. In another example, the 3' end of the target DNA fragment is ligated to the 5' start of the polynucleotide adaptor sequence.

### Methods of using polynucleotide sequences

The present disclosure provides polynucleotide sequences comprising two or more calibration sequences, which allows for comparison of their detection and/or quantitation in a variety of sequencing methods. The inclusion of the two calibration sequences within a single polynucleotide sequence allows the omission of technical differences that may otherwise result during the preparation of separate polynucleotide sequences. By combining two calibration sequences into a single genetic sequence, we can ensure that both calibration sequences are subject to the same manufacturing conditions and the same process parameters when used in calibration methods, thereby allowing a more accurate comparison of the two calibration sequences within the polynucleotide sequence.

The polynucleotide sequences disclosed herein can be added directly to a RNA or DNA sample of interest at a predetermined fractional concentration. Thus, the polynucleotide sequence and a RNA or DNA sample of interest can undergo library preparation and sequencing together. In one example, the polynucleotides disclosed herein are added to a human genome DNA sample and then undergo library preparation and sequencing together. In another example, the polynucleotides disclosed herein are added to a DNA extracted from a cell-line, and then undergo library preparation and sequencing together. For example, the polynucleotide sequence can be added to the RNA sample and they together undergo reverse transcription, library preparation and sequencing.

Advantageously, the polynucleotide sequences disclosed herein can be subjected to any sequencing methods separately to methods applied to a sample containing a polynucleotide sequence of interest. Performance of the methods under identical conditions to those applied to the sample containing a polynucleotide sequence of interest, but in the absence of the sample of interest, reduces the likelihood of the calibration sequences interfering with the results of the sequencing process performed on the sample.

The present disclosure also provides methods of using polynucleotide adaptor sequences disclosed herein to calibrate a polynucleotide sequencing and/or quantitation method. The calibration sequences can be used to identify technical errors, including any one or more of: quantitative accuracy, quantitative sensitivity, sequencing error, PCR amplification bias, the use of targeted-enrichment steps, ligation bias during library preparation, library complexity and sample input, bioinformatics artefacts, and sequencing errors.

The present disclosure also provides methods wherein calibration sequences in one library are compared to calibration sequences in a second library, and wherein the comparison identifies differences in library depth, library preparation methods, sequencing technologies, and/or other batch-specific effects that result from sample preparation by different laboratory sites or personnel.

The present disclosure also provides methods wherein the quantity of calibration sequences within the normalization of sequence quantity between two or more libraries is determined from the quantity of calibration sequences within the two or more libraries.

Further, the present disclosure provides methods wherein scaling factors that are determined from the calibration sequences can be used to normalize the determination of target RNA or DNA sequence quantity.

The present disclosure provides a method wherein the polynucleotide adaptor sequences are sequenced using next-generation sequencing methods.

The present disclosure provides methods wherein the quantity of calibration sequences within the sequenced reads within the library data file can be determined by the quantity of the calibration sequence within the composition of polynucleotide adaptor sequences. In one example, the quantity of calibration sequences within the output data file from a high-throughput next-generation sequencing process is determined by the quantity of the calibration sequence within the composition of polynucleotide adaptor sequences. The present disclosure also provides a method wherein the quantity of the calibration sequence in the composition of polynucleotide sequences determines the quantity of sequenced reads in the output library data file that include the calibration sequence.

The present disclosure provides a method wherein the determined quantity of one calibration sequence within the sequenced reads within the library data file is compared to the reference quantity of the calibration sequence in the composition, and wherein differences between the determined and refence quantity indicate the error in sequence determination and quantification. The present disclosure provides a method wherein the comparison of the calibration sequence counts between two or more different libraries enables normalization between the libraries. The present disclosure provides a method wherein the polynucleotide adaptor sequence is used as a reference sequence of known quantity to calibrate the measurement of target RNA and DNA sequence quantity.

The present disclosure provides a method wherein a composition of polynucleotide adaptor sequences is used as primer oligonucleotides to initiate DNA synthesis. The present disclosure provides a method wherein a composition of polynucleotide adaptor sequences is used as primer oligonucleotides during a polymerase chain reaction. The present disclosure provides a method wherein a mixture is provided comprising one polynucleotide adaptor sequence that is present at a different quantity to a second polynucleotide adaptor sequence within the mixture, and wherein the mixture of polynucleotide adaptor sequences is used during the synthesis of two or more target DNA sequences, and wherein the synthesis of the two or more DNA targets is calibrated by comparisons to the reference quantity of the respective calibration sequences.

In one embodiment, the quantity of polynucleotide adaptor sequence, comprising calibration sequences, that is ligated to a natural DNA target fragment is determined by the known quantity of the polynucleotide adaptor in the mixture. In one embodiment, the quantity of amplified DNA fragments that contain the polynucleotide sequence will determined by the known quantity of the polynucleotide adaptor in the mixture. In one embodiment, the quantity of sequence matching the polynucleotide sequence in the output library data file is determined by the quantity of polynucleotides in the mixture.

The present disclosure provides a method wherein the polynucleotide adaptor sequences, as described herein, are used with methods of targeted enrichment using hybridization to complementary oligonucleotide probes. The present disclosure provides a method of using polynucleotide adaptor sequences that contain calibration sequences to calibrate polynucleotide sequencing processes, wherein sequencing processes include are single-end sequencing, paired-end sequencing, nanopore sequencing, sequencing-by-synthesis, short-read, long-read sequencing technologies. The present disclosure provides a method wherein the polynucleotide adaptor sequences described herein are used with sequencing methods, where such methods include whole genome sequencing, shotgun sequencing methods, metagenomic sequencing methods, single-cell sequencing methods, nanopore sequencing methods, and long-read sequencing methods.

The present disclosure provides a method wherein the polynucleotide adaptor sequences, described herein, are used for the detection of genetic sequences that indicate disease, including inherited human disease, cancer diagnosis, pathogen detection, and drug resistance. The present disclosure provides a method wherein, the polynucleotide adaptor sequences are used in the clinical diagnosis of cancer. In one embodiment, the polynucleotide adaptor sequences are used in the analysis of circulating tumour DNA and non-invasive prenatal testing.

The present disclosure provides a method wherein the polynucleotide adaptor sequences are with sequencing applications, wherein sequencing applications include as whole genome sequencing, targeted sequencing applications, RNA sequencing, or metagenomic sequencing. The present disclosure provides a method wherein the polynucleotide adaptor sequences are with polymerase chain reaction amplification, DNA synthesis, DNA sequencing, RNA sequencing, reverse transcription and cDNA sequencing. The present disclosure provides a method wherein polynucleotide sequences used in sequencing methods based on the polymerase chain reaction, wherein sequencing processes include multi-plex PCR amplification, real-time PCR amplification, reverse transcription, amplicon gene panels, immune-repertoire profiling.

The present disclosure provides a method wherein the polynucleotide adaptor sequences are used during the preparation of libraries that are then subject to next-generation sequencing process and then generate an output library of sequenced reads (e.g. a .FASTQ file). The present disclosure provides a method wherein the calibration sequences can be identified in the read sequences. The present disclosure provides a method wherein the calibration sequences can be identified and analysed during the removal of adaptor sequences in a library of sequenced reads. The present disclosure provides a method wherein the quantity and sequence of calibration sequences in the output library file are compared to the reference quantity and reference sequence of the calibration sequences, wherein differences between the determined and reference sequence or quantity of calibration sequence indicates errors in the determination of the sequence and quantity in the sequencing process. The present disclosure provides a method wherein calibration sequences described are used with methods of library preparation that include PCR-free, transposase-based, amplicon based and target enrichment methods. The present disclosure provides a method wherein calibration sequences described are used with methods of sequencing that include nanopore sequencing, sequencing-by-synthesis, or short-read or long-read sequencing technologies.

The present disclosure provides a method wherein the determined quantity of calibration sequences can be compared to the reference quantity of calibration sequences to indicate the presence of technical errors and variation. The present disclosure provides a method wherein the determined quantity of calibration sequences can be compared to the reference quantity of calibration sequences to indicate the presence of technical errors and variation, wherein those errors include sequencing errors, PCR amplification bias, ligation bias and bioinformatics artefacts. The present disclosure provides a method wherein the calibration sequences present in one library are compared to the calibration sequences present in a second library, wherein differences between the libraries indicate errors.

When two or more calibration sequences are included in an adaptor sequence, the inclusion of the two calibration sequences allows the omission of technical differences that may otherwise result during the preparation of separate polynucleotide sequences. By combining two calibration sequences into a single genetic sequence, we can ensure that both calibration sequences are subject to the same manufacturing conditions and the same process parameters when used in calibration methods, thereby allowing a more accurate comparison of the two calibration sequences within the polynucleotide sequence.

The adaptor sequences disclosed herein can be added directly to a polynucleotide sample of interest at a predetermined fractional concentration. Thus, the adaptor sequence and an RNA or DNA sample of interest can undergo library preparation and sequencing together. For example, the adaptor sequence can be added to the RNA sample and they together can undergo reverse transcription, library preparation and sequencing. The polynucleotide sequences disclosed herein can be used to calibrate a sequencing process. For example, the polynucleotide sequences disclosed herein can be used to calibrate a next-generation sequencing process. The polynucleotide sequences can be used to measure any one or more of true-positive, true-negative, false-positive, and false-negative rate in any method of detecting genetic variants. Thus, the polynucleotide sequences disclosed herein can be used as a quantitative and qualitative control in sequencing methods, such as NGS methods. It will be appreciated that differences between the sequence and/or copy number of the calibration sequences measured during a given sequencing method, compared to the known sequence and/or copy number of those calibration sequences, indicates potential error and/or bias in the sequencing methodology. As a result, such differences can be used to calibrate those sequencing methodologies.

The polynucleotide sequences disclosed herein can be used with library preparation and sequencing methods. For example, the polynucleotide sequences can be used with PCR-based, transposon-based, PCR-free, PCR amplification, and target enriched library preparation methods. The polynucleotide sequences disclosed herein can also be used with nanopore sequencing, sequencing-by-synthesis, or short-read or long-read sequencing technologies.

In one example, the polynucleotide sequences disclosed herein are used with methods of targeted enrichment of DNA sequences. Thus, the polynucleotide sequences disclosed herein can be amplified with polymerase chain reaction.

The polynucleotide sequences disclosed herein can also be used with methods of targeted enrichment using hybridization to complementary oligonucleotide probes. The polynucleotide sequences disclosed herein can also be used with any one or more of whole genome sequencing, shotgun sequencing, metagenomic sequencing, nanopore sequencing, and/or long-read sequencing methods.

The polynucleotide sequences described herein can be used with fluorescent oligonucleotide probes. Thus, the methods disclosed herein can comprise the addition of one or more fluorescent probes. In one example, the polynucleotide sequences disclosed herein are used with fluorescent in situ hybridization. In another example, the polynucleotide sequences disclosed herein are used with fluorescence microscopy. In another example, the polynucleotide sequences disclosed herein may be used with fluorescence detection of target DNAs using oligonucleotide probes.

The polynucleotide sequences disclosed herein can be used with targeted sequencing applications. For example, the polynucleotide sequences disclosed herein can be used with PCR amplification. The polynucleotide sequences disclosed herein can also be used with targeted gene panels. In one example, oligonucleotide probes hybridize, capture and enrich the polynucleotide sequences disclosed herein, during library preparation for next-generation sequencing. Thus, the extent of enrichment of the calibration sequences can be used to calibrate a method of enriching a target sequence of interest in a sample. Primers may be designed to target the polynucleotide or calibration sequences disclosed herein, during DNA synthesis and amplification. Thus, primers and oligonucleotide probes which are complementary to the polynucleotide sequence, or one or more calibration sequences disclosed herein, can be used. Thus, the polynucleotide sequence can comprises calibration sequences that are targeted for enrichment. In one example, the polynucleotide sequence comprises one or more calibration sequences that are targeted for enrichment, and one or more calibration sequences that are not targeted for enrichment. Thus, the polynucleotide sequence can be used to calibrate the enrichment of the first calibration sequence by comparison to the enrichment of the second calibration sequence. Alternatively or in addition, the polynucleotide sequence can be used to calibrate the sequencing of the first calibration sequences by comparison to the sequencing of the second calibration sequence. In one example, the enrichment of one or more calibration sequences can be measured and calibrated by comparing abundance relative to the expected copy number of the calibration sequence within the polynucleotide sequence.

In one example of the methods disclosed herein, the polynucleotide sequences can be added to the circulating DNA extracted from a blood sample prior to library preparation and sequencing.

In another example, the polynucleotide sequences disclosed herein can be added to DNA extracted from a tumor biopsy prior to library preparation and sequencing.

The polynucleotide sequences disclosed herein can act as internal quantitative and qualitative controls throughout the sequencing workflow. Thus the methods disclosed herein enable the assessment, optimization and calibration of sequencing methods. The polynucleotide sequences disclosed herein can also provide an empirical measurement of the diagnostic power in a next-generation sequencing test. For example, the polynucleotide sequences described herein can be used to measure true-positive, true-negative, false-positive, and/or false-negative rates for identifying mutations in genomic DNA. The polynucleotide sequences disclosed herein can also allow the normalization of multiple DNA or RNA samples. In one ladder, the polynucleotide sequences disclosed herein can allow the normalization of multiple DNA or RNA samples from different sites or times.

### Analysis of polynucleotide sequences

The abundance of calibration sequences within the polynucleotide sequence of the disclosure can be determined by counting the number of derivative sequenced reads within the output library. In one example, the abundance of calibration sequences can be measured by counting the alignments of sequence reads to a reference genome or chromosome index. The abundance of calibration sequences can also be determined in other suitable ways known in the art, for example, by measuring fluorescence using a microarray or in situ hybridization. In one example, the abundance of calibration sequences is measured using hybridization to oligonucleotide probes.

The present disclosure provides a method of calibrating a sequencing process using the polynucleotide sequences disclosed herein. The polynucleotide sequence can undergo concurrent library preparation and sequencing along with the sample. In such methods, the polynucleotide sequence can accrue technical variation along with the accompanying sample downstream from the point of addition. Thus, the polynucleotide sequences disclosed herein can be used to measure the generation of technical errors during library preparation, sequencing and bioinformatic analysis. Accordingly, the polynucleotide sequences disclosed herein can be used to measure technical variation resulting from PCR amplification bias, targeted-enrichment steps, ligation bias during library preparation, library complexity, sample input, bioinformatics errors, and/or sequencing errors. The magnitude of technical variation that may accumulate throughout the NGS workflow can be proportional to the deviation between the expected copy number and observed abundance for calibration sequences within the polynucleotide sequence.

The present disclosure also provides methods of using the polynucleotide sequences disclosed herein to calibrate the sequencing of RNA or DNA sequences in another, separate, sample. The polynucleotide sequences disclosed herein can be used to determine or estimate the uncertainty associated with the measurement of polynucleotide sequences. For example, the polynucleotide sequence can be used to calculate a scaling factor from the technical variation measured during the sequencing process. This scaling factor can then be used during the analysis of polynucleotide sequences derived from the accompanying RNA or DNA sample.

The polynucleotide sequences can be used to measure the accuracy of measurements using PCR amplification, oligonucleotide probe hybridization, fluorescence or other quantitative measures of abundance. In one example, sequenced reads derived from calibration sequences within the polynucleotide sequence are counted. The measured read count of calibration sequences is then compared to the expected copy-number by which the calibration sequence is repeated within the polynucleotide sequence, wherein differences between the measured read count and the expected copy-number indicate errors in the library preparation and/or sequencing process, and wherein this error can be measured using statistics including, for example, the sum of residuals, correlation and slope, so that the magnitude of technical variation that accrues across the NGS workflow may be measured. The polynucleotide sequences can therefore be used to normalize technical differences between two or more next-generation sequencing libraries.

### Kits

The present disclosure also provides kits comprising one or more polynucleotide sequences disclosed herein. In some examples, the kit comprises one or more polynucleotides of the disclosure which are ladders. In other example, the kit comprises one or more polynucleotides of the disclosure which are adaptor sequences. In other examples, the kit comprises both ladder sequences and adaptor sequences. The kits may also provide information describing the particular polynucleotide sequences contained therein, such as (but not limited to) its sequence, concentration, structural genomic features of interest, etc.

The kits may comprise a mixture of any one or more of the polynucleotide sequences described herein, in any combination. The mixture of polynucleotide sequences may be provided together, in a single buffer, which can be provided in one or more containers. Alternatively, the mixture of polynucleotide sequences can be provided in the form of multiple, separate containers, each comprising a single polynucleotide sequence, or a single concentration of a polynucleotide sequence. In one example, the separate containers are provided in association with each other as a kit.

The kits may further comprise a computer apparatus, computer programmable media, and/or the computer software disclosed herein. In one example, the kits are provided as a package allowing the physical reference standards to be used experimentally and allowing the computer apparatus and software to be used to relate the experimentally derived sequencing information to the reference standards described herein.

A reference standard as described herein can be designed based using naturally occuring genetic sequences. For example, kmers can be selected from naturally occuring genetic sequences. The kmers ca then be conjoined at one or-more known copy-number units to form a single contiguous sequence. The contiguous sequence can then be synthesized into vector. The vector can then be used in a manufacturing process comprising: bacterial transformation, amplification, extraction, restriction digestion, purification and quantification. The vector can also be used in a manufacturing process comprising: PCR amplification in which primers can be designed to amplify the sequence, and the products of the amplification can then be purified and quantified. The resulting reference standards can be used as a stock inventory for that reference standard.

These manufactured reference standards can be combined with other different manufactured reference standards to form a mixture. These reference standards can be combined at different concentration to emulate homozygous, heterozygous and other allele frequencies, copy-numbers, chromosomal ploidy, and microbial and pathogen abundance.

The reference standards or mixture of reference standards can be added at a fractional concentration to RNA/DNA extracted from a human sample or other sample of interest, and then together undergo library preparation and sequencing. The reference standards alone can be subject to library preparation and sequencing. NGS sequencing can be used to produce a single (.FASTQ) library of reads of the reference standards or mixture of reference standards and sample. The reads derived from the ladder reference standards can be identified by the presence of known kmers within the read sequence. Thus, reads can be classified as being from standards if they comprise the known kmers, or classified as from the sample if they do not comprise the known kmers. This output (.FASTQ) library can be partitioned into two smaller libraries from either reference standards or sample. Data relating to the ladder reference standards can be analyzed in the output library.

### Computer system and computer implemented method:

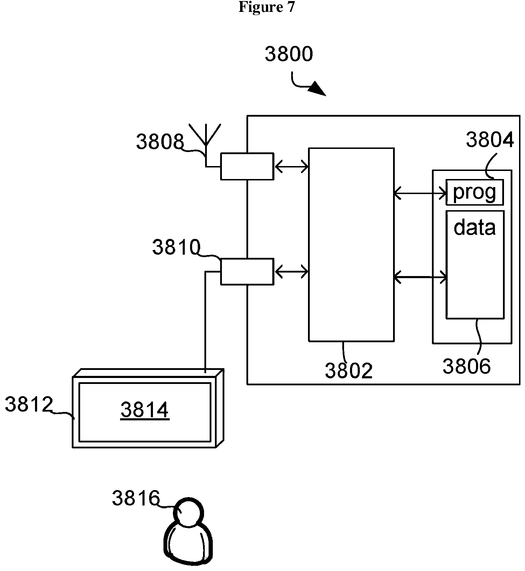

The present disclosure also provides a computer system and a computer implemented method. Figure 7 illustrates a suitable computer system 3800 for calibrating a polynucleotide sequencing or quantitation method. The computer system 3800 comprises a processor 3802 connected to a program memory 3804, a data memory 3806, a communication port 3808 and a user port 3810. The program memory 3804 is a non-transitory computer readable medium, such as a hard drive, a solid state disk or CD-ROM. Software, that is, an executable program stored on program memory 3804 causes the processor 3802 to perform the method disclosed herein.

The processor 3802 may then store the initial and/or calibrated results on data store 3806, such as on RAM or a processor register. Processor 3802 may also send the initial and/or calibrated results via communication port 3808 to a server, such as sample sequence database or computer system that manages a polynucleotide sequencing experiment.

The processor 3802 may receive data, such as data indicative of a polynucleotide sequence or quantity of a polynucleotide sequence, or sequences of the sample, from data memory 3806 as well as from the communications port 3808 and/or the user port 3810, which is connected to a display 3812 that shows a visual representation 3814 of the sequencing and/or quantitation result to a user 3816. In one example, the processor 3802 receives sequence data from a sequencing device via communications port 3808, such as by using a Wi-Fi network according to IEEE 802.11. The Wi-Fi network may be a decentralised ad-hoc network, such that no dedicated management infrastructure, such as a router, is required or a centralised network with a router or access point managing the network.

Although communications port 3808 and user port 3810 are shown as distinct entities, it is to be understood that any kind of data port may be used to receive data, such as a network connection, a memory interface, a pin of the chip package of processor 3802, or logical ports, such as IP sockets or parameters of functions stored on program memory 3804 and executed by processor 3802. These parameters may be stored on data memory 3806 and may be handled by-value or by-reference, that is, as a pointer, in the source code.

The processor 3802 may receive data through all these interfaces, which includes memory access of volatile memory, such as cache or RAM, or non-volatile memory, such as an optical disk drive, hard disk drive, storage server or cloud storage. The computer system 3800 may further be implemented within a cloud computing environment, such as a managed group of interconnected servers hosting a dynamic number of virtual machines.

It is to be understood that any receiving step may be preceded by the processor 3802 determining or computing the data that is later received. For example, the processor 3802 may determine the sequence data of the polynucleotide sequence and may store the sequence data in data memory 3806, such as RAM or a processor register. The processor 3802 may then request the data from the data memory 3806, such as by providing a read signal together with a memory address. The data memory 3806 may provide the data as a voltage signal on a physical bit line and the processor 3802 may receive the sequence data via a memory interface.

It is to be understood that throughout this disclosure unless stated otherwise, data may be represented by data structures, such as ["G","A","T","C"] strings or list of binary tuples encoding the nucleotides. The data structures can be physically stored on data memory 3806 or processed by processor 3802.

It should be understood that the techniques of the present disclosure might be implemented using a variety of technologies. For example, the methods described herein may be implemented by a series of computer executable instructions residing on a suitable computer readable medium. Suitable computer readable media may include volatile (e.g. RAM) and/or non-volatile (e.g. ROM, disk) memory, carrier waves and transmission media. Exemplary carrier waves may take the form of electrical, electromagnetic or optical signals conveying digital data steams along a local network or a publically accessible network such as the internet.

It should also be understood that, unless specifically stated otherwise as apparent from the following discussion, it is appreciated that throughout the description, discussions utilizing terms such as "processing" or "computing" or "calculating", or "determining" or "displaying" or "calibrating" or "normalizing" or the like, can refer to the action and processes of a computer system, or similar electronic computing device, that processes and transforms data represented as physical (electronic) quantities within the computer system's registers and memories into other data similarly represented as physical quantities within the computer system memories or registers or other such information storage, transmission or display devices.

The present disclosure is now described further in the following non-limiting examples.

### Example 1: Use of polynucleotide sequence to calibrate measurement of a cancer gene.

This example shows the design and synthesis of a polynucleotide sequence that comprise two or more calibration sequences from the human genome. Three ~750nt calibration sequences were selected from different exon sequences from the *ERBB2* (*HER2*) gene (Figure 1; see SEQ ID NOs:11-13). The calibration sequences were then repeated; with sequence A present at one copy, sequence B present at two copies, sequence C present at 3 copies, thereby emulating a deletion, normal (diploid genotype) and amplification of the cancer genes. The six copies (representing 3 calibration sequences) were then arranged into a linear order as follows C-B-C-A-B-C. Intervening randomly-generated spacer sequences of ~600nt were then included between each calibration sequence, and additional flanking sequences were added to the 3' and 5' termini to form a final polynucleotide sequence. Final polynucleotide sequences that were selected from the *EGFR, MET, BRAF* and *FGFR1* genes that are recurrently found to be amplified or deleted in cancer see SEQ ID NOs: 2-4 and 14-25 were also built.

The polynucleotide sequence was then synthetized and hosted within a host pMA plasmid by a commercial vendor (ThermoFisher). Within the plasmid, the polynucleotide sequence was flanked by Sap1 restriction enzyme sites. The plasmid was transformed in *E. coli,* expanded in culture, and purified (using Plasmid Midi-Prep, Qiagen^{®}). The polynucleotide sequence was validated as correct using Sanger sequencing. The polynucleotide sequence was excised from the pMA plasmid by endonuclease digestion with a Sap1 enzyme (NEB, according to manufacturer's protocol). The excised polynucleotide sequence was separated and purified from the remaining plasmid using agarose gel electrophoresis. Purified polynucleotide sequence DNA was quantified using the BR dsDNA Qubit Assay on a Qubit 2.0 Fluorimeter (Life Technologies) and verified on the Agilent 2100 Bioanalyzer with an Agilent High Sensitivity DNA Kit (Agilent Technologies). The five individual polynucleotide sequences were then combined at equal concentrations (using Eppendorf^{®} Epmotion) to formulate a mixture.

Libraries were prepared with a KAPA Library Preparation Kit (Illumina platform KR0935-v2.14), in conjunction with SeqCap Adapter Kits (Roche-NimbleGen), as per the manufacturer's protocol. Target-enriched sequencing using a custom gene panel (SeqCap, Roche-NimbleGen) that targets -183 cancer genes was then performed. Enriched libraries were quantified on an Agilent 2100 Bioanalyzer, before performing paired-end sequencing on a HiSeq 2500, as above. Sequencing (Illumina^{®} HiSeq 2500) was performed per manufacturer's instructions to produce output library file (.FASTQ file).

To analyze the polynucleotide sequence (which was prepared as a ladder, as indicated above), the output library (.FASTQ) was aligned to the human genome reference assembly (hg38; GRCh38.p12, Dec 2013 assembly) using BWA (Li, 2013). Alignments that contain spacer and flanking sequences were omitted from further analysis. The alignment coverage of the polynucleotide was calibrated so that calibration sequence B (present at two copies) had ~30x fold coverage. The observed alignment coverage across the targeted gene exons was plotted relative to the known copy-number of the calibration sequences within the polynucleotide sequences. The resulting scatterplot indicated the quantitative accuracy for detecting gene deletions and amplifications using the target-enriched NGS protocol. To evaluate the quantitative accuracy for measuring copy-number variation, the deviation of the observed alignment coverage relative to the expected alignment coverage was measured by sum of residuals, and the deviation of the correlation and slope was measured from the value of one.

### Example 2: Use of polynucleotide sequence to calibrate a gene panel.

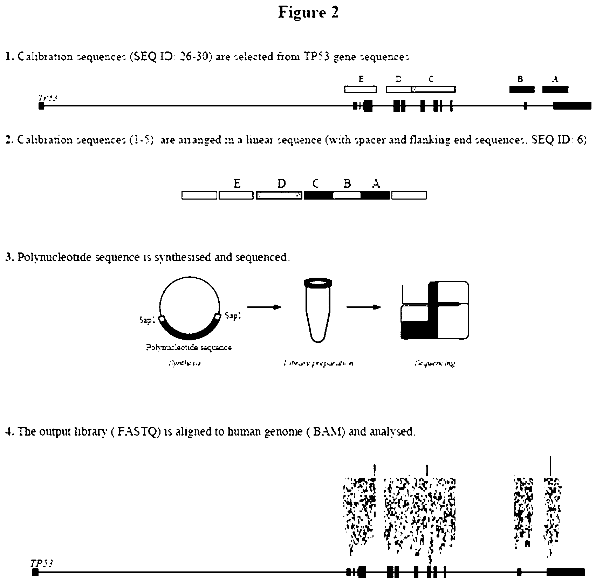

This example shows the design and synthesis of a polynucleotide sequence that comprises two or more calibration sequences that were selected from exon sequences from -97 genes causally associated with cancer. 5 calibration sequences (A-E) that overlapped with the TP53 gene exons (Figure 2; see SEQ ID NOs: 26-30) were selected. The calibration sequences (600nt, 640nt, 1549nt, 871nt, 1103nt) were arranged with intervening spacer sequences of ~600nt (SEQ ID NO: 42), whilst additional flanking sequences were added to the 3' and 5' termini to form a final polynucleotide sequence of ~7.6kb (see SEQ ID NO: 6).

The polynucleotide sequence was then synthesized and prepared as described above (see Example 1). Briefly, the polynucleotide was synthesized for library preparation, target enrichment and sequencing. The output library (.FASTQ) was then aligned to the human genome reference assembly (hg38, GRCh38.p12, Dec 2013 assembly) using BWA (Li, 2013).

The alignment coverage was calibrated across all calibration sequences to median alignment fold coverage equal to ~30-fold coverage. Differences in the alignment coverage across calibration sequences 1-5 were evaluated within the TP53 gene to measure differences in the technical performance of the NGS test.

### Example 3: Use of polynucleotide sequence to calibrate the sequencing of a microbe community.

This example shows the design and synthesis of a polynucleotide sequence comprising calibration sequences selected from prokaryotic genomes. Calibration sequences of -1,500nt that from the 16S rRNA gene were selected from three microbial genomes (*Escherichia coli K-12, Haemophilus influenzae Rd KW20, Pseudomonas aeruginosa PAO1*; Figure 3; see SEQ ID NOs: 31-33). Each calibration sequence was then repeated at one-, two-, and four-fold copy-numbers. The copies were then arranged with intervening spacer sequences and flanking terminal sequences to form the final polynucleotide sequence (see SEQ ID NO: 7).

The polynucleotide sequence was manufactured as described above (see Example 1), with the following exceptions. The polynuceltide sequence DNA fragments were prepared using the Nextera XT Sample Prep Kit (Illumina^{®}) according to the manufacturer's instructions. Prepared libraries were quantified on a Qubit Fluorometer (Life Technologies) and verified on an Agilent 2100 Bioanalyzer with an Agilent High Sensitivity DNA Kit (Agilent Technologies). Sequencing (Illumina^{®} HiSeq 2500) was performed per the manufacturer's instructions to produce an output library file (.FASTQ file) for analysis.

To analyze the ladder, the output library (.FASTQ) was aligned to the reference genome assemblies for the four microbial genomes using BWA (Li, 2013). Alignments that contain spacer and flanking sequences were omitted from further analysis. The observed alignment coverage across the microbial sequences is plotted relative to the known copy-number of each calibration sequence within the polynucleotide sequence to evaluate NGS library performance.

### Example 4: Use of a polynucleotide sequence to calibrate detection of genetic variants in the human genome.

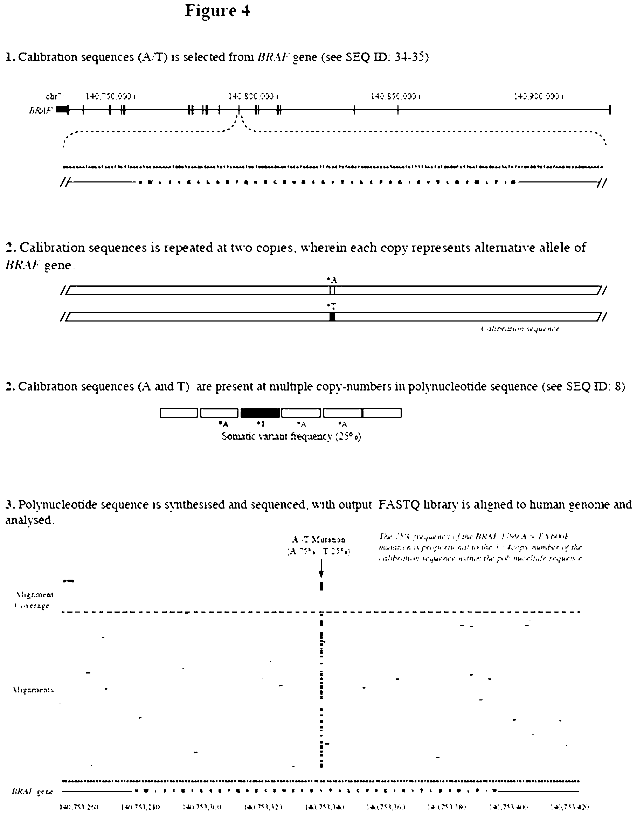

This example shows the design and use of polynucleotide sequences comprising calibration sequences that each represent different genetic variants at a single chromosomal region. A 1,100nt calibration sequence from the *BRAF* gene sequence (Figure 4) was selected. The calibration sequence was repeated in two copies; one copy harbored the reference T nucleotide, whilst one copy harbored the mutation A which results in the val600-to-glu (V600E) amino acid substitution associated with malignant melanomas (see SEQ ID NO: 34-35). The two calibration sequence copies were joined together, along with an intervening spacer sequence, to form a single polynucleotide sequence of 7.6 kb length (see SEQ ID NO:8).

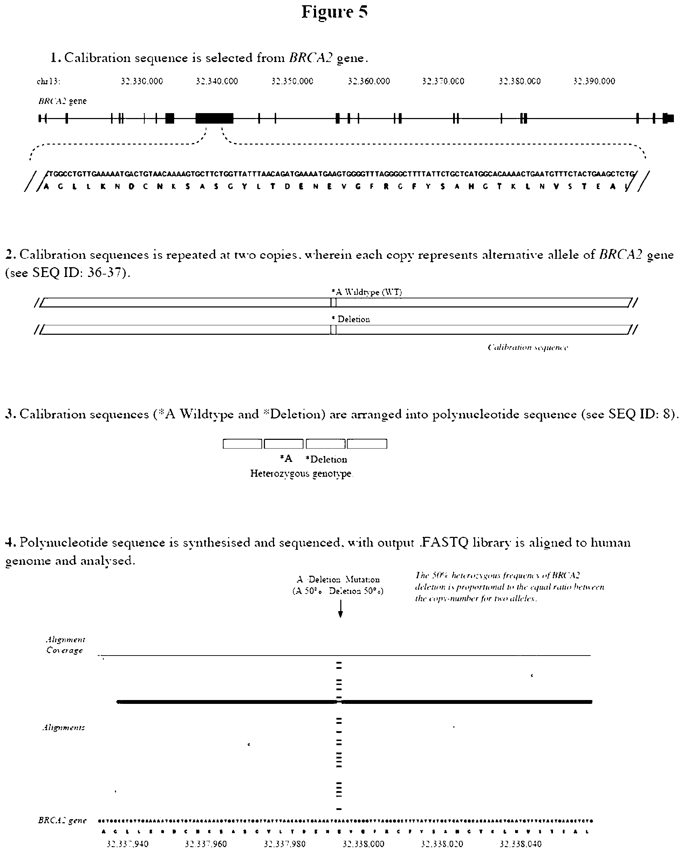

The inventors next selected a 2,200nt calibration sequence selected from the *BRCA2* gene sequence (Figure 5). The calibration sequence was repeated in two copies; one copy harbored the reference A nucleotide, whilst one copy harbored are small single nucleotide deletion (see SEQ ID NO: 36-37). The two calibration sequence copies were joined together, along with an intervening spacer sequence, to form a single polynucleotide sequence of 6.2kb length (see SEQ ID NO: 9).

The polynucleotide sequences were synthetized and prepared as described above (see Example 1). Briefly, the polynucleotide was synthesized for library preparation, target enrichment and sequencing. The output library (.FASTQ) was then aligned to the human genome reference assembly (hg38; GRCh38.p12, Dec 2013 assembly) using BWA (Li, 2013). To analyze the ladder, the output library (.FASTQ) was aligned to the human reference genome assembly (hg38) using BWA (Li, 2013). Alignments were calibrated to 30x coverage, and spacer and flanking sequences were omitted from further analysis.

VarScan2 (Koboldt et al., 2012) was used to identify the mutations in the BRAF and BCRA2 genes. The observed allele frequency of the mutations was compared to the expected ratio of the repeated copy-numbers to evaluate the accuracy of the NGS library. The frequency of BRAF V600E mutation is expected at 25% (to reflect somatic mutation) and BRCA2 expected at 50% (to reflect heterozygous mutation).

### Example 5: Use of a polynucleotide sequence to calibrate detection of genetic variants in circulating DNA.

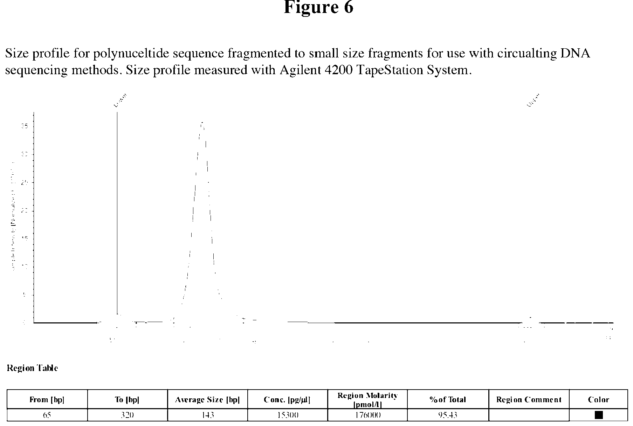

This example shows the design and use of polynucleotide sequences for use with circulating tumor DNA detection methods. The polynucleotide sequences comprising the BRAF V600E and BCRA2 deletion, as described above (see Example 4), were fragmented to be similar to smaller DNA fragments as typically observed during circulating DNA sequencing. Fragmentation of the polynucleotide sequences was performed using the Enzymatic DNA Fragmentation Kit according to manufacturer's instructions (New England Biolabs NEBNext Ultra II FS). The size of the fragmented polynucleotide sequences was measured using a Agilent 4300 TapStation System, showing the polynucleotide sequence was fragmented to a median size of 165nt (Figure 6), which is similar to the length typically observed in circulating DNA (Lapin et al., 2018).

The polynucleotide sequence was synthetized and prepared as described above (see Example 4). Briefly, the polynucleotide was synthesized for library preparation, target enrichment and sequencing. The output library (.FASTQ) was then aligned to the human genome reference assembly (hg38, GRCh38.p12, Dec 2013 assembly) using BWA (Li, 2013) and mutations identified using VarScan2 (Koboldt et al., 2012).

### Example 6: Use of a polynucleotide sequence to calibrate RNA sequencing.

This example shows the design and synthesis of RNA polynucleotide sequences to measure gene expression. Calibration sequences were selected that were 200nt in lengthencompassing exons from 3 different genes (BCR, ABL and ALK) whose expression is associated with cancer (see SEQ ID NOs: 38-40). The calibration sequences were represented at 1, 2 and 3 copies, and assembled, along with spacer and flanking sequences, into a single polynucleotide sequence (see SEQ ID NO: 10).

The polynucleotide sequence was then synthesized and hosted within a host pMA plasmid as described in Example 1 above. Within the plasmid, the polynucleotide sequence was proceeded 5' by a SP6 promoter (to enable *in vitro* transcription of the polynucleotide sequence) and followed 3' by a 60nt poly-adenine tract followed by a Not1 restriction enzyme site. The plasmid was linearized by Not1 digestion, and then transcribed into RNA (using the MEGAscript SP6 Transcription Kit, Thermo Fisher Scientific^{®}) as per manufacturer's instructions. The resulting RNA transcripts, comprising the polynucleotide sequence, were then combined at equal concentrations (using Eppendorf^{®} Epmotion) to formulate a mixture.

That was then subject to library preparation (using the Illumina^{®} KAPA RNA HyperPrep Kits). Libraries were quantified on an Agilent 2100 Bioanalyzer, before performing paired-end sequencing on a HiSeq 2500, as described above. Sequencing (Illumina^{®} HiSeq 2500) was performed per the manufacturer's instructions to produce output library file (.FASTQ file).

The output library (.FASTQ) was then aligned to the human genome reference assembly (hg38) using BWA (Li, 2013). The library was then analyzed using Kallisto (Bray et. al., 2016) to assign a transcript per million (TPM) values for each calibration sequence within the polynucleotide sequence. The measured TPM values were then plotted relative to the expected copy number of the calibration sequence within the polynucleotide sequence. Analysis of the polynucleotide sequence indicated the quantitative accuracy for measuring gene expression during the RNA sequencing experiment.

### Example 7: Illumina sequencing

This example shows the design of a polynucleotide adaptor sequence for use with next-generation sequencing (see Figure 11). 4 different calibration sequences (Y1-4) were selected, with each calibration sequence comprising an artificial 12nt sequence. These sequences were randomly generated sequences that did not include low-complexity or repeat sequences. The calibration sequences were then tested to ensure that they had minimal complementarity (less than 5 contiguous nucleotides) to other sequences within the adaptor (including P5, P7 sequences or complementary sequences). The calibration sequences also encoded different nucleotides at corresponding sites within the calibration. This was to ensure optimum cluster discrimination by having different nucleotides at each cycle of the index read. An example of the design of a pair of Y-adaptor sequences (termed Y1A and Y1B) was as follows:
For the first adaptor sequence, a calibration sequence with a 5' terminal thymine was designed (single example shown):
   CACGGTGTTCTT (SEQ ID NO: 48)
This was then assembled with a P5 primer binding sequence:
   AATGATACGGCGACCACCGA (SEQ ID NO:47)
and a sequence with complementarity to the (Y-adaptor) partner:
   -GA-TCTACACTCTTTCCCTACACGACGCTCTTCCGATCT (SEQ ID NO:49)
to form adaptor sequence (Y1A): 
CACGGTGTTCTT-AATGATACGGCGACCACC-GA-
GATCTACACTCTTTCCCTACACGACGCTCTTCCGATCT (SEQ ID NO:46)
For the second adaptor sequence, we designed a calibration sequence (single example shown):
   AGAACACCGTG (SEQ ID NO: 64)
This was then assembled with sequence with complementarity to the (Y-adaptor) partner and index sequence:
   GATCGGAAGAGCACACGTCTGAACTCCAGTCAC-ATCACG - (SEQ ID NO: 65)
and a P7 primer binding site:
   ATCTCGTATGCCGTCTTCTGCTTG (SEQ ID NO: 63).
The above sequences were then assembled to form adaptor sequence (Y1B).

AGAACACCGTG-AGATCGGAAGAGCA-CACGTCTGAACTCCAGTCAC-ATCACG-
ATCTCGTATGCCGTCTTCTGCTTG (SEQ ID NO: 62).

Using this approach, 8 sequences were designed that formed 4 pairs of Y-adaptors (Y1-Y4; see SEQ ID NOs: 46-61).

The above polynucleotide sequences were then synthesized (by commercial vendor; GeneArt). Each pair of adaptors was paired to a corresponding adaptor and hybridized by cooling from 68°C over 20 minutes. This enabled annealing between each pair of custom adaptor sequences to form Y-shaped adaptors (see Figure 11). The polynucleotide Y-adaptors were then combined in the following relative amounts (calibration sequence Y1 = 10%; Y2 = 20%; Y3 = 30% and Y4 = 40%) to formulate a mixture of adaptor sequences (Eppendorf Epmotion).

This adaptor sequence mixture was then used during preparation of libraries from a test sample using the Illumina TruSeq Library Preparation Kit (according to manufacturer's instructions, with exception for the synthesized custom adaptors). The test sample comprised a synthetic mock microbial community (Mock Community A). This comprised a mixture of synthetic DNA sequences (known as sequins) that mirrored the genome sequences of a range of microbial genomes (Hardwick et. al., 2019). The prepared library was validated using the Agilent 2100 Bioanalyzer then sequenced (Illumina^{®} NextSeq) per manufacturer's instructions.

The calibration sequences were then identified at the 5' start of sequenced reads in the output library (.FASTQ) using CutAdapt (Martin et. al., 2011; cutadapt 1.8.1 with Python 2.7.6), (with the modification of providing our own custom adaptor sequences (-g option) that comprised the calibration sequences). An example first pair read includes (the calibration sequence CACGGTGTTCTT (SEQ ID NO:48) is underlined):

@M00813:119:000000000-G4NKV:1:1101:16007:1887 1:N:0:CAGATC
CACGGTGTTCTTGCGCCAGCACGATGGCCAGGCCGAGCCCGACCTCGGCCGCCGCGATGAC
GATGACGAACAACGTGAGCACCTGCCCGCTGTGCAGCCGGTCGCGCAGCCAGACGTCGAAC
GCGACCAGGTTGAGGTTGACCGCGTTG (SEQ ID NO:219)
+
A1>11>1>A@DF1111A0AA0B00FAAAFB00FFAE/EEF/EEGGHFGGGGGGGGGGCHHCAFFHH
GEGGGHHGHHGHHHHHHHHHGGGGGHHHFHHG/CGGGGGGCCHGFFGGGGCCEHGGGGG
GGGEGFGGGGFFFFFF@@?@@@

The calibration sequences were identified and counted within the library: Y1 (10%) 135,312 reads, Y2 (20%) 302,356 reads, Y3 (30%) 581,931 reads and Y4 (40%) 1,324,455 reads. The observed fraction of calibration sequences were then plotted relative to the expected fraction of calibration sequences (see Figure 11). This plot indicates the quantitative accuracy, linearity and sensitivity of the NGS library, and enables an estimate of slope (3.847e+006 ± 943048) and correlation (R2-0.8927). Differences, or residuals, between the observed and expected fractional abundance of calibration sequence in the library indicate technical variation during sequencing.

### Example 8: Using adaptor sequence with Oxford Nanopore Sequencing

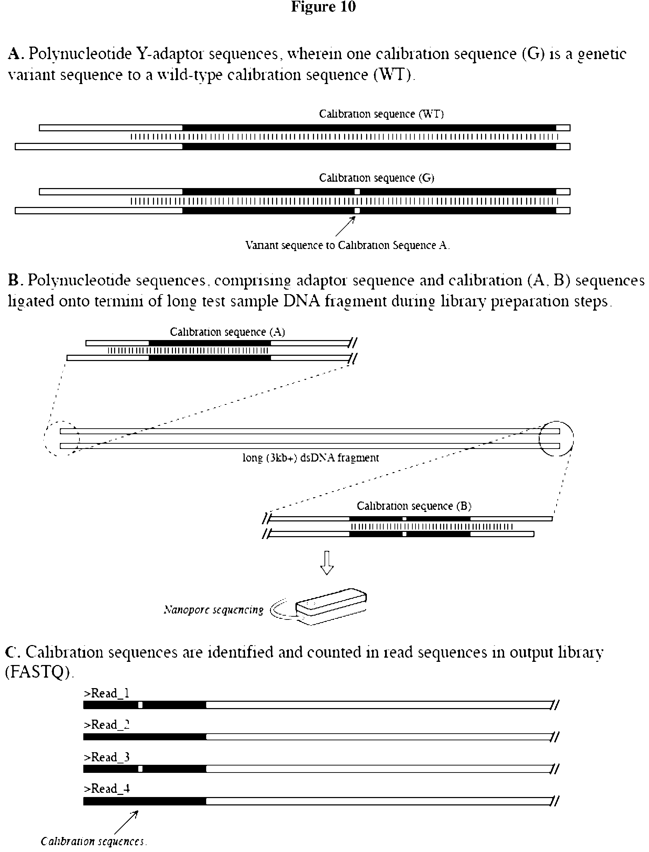

This example shows the design of a polynucleotide adaptor sequence for use with long-read sequencing with the Oxford Nanopore MinION^{™} instrument (see Figure 10). Four calibration sequences that were 24 nucleotides in length were selected. These sequences were randomly generated, and curated to ensure they did not share substantial homology with natural sequences or complementarity to other adaptor or primer sequences. An example of a calibration sequence being assembled into adaptor sequences is as follows (calibration sequence is underlined):
For the first adaptor sequence, a calibration sequence was designed (single example shown):
   TCCATGTAACACCATGAACACATC*T (SEQ ID NO: 79)
This was then assembled with a sequence with complementarity to the (Y-adaptor, e.g. Y5B) partner:
   TGACAGACAAGGGTAA (SEQ ID NO: 80)
The above sequences were than assembled to form first adaptor sequence (Y5A, calibration sequence underlined).
   TGACAGACAAGGGTAA- TCCATGTAACACCATGAACACATC*T (SEQ ID NO: 78)
For the second adaptor sequence, we designed a calibration sequence (single example shown):
   GATGTGTTCATGGTGTTACATGGA (SEQ ID NO: 91)
This was then assembled with a primer sequence with complementarity to the (Y-adaptor, i.e. Y5A) partner:
   TTACCCTTGTCTGTCA (SEQ ID NO: 92)
The above sequences were then assembled to form adaptor sequences.
   GATGTGTTCATGGTGTTACATGGA-TTACCCTTGTCTGTCA (SEQ ID NO: 90)

Using this approach, 4 pairs of adaptor sequences were designed (Y5-Y8; see SEQ ID NOs: 78, 81, 84, and 87).

The polynucleotide adaptor sequences were prepared as described above (see Example 7). The polynucleotide Y-adaptors were then combined in the following relative amounts (calibration sequence Y5 = 10%; Y6 = 20%; Y7 = 30% and Y8 = 40%) to formulate a mixture of adaptor sequences (Eppendorf Epmotion).

This adaptor sequence mixture was then used during preparation of libraries from a test sample using the Oxford Nanopore LSK108 kit (1D ligation) according to manufacturer's instructions (with the exception of the synthesized custom adaptors). The test sample comprised a synthetic mock microbial community (Mock Community A). This comprised a mixture of synthetic DNA sequences (known as sequins) that mirrored the genome sequences of a range of microbial genomes (Hardwick et. al., 2019). The library was sequenced on a MinION^{™} instrument (Oxford Nanopore Technologies). Base-calling was achieved using ONT Albacore Sequencing Pipeline Software (version 1.2.6).

The presence of calibration sequences within the ONT reads was determined. Here is an example of a raw read sequence (the Y8A calibration sequence GAAACCCATCAATCGCTTAACATC (SEQ ID NO: 88) is underlined (for brevity, quality scores have been removed):
@3d4f1939-915f-4eaa-8e2f-b8d390eac3d5 runid=29bac5664b009eaf1f91a4857ce89c11f0afdca0 read=61 ch=49 start_time=2019-11-12T05:37:03Z flow_cell_id=ABF060 protocol_group_id=FXFN061896 sample_id=FXFN061896

CATGACAGACAAGGGTAAGAAACCCATCAATCGCTTAACATCACAGAGCCCAGTTCTGTTGT
CTATGACTTGGCGAAGAAGAACAAGTCTGAACTGATATACTCTCGTGATTCCACTTTTAAGT

AGTACCATAAATAGAATTATACGGAAGTAGTAGGTTCAGTGTGCATTTAAATCTAACTTAGT
GGGAGTATTTCAAAACTTACCGTAACTATTAGCTCTTTAGAGTTAGAGCGGTGAATTGGCTT
TGCGATTATATTTACGAGGACTTCTGCGGTTACCTAGAACTTAACTACGTCGTGGTACGTGC
AAAAGTCATTTCTTTCAACGTGTGGTAAAAAAGTTACAAGCCACTAGACTAAAGACACGTAC
AAGTTGTGAGAAGTCAACTCATAAATTTATTAGGTTATAAGGACGTTGAATAGTTATTTGGT
TATTCAATGGTAGTACTACCTACCAACAATAGTACACCAGTTCAGGCTTGTTCTTCGCCAAGT
CATAGACAACAGAACTGGTGTGCTCATTGGGAATGATATACTCACTATAAGCAACTCAAGCT
CTTCCTGGCGATCCTGGAGTCATCATTGACTTCACTTAACGAGTCTGGTGTTGCAGCAAGTA
AATAAATATCATAAAATAATACTGCTCAGAAGCAATAGACACGACAGTTGGGCCAAATGCA
ATAATTCTTTCGTATATTATTCATATTTTCACGTGAGAAACTACCCTCTTTGCGATTATTCTGT
TGAGCTTTCAGATCTTCAACGTTGATGTCAGCTAACT (SEQ ID NO :220)
+

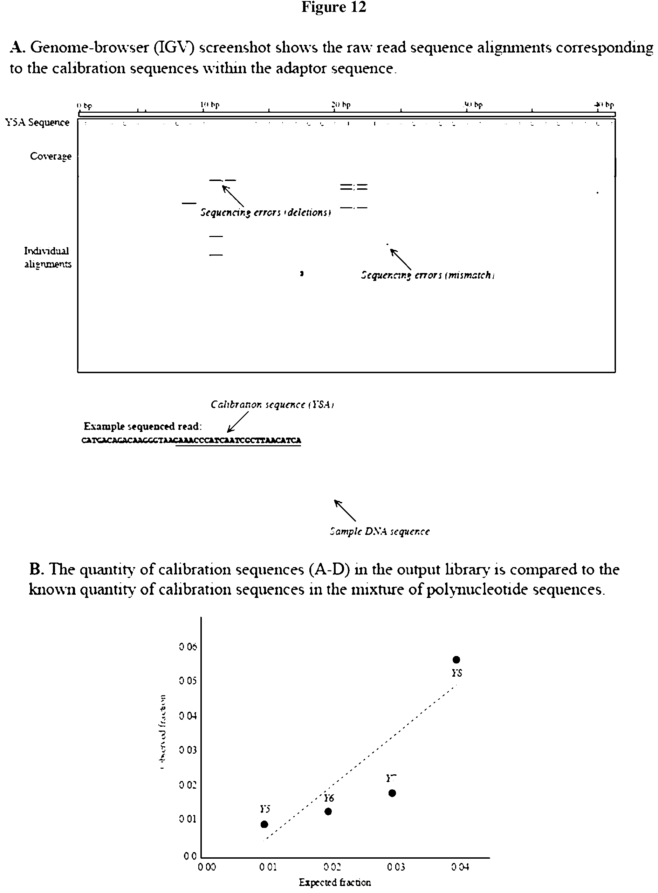

The expected fraction of calibration sequences in the library was plotted relative to the observed calibration sequences identified in the library (see Figure 12). This plot indicates the quantitative accuracy, linearity and sensitivity of the NGS library, and enables an estimate of slope and correlation. Differences between the observed number of reads, and the expected fractional abundance of the calibration sequence indicate technical variation during sequencing.

The calibration sequences within the library were then identified and counted: Y5 (10%) 539 reads, Y6 (20%) 758 reads, Y7 (30%) 2,119 reads and Y8 (40%) 3,201 reads. The observed fraction of calibration sequences were then plotted relative to the expected fraction of calibration sequences (see Figure 12). This plot indicates the quantitative accuracy, linearity and sensitivity of the NGS library, and enables an estimate of slope (9347 ± 1695) and correlation (R2 = 0.9383). Differences, or residuals, between the observed and expected fractional abundance of calibration sequence in the library indicate technical variation during sequencing.

### Example 9: Using adaptor sequences that comprise calibration sequences comprising natural genetic sequences.

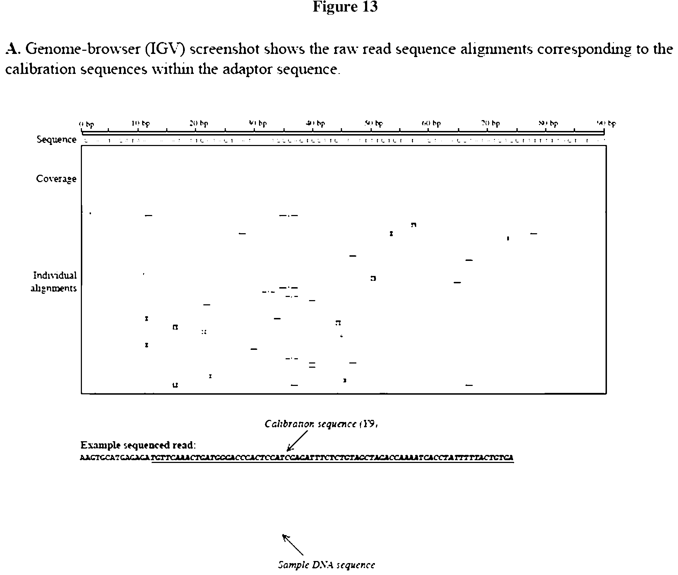

This example shows the design of a polynucleotide adaptor sequence that contains calibration sequences representing a natural genetic sequence of clinical importance (see, e.g., Figure 13). A calibration sequence was selected from the chr7 : 140753300 - 140753372 (+) coordinates in the human genome (GRCh38.p13). This sequence overlaps a recurrent mutation (known as V600E ) that occurs in the coding exon of the BRAF gene. The presence of this mutation informs the treatment of patients with cancer.

This calibration sequence (Y9) was then assembled within an adaptor sequence as follows (where the calibration sequence is underlined): 
CAAGTGCATAAGGAGATGTTCAAACTGATGGACCCACTCCATCGAGATTTCTCTGT
AGCTAGACCAAAATCACCTATTTTTACTGTGA*T (SEQ ID NO: 102)
In addition, and the Y-adaptor partner sequence was synthesized: 
TCACAGTAAAAATAGGTGATTTTGGTCTAGCTACAGAGAAATCTCGATGGAGTGGGT
CCCATCAGTTTGAACATCTCCTTATGCACTTG (SEQ ID NO: 105).
These adaptor sequences (SEQ ID NO:57 and 60) were prepared and used during library preparation of a DNA sample for Oxford Nanopore sequencing as described above (see Example 8). The presence of calibration sequences within the ONT reads was determined as described above (see Example 8). The following provides an example of a raw read sequence (the Y9 calibration sequence is underlined (for brevity, quality scores have been removed)):
   @e5f0f05f-d7da-46e8-92b4-1c2163cced5d runid=29bac5664b009eaf1f91a4857ce89c11f0afdca0 read=1731 ch=90 start_time=2019-11-12T06:01:42Z flow_cell_id=ABF060 protocol_group_id=FXFN061896 sample_id=FXFN061896 
AAGTGCATGAGAGATGTTCAAACTGATGGACCCACTCCATCGAGATTTCTCTGTAGCTAGA
CCAAAATCACCTATTTTACTGTGATTTTGTTAACCAGGCTAATATAAAAGTGATGGATCGA
AAGTACGCTCGTTCATTATTAGATCTTATCTTATTGAAATTTATTATATCGTTTTATTAAAAA
CTATATAATATTGCTATACGTTTTACAAATATCTGTAAATTAAACAAAGTATAATATAAAA
AATAGTATAAGAAATTAATGTCGTTAATATCGATGTATTTTATTACTCTTGACTTATACTAAG
TAATCTTTTAAAAAGATCAATTATCTACTAAAAACGTTCATAAGACTTCTTCATTATTTTTAT
ATACTATGGTTTGGGAGTATCTTTTTCGTTAAGTAGTATATTTTAGCATCGATGTTCCGTCCG
CGTTTTGCGAAAGGAAGGTTTCTTGGCGTGCTCGCTTAAATGAGTCGTATGACGAGTCTGTT
AAATGATATATTTATGCAGGTAAGCTACAATAAAAGATCAGAAAAATAGATTCATTTAACA
GACTCGTCATACGACTCATTTAGCGAGCACGCCAAATTTCCTTTCACAAAAACACAGGCAGA
TATCGACCATCCCCCAAACCATAATTATAGTATAATTTTAATCAGATAATTGATCTTTTAAAG
ATTACTTCTTAGTACTCAAGTCAAGGTAACAAAAAATCAACCTATTACCTGCGCATGTTATG
CTGTTTAATTCATAGATGACTAACAAAACATATAACTGTATTATATAGTTTTAGCAAAACG
ATATAACCAACTTCAATAAAAGACAAGATCTAATAATGAACGGGCGTGCTCGTCACTTTATA
TTAAAAATTGGTTAACAAATCACGGGCTGATCTCAGCTACAGAAAATCTCGTATAGTCCCTC
CTTTGAACATCATACGCAGTATACACATGGC (SEQ ID NO:m221)
+
Read sequences wherein the adaptor sequence (including calibration sequence Y9) were present 3' to the test sample DNA sequence were also identified:
   @b073934f-4d47-44fd-a6e7-98cbdbf6ab1b runid=29bac5664b009eaf1f91a4857ce89c11f0afdca0 read=2199 ch=66 start_time=2019-11-12T06:07:49Z flow_cell_id=ABF060 protocol_group_id=FXFN061896 sample_id=FXFN061896

AAGTGCATAAGGAGATGGACCCTCCCATCGAGATTTCTCTGTAGCTAACCAAAATCACCTA
GGCCAGTTAATTAAGAATTTACCGCTCTTCAGCAAAATCAAGAACGAGCTAGAAATCAGCC
GCCAGGGCTTTCTGAAAGCGTAGACTCAACCCGCTATGGTGGTCAGCGCATGCTATACCGTA
TAGCTCTCTGACGGTGGTTATCGATCTCCGGGCGACAGTCACCACTGCCCACCCTATATTTTC
CTCTTATTACCGGTCCCATCACTCGTAAAGGTCACCCAGCACAGAGTTGACCTCAGGTTTCCT
CATACCACCTCCAGCGGAAGGACGTATCCACAGGCCGAGGTACACAAGTGCATAAGGAGAT
GTTCAAACTGATGGGACCCACTCCATCGAGATTTCTCTGTAGCTAGACCAAAATCACCTATT
TTTACTGTGAGAAGCAATACGTA (SEQ ID NO: 222)
+

Given the sequence of the calibration sequence is known, the rate of errors in the calibration sequence in the reads indicates the sequencing accuracy of the library. 5,863 reads identified the BRAF V600E nucleotide correctly (WT = A), whilst 50 reads were incorrect, comprising an error rate of 0.0085%. This included a nucleotide A mismatch (3 reads), C mismatch (4 reads) and 43 single nucleotide deletions and provides an indication of the sequencing accuracy from the library. This illustrates how the calibration sequence that represents natural sequences of clinical importance can be used to evaluate the sequencing accuracy of the library and calibrate the sequencing process.

### Example 10: Using adaptor sequences that comprise calibration sequences that encode genetic variation.

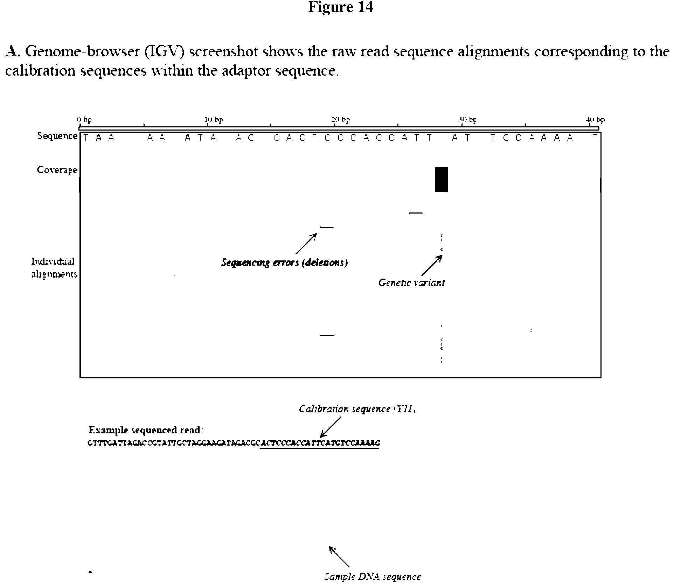

This example shows the design and use of a polynucleotide adaptor sequence that contains calibration sequences representing genetic variants (see, e.g., Figure 14). Two artificial 21 nucleotide sequences that are identical with the exception of a single variant nucleotide difference (the Y10 sequence encodes a G, whilst the mutant Y11 sequence encodes a C) were generated. The adaptor sequence is as follows (with calibration sequence underlined and variant nucleotide in bold):
Y10A: TAAGGAAGATAGACGCACTCCCACCATTGATGTCCAAAAG*T (SEQ ID NO: 108)
Y11A: TAAGGAAGATAGACGCACTCCCACCATTCATGTCCAAAAG*T (SEQ ID NO: 111)

These calibration sequences were assembled into adaptor sequences as described above (see above, SEQ ID NOs: 108 and 111). These adaptor sequences were prepared and used during library preparation of a DNA sample for Oxford Nanopore sequencing as described above (see Example 8). The presence of calibration sequences within the read sequences was determined as described above (see Example 8). Here is an example of a raw read sequence (the Y11 calibration sequence (that encodes the C variant) is underlined (for brevity, quality scores have been removed):
@55bae27d-da02-402d-90f4-276dd5abc2b6 runid=29bac5664b009eaf1f91a4857ce89c11f0afdca0 read=89 ch=70 start_time=2019-11-12T05:37:33Z flow_cell_id=ABF060 protocol_group_id=FXFN061896 sample_id=FXFN061896

GTTTGATTAGACCGTATTGCTAGGAAGATAGACGCACTCCCACCATTCATGTCCAAAAGTAG
GCCAGTTAATTAAGAGGTACCGCTCTTCAGCAAAATCAAGAACGAGCTAGAAATCAGCCGC
GAGGGCTTTCTAGGCGTAGACTTTCAACCCGCTATGTGGTAGGTCAGCACGCCCTATACCGT
ATAGCTCTGACGGTGGTTATCGATCTCCGGACGCGAGTCCAGTCATACATCACTCGTAAGGT
CACCCAGCACAGGGAGTTGACCCTCCGGGTTTCACATGCCTCAGCGGAAGGCATTATCCTG
AGCCAGTGCCACGCTACTAGACCTGCCGGTGAAAACCTAAGACTACAGTAACGCCAAGACC
CTCTCCCACAGGCTAGTTTGCCAGACCTCAAAAGTACGGCCCCCAGCAGTAAACTGCCGGAC
CACAAGGTGCTGGGTGTAGCGCTCTTGAAGATGGAGCCCTTCCGGCTTCTTCCACCACTTCG
CTTTGAACGACGTACTACTCCAAAGCACTATGACGCGGTCAGAATTCCCTTTGAGTCCGACC
AGACCTGATGGGAGGAAAAAATTGGGATATAAGCTCGAATCACCAAGTCTTGTGGGCTGAT
ACAGGGAGCCACAGGTCACCACCGCCGCCTCTAAACCCGGTAAACCCACTATATTCCATTCC
AAGTTGACTTGGGCTCAACGCGCAAATTACGGCCCAACATGCGCCGTATTAGAAGAAAGCA
CCAGCCAGCTGCACCAAGTACCAAAGTAAGCGTCTCGGAACAGCGTCACCATCCTCGGGTA
GAACTATGAGGCAAGTGCCATTAGCGACCAGTACTTCCAACCTTTCAGAGTCGAAGGGCCA
ATCGTTGCCTCATAAAGCGGACACCGCTTTTAAGACACCCGGCACTTCCATGGAAGGTGCTT
CGCTAGGTTTACCAGTCATACCCTGCGAACTACTGTGATAGCCGCGCTCCGACCAATGATAC
CGATCCTCGGGAGGGACCGAAACGATGCGAGTTTGGTACGTGAACGTAATGTCTTCCTGTTG
CGAGAATACAACGCAACGAAGGTCCTAACATACACAGCCTAGAGGCTGCAATGCAAAGCGA
GGTTACGTTGCCACGACGGCAATAAGTGTAACGGCTTTCAGGGCACAACTGGGGCGTGAGC
CGCGCTCTAGCCGACACACGCAGCGTCAGCCAGCCTATGCTAAATTACCCCTCGCTGAAGA
GCAGCTCATGGCGCGCCTAGGCCTAGCAATACGTAA (SEQ ID NO: 223)
+

The count of reads containing either of the two reference and variant sequences in the output library was measured. 6,895 reads with the G variant were identified, whilst 8403 reads had the C variant. This corresponds to a 45% allele frequency, with the difference to the expected heterozygous genotype frequency (i.e. 50%) indicating the scale of technical variation. This variant also provides a ground-truth example of a genetic variant that can be used to evaluate and optimise the detection of genetic variants.

### Example 11: Using adaptor sequences during PCR Amplification

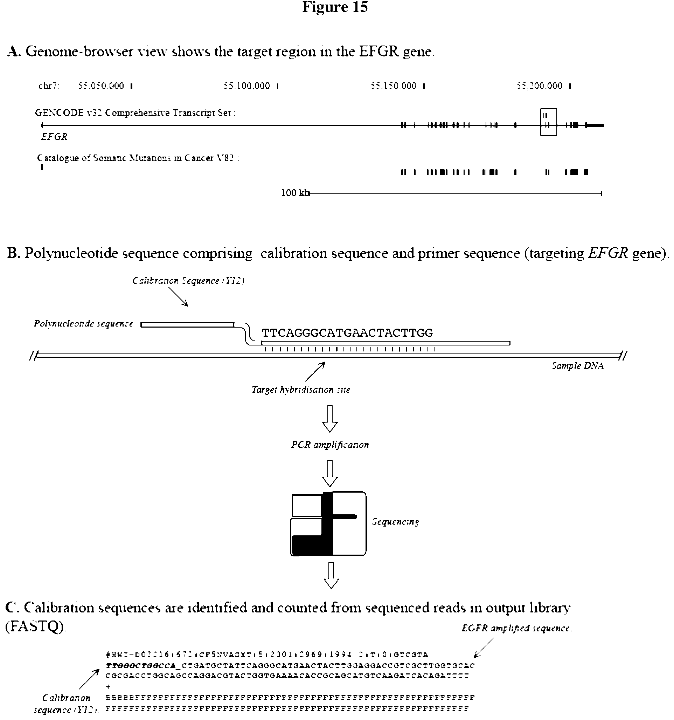

This example shows the design of a polynucleotide adaptor sequence for use during PCR amplification and amplicon sequencing (see Figure 15). A first calibration sequence from the chr7:55191816 - 55191828 (GRCh38.p12) coordinates was selected:

| | |
|---|---|
| Y12 (WT): | TTGGGCTGGCCA (SEQ ID NO: 131) |

This sequence was then modified to incoporate two mutations (A and G, corresponding to the EFGR L585 mutation), thereby generating a further two calibration sequences:

| | |
|---|---|
| Y13(A): | TTGGGCAGGCCA (SEQ ID NO:134) |
| Y14(G): | TTGGGCGGGCCA (SEQ ID NO: 137) |

Forward and reverse primer sequences flanking the chr7:55,191,755-55,191,888 region of the human genome (which encompassed the EGFR exon) were then selected.

| | |
|---|---|
| Fwd: | TTCAGGGCATGAACTACTTGG (SEQ ID NOs: 121, 124, and 127) |
| Rev: | TGCCTCCTTCTGCATGGTAT (SEQ ID NOs: 130, 133, and 136) |

The calibration sequence was then assembled, along with a random spacer sequence and the primer sequences, to form a polynucleotide adaptor sequence as follows:

| | |
|---|---|
| Y12F: | TTGGGCTGGCCA - CTGATGCTA -TTCAGGGCATGAACTACTTGG (SEQ ID NO: s120) |
| Y13F: | TTGGGCGGGCCA - GCATTGCAG - TTCAGGGCATGAACTACTTGG (SEQ ID NO: 123) |
| Y14F: | TTGGGCAGGCCA - TCACTATCC - TTCAGGGCATGAACTACTTGG (SEQ ID NO: 126) |

The above polynucleotide sequences (SEQ ID NOs: 120, 123, and 126) were then synthesized (by commercial vendor; GeneArt). The polynucleotide adaptors then combined in the following relative amounts (adaptor sequence Y12 = 89%; Y13 = 10%; Y14 = 1%) to formulate a mixture of adaptor sequences (Eppendorf Epmotion).

PCR was then performed (Taw DNA Polymerase; New England Bioscience) using the mixture of polynucleotide adaptor sequences and a human genome DNA sample (NA12878 genome, NIST). PCR was performed according to the following cycling parameters: Initial Denaturation; 95°C; 30 seconds; 30 Cycles 95°C; 45-68°C; 68°C for 15 seconds; 20 seconds; Final Extension 68°C for 5 minutes; Hold 4-10°C. The resulting amplicons were purified, and size selected using QIAquick PCR Purification Kit according to the manufacturer's instructions. The amplicon DNA product was then validated using an Agilent 2100 Bioanalyzer and used as input DNA for library preparation using the Illumina TruSeq Library Preparation Kit (according to the manufacturer's instructions). The library was then sequenced (Illumina^{®} NextSeq) per manufacturer's instructions.

The calibration sequences were then identified within the sequenced reads in the output library (.FASTQ) as described above (see Example 1). An example of a raw read sequence is shown below (with the Y12 calibration sequence underlined):
@HWI-D00116:342:CD1NVANXX:7:1101:5759:2792 1:N:0:CTTGTA

TTGGGCTGGCCACTGATGCTATTCAGGGCATGAACTACTTGGAGGACCGTCGCTTGG
TGCACCGCGACCTGGCAGCCAGGACGTACTGGTGAAAACACCGCAGCATGTCAAGA
TCACAGATTTT (SEQ ID NO: 224)
+
BBBBBFFFFFFFFFFFFFFFFFFFFFFFFFFFFFFFFFFFFFFFFFFFFFFFFFFFFFFFF
FFFFFFFFFFFFFFFFFFFFFFFFFFFFFFFFFFFFFFFFFFFFFFFFFFFFFFFF

Given the sequence of the calibration sequence is known, the rate of errors in the calibration sequence in the reads indicates the sequencing accuracy of the library. 95,616 reads (90.7%) identified the EGFR WT nucleotide sequence correctly, whilst 9,032 (8.5%) identified the A variant, whilst 743 reads (0.7%) identified the G variant. This indicates the sensitivity and the quantitive accuracy of the library for detecting the EFGR L585. This variant also provides a ground-truth example of a genetic variant that can be used to evaluate and optimise the detection of somatic genetic variants.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

All publications discussed and/or referenced herein are incorporated herein in their entirety.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each of the appended claims.

The invention may be described according to one or more of the following statements:
Statement 1. An isolated polynucleotide sequence comprising two or more calibration sequences, wherein the calibration sequences each independently represent a naturally occurring polynucleotide sequence, and wherein the calibration sequences are arranged consecutively within the polynucleotide sequence in an arrangement not found in any naturally occurring genome.
Statement 2. The polynucleotide sequence of Statement 1, wherein one or more of the calibration sequences are present in two or more copy numbers within the polynucleotide sequence.
Statement 3. The polynucleotide sequence of Statement 1 or 2, wherein a first calibration sequence represents an exon, intron, gene, allele, chromosome or genome sequence, and wherein a second calibration sequence differs from the first calibration sequence and independently represents a different exon, intron, gene, allele, chromosome or genome sequence.
Statement 4. The polynucleotide sequence of Statement 3, wherein the second calibration sequence represents a variant of the first calibration sequence.
Statement 5. The polynucleotide sequence of Statement 4, wherein the second calibration sequence is at least 90% identical to the first calibration sequence.
Statement 6. The polynucleotide sequence of Statement 5, wherein the variant comprises one or more SNPs, one or more insertions, one or more deletions, one or more microsatellites, multiple nucleotide polymorphisms, one or more duplications, one or more tandem repeat count variants, one or more inversions, one or more transitions, one or more transversions, and/or one or more translocations in comparison to the first calibration sequence.
Statement 7. The polynucleotide sequence of any one of Statements 3-6, wherein the first calibration sequence and/or the second calibration sequence is indicative of any one or more of cancer, inherited disease, a pathogen, drug-resistance, circulating tumor DNA, circulating fetal DNA, circulating maternal DNA, or other attribute of interest.
Statement 8. The polynucleotide sequence of any one of Statements 2-7, wherein the two or more copy numbers are selected to replicate naturally occurring frequencies of the naturally occurring sequences represented by the calibration sequences.
Statement 9. The polynucleotide sequence of Statement 8, wherein the two or more copy numbers are selected to replicate any one or more of homozygous, heterozygous and/or somatic allele frequencies of naturally occurring human polynucleotide sequences.
Statement 10. The polynucleotide sequence of Statement 8, wherein the two or more copy numbers are selected to replicate any one or more of: genome abundance, chromosome abundance, gene expression levels, isoform expression levels, variant allele frequency, homozygous genotype, heterozygous genotype, somatic mutation frequency, copy-number variation, gene amplifications, gene deletion, trisomy, chromosomal aneuploidy, microbe abundance, viral abundance, or varying mRNA expression levels.
Statement 11. The polynucleotide sequence of any one of Statements 1-10, wherein one calibration sequence represents a maternally derived polynucleotide and another calibration sequence represents a paternally derived polynucleotide.
Statement 12. The polynucleotide sequence of any one of Statements 1-11, wherein the naturally occurring polynucleotide sequence is derived from a human, prokaryote, bacteria, virus, phage or organelle genome.
Statement 13. The polynucleotide sequence of any one of Statements 1-12, wherein the calibration sequences are independently between 18 nucleotides and 20,000 nucleotides in length.
Statement 14. The polynucleotide sequence of Statement 13, wherein the calibration sequences are independently between 100 nucleotides and 2,000 nucleotides in length.
Statement 15. The polynucleotide sequence of any one of Statements 1-14, wherein two or more of the calibration sequences are separated by a spacer sequence.
Statement 16. The polynucleotide sequence of any one of Statements 1-15, comprising one or more flanking or terminal sequence located 5' and/or 3' of one or more of the calibration sequences.
Statement 17. The polynucleotide sequence of any one of Statements 1-14, wherein two or more of the calibration sequences are arranged contiguously within the polynucleotide sequence.
Statement 18. The polynucleotide sequence of any one of Statements 1-17, wherein at least two of the calibration sequences are of different nucleotide lengths.
Statement 19. The polynucleotide sequence of any one of Statements 1-18, which is a DNA or an RNA polynucleotide sequence.
Statement 20. A vector comprising a DNA sequence encoding the polynucleotide sequence of any one of Statements 1-19, operably linked to any one or more of the following: a 5' promoter; flanking (3' and 5') endonuclease sites; and a 3' poly-adenine repeat tract.
Statement 21. A method of making the polynucleotide sequence of any one of Statements 1-19, comprising providing and ligating the two or more calibration sequences; optionally further comprising providing and ligating flanking or terminal sequences and/or a spacer sequence, if present.
Statement 22. A composition comprising two or more polynucleotide sequences of any one of Statements 1-19.
Statement 23. A method of providing a polynucleotide for calibrating a circulating DNA detection method, the method comprising fragmenting the polynucleotide sequence of any one of Statements 1-19 to produce one or more polynucleotide fragments, which one or more polynucleotide fragments are suitable for calibrating a circulating DNA detection method.
Statement 24. A method of providing a polynucleotide for calibrating an RNA sequencing method, the method comprising performing *in-vitro* transcription of the polynucleotide sequence of any one of Statements 1-19 which polynucleotide sequence is a DNA polynucleotide sequence, to produce one or more RNA fragments, which one or more RNA fragments are suitable for calibrating an RNA sequencing method.
Statement 25. A method of calibrating a polynucleotide sequencing process, comprising:
   i) subjecting the polynucleotide sequence of any one of Statements 1-19 to a polynucleotide sequencing process under a given set of parameters;
   ii) recording the sequence of the calibration sequences as determined by the sequencing process performed in i);
   iii) comparing the sequence of the calibration sequences recorded in ii) with the known sequence of respective calibration sequences in the polynucleotide sequence of any one of Statements 1-19; and
   iv) subjecting a sample comprising a polynucleotide of interest to the same sequencing process performed in i) under the same set of parameters;
   wherein the accuracy of the sequence determination in iii) is used to calibrate the sequence determination in iv).
Statement 26. The method of Statement 25, wherein the quantitation process in iv) is performed in the absence of the polynucleotide sequence of any one of Statements 1-19.
Statement 27. A method of calibrating a polynucleotide quantitation process, comprising:
   i) subjecting the polynucleotide sequence of any one of Statements 1-19 to a polynucleotide quantitation process under a given set of parameters;
   ii) recording the quantity of the calibration sequences as determined by the quantitation process performed in i);
   iii) comparing the quantity of the calibration sequences recorded in ii) with the known quantities of respective calibration sequences in the polynucleotide sequence of any one of Statements 1-15; and
   iv) subjecting a sample comprising a polynucleotide of interest to the same quantitation process performed in i) under the same set of parameters;
   wherein the accuracy of the quantity determination in iii) is used to calibrate the quantity determination in iv).
Statement 28. The method of Statement 27, wherein the quantitation process in iv) is performed in the absence of the polynucleotide sequence of any one of Statements 1-19.
Statement 29. A method of calibrating a target DNA enrichment process, the method comprising:
   i) combining the polynucleotide sequence of any one of Statements 1-19 with an oligonucleotide of generally complementary sequence under conditions allowing hybridization;
   ii) enriching an oligonucleotide hybridized to a polynucleotide sequence;
   iii) sequencing the polynucleotide enriched in ii) under a given set of parameters;
   iv) comparing the polynucleotide sequence determined in iii) with the known sequence of the polynucleotide sequence; and
   v) sequencing the oligonucleotide enriched in ii) under the same set of parameters in iii);
   wherein the accuracy of the sequence determination in iv) is used to calibrate the sequence determination in v).
Statement 30. A method of calibrating a target DNA enrichment process, the method comprising:
   i) combining a known quantity of the polynucleotide sequence of any one of Statements 1-19 with an oligonucleotide of generally complementary sequence under conditions allowing hybridization;
   ii) enriching an oligonucleotide hybridized to a polynucleotide sequence;
   iii) quantifying the polynucleotide enriched in ii) under a given set of parameters;
   iv) comparing the polynucleotide quantity determined in iii) with the known quantity of the polynucleotide sequence in i); and
   v) quantifying the oligonucleotide enriched in ii) under the same set of parameters in iii);
   wherein the accuracy of the quantity determination in iv) is used to calibrate the quantity determination in v).
Statement 31. Use of the polynucleotide sequence of any one of Statements 1-19 to calibrate a polynucleotide sequencing and/or quantitation process.
Statement 32. A kit comprising a polynucleotide sequence of any one of Statements 1-19 and one or more nuclease enzymes capable of fragmenting the polynucleotide sequence.
Statement 33. An isolated polynucleotide adaptor sequence comprising a calibration sequence and a primer sequence.
Statement 34. The adaptor sequence of Statement 33, wherein the primer sequence is capable of binding to a complementary polynucleotide sequence in order to facilitate polynucleotide synthesis.
Statement 35. The adaptor sequence of Statement 34, wherein the primer sequence present in the adaptor sequence is complementary to a separate primer sequence that is capable of being added to a sample containing a polynucleotide to be replicated.
Statement 36. The adaptor sequence of Statement 35, wherein the calibration sequence is located 3' of the primer sequence present in the adaptor sequence.
Statement 37. The adaptor sequence of Statement 34, wherein the primer sequence present in the adaptor sequence is complementary to a portion of a target polynucleotide to be replicated.
Statement 38. The adaptor sequence of Statement 37, wherein the calibration sequence is located 5' of the primer sequence present in the adaptor sequence.
Statement 39. The adaptor sequence of any one of Statements 33-38 wherein the primer sequence is complementary to a universal primer sequence, or comprises a universal primer sequence.
Statement 40. The adaptor sequence of any one of Statements 33-39, further comprising any one or more of: a sequence complementary to a flow-cell DNA sequence; an index sequence that indicates the identity of a sample containing a polynucleotide to be replicated; and a unique molecular identifier (UMI) sequence that indicates the identity of an individual adaptor sequence.
Statement 41. The adaptor sequence of any one of Statements 33-40, wherein the calibration sequence represents a naturally occurring polynucleotide sequence.
Statement 42. The adaptor sequence of any one of Statements 33-41, wherein the calibration sequence represents an exon, intron, coding, noncoding, gene, chromosome, allele, genome, transcriptome, repeat, and/or variant genetic sequence.
Statement 43. The adaptor sequence of any one of Statements 33-42, wherein the calibration sequence represents a genetic variation sequence selected from any one or more of: nucleotide transitions, transversions, heterozygous mutations, homozygous mutations, somatic mutations, insertions, deletions, large-scale structural variations, inversions, translocations, tandem-duplications and copy-number variations.
Statement 44. The adaptor sequence of any one of Statements 33-43, wherein the calibration sequence represents a sequence that is indicative of any one or more of cancer, inherited disease, a pathogen, drug-resistance, circulating tumor DNA, circulating fetal DNA, circulating maternal DNA, or other attribute of interest.
Statement 45. The adaptor sequence of any one of Statements 33-44, wherein the calibration sequence is an artificial polynucleotide sequence having less than 100% sequence identity with sequences selected from natural genome or transcriptome sequences across its entire length.
Statement 46. The adaptor sequence of Statement 45, wherein the calibration sequence is produced by selecting a naturally occurring genetic sequence and modifying said naturally occurring genetic sequence by any one or more of reversing, shuffling, inserting, deleting or substituting one or more nucleotides to reduce the sequence identity with the naturally occurring sequence from 100%.
Statement 47. The adaptor sequence of any one of Statements 33-46, wherein the calibration sequence comprises any one or more of: a single nucleotide repeated greater than 3 times in succession; G or C nucleotides for at least 70% of the length of the calibration sequence; and A or T nucleotides for at least 70% of the length of the calibration sequence.
Statement 48. The adaptor sequence of any one of Statements 33-47, which is a single stranded or double stranded polynucleotide sequence.
Statement 49. The adaptor sequence of any one of Statements 33-48 which is a DNA or a RNA polynucleotide sequence.
Statement 50. The adaptor sequence of any one of Statements 33-49, which comprises two or more calibration sequences.
Statement 51. The adaptor sequence of any one of Statements 33-50, comprising a first calibration sequence and a second calibration sequence, wherein the second calibration sequence is the reverse complement of the first calibration sequence.
Statement 52. The adaptor sequence of any one of Statements 33-51, comprising a first and a second calibration sequence, wherein the second calibration sequence is a variant of the first calibration sequence.
Statement 53. The adaptor sequence of any one of Statements 33-52, comprising two or more copy numbers of one or more calibration sequences.
Statement 54. The adaptor sequence of any one of Statements 33-53, wherein the final 3' terminal nucleotide of the adaptor sequence and/or of the calibration sequence is thymine.
Statement 55. The adaptor sequence of any one of Statements 33-54, wherein the calibration sequence constitutes at least 10% of the length of the adaptor sequence.
Statement 56. The adaptor sequence of any one of Statements 33-55, wherein the calibration sequence is from 9 to 10,000 nucleotides in length.
Statement 57. A composition comprising one or more adaptor sequence of any one of Statements 33-56.
Statement 58. The composition of Statement 57, comprising at least two different adaptor sequences.
Statement 59. The composition of Statement 58, wherein the different adaptor sequences are present in the composition in different quantities.
Statement 60. The composition of Statement 59, wherein the ratios of the different quantities of different adaptor sequences are known, constant, integer and/or logarithmic ratios.
Statement 61. The composition of any one of Statements 57-60, comprising a first adaptor sequence having a first calibration sequence and a second adaptor sequence having a second calibration sequence, wherein the ratio of the quantity of the first adaptor sequence to the second adaptor sequence is 1:1 or 1:2.
Statement 62. The composition of any one of Statement 57-61, further comprising a liquid carrier.
Statement 63. A container comprising the composition of any one of Statements 57-62.
Statement 64. A vector comprising the adaptor sequence of any one of Statements 33-56.
Statement 65. A kit comprising one or more adaptor sequence of any one of Statements 33-56 or the composition of any one of Statements 57-62 and a ligase enzyme and/or a transposase enzyme and/or one or more separate oligonucleotide primer.
Statement 66. The kit of Statement 65, wherein the one or more separate oligonucleotide primer is complementary to or generally complementary to the primer sequence in a polynucleotide adaptor sequence present in the kit.
Statement 67. A method of making the adaptor sequence of any one of Statements 33-56, the method comprising excising the adaptor sequence from the vector of Statement 64 by endonuclease digestion, or by amplifying the adaptor sequence from the vector of Statement 64, or by transcribing the adaptor sequence comprised within the vector of Statement 64.
Statement 68. A method of preparing a sample comprising a target polynucleotide for a sequencing or quantification process, the method comprising a step of attaching the polynucleotide adaptor sequence of any one of Statements 33-56 to the 5' termini of the target polynucleotide.
Statement 69. The method of Statement 68, wherein the attaching step comprises forming a phosphodiester bond between the 3' hydroxyl group of the polynucleotide adaptor sequence and the 5' hydroxyl group of the target polynucleotide.
Statement 70. The method of Statement 68 or 69, wherein the attaching step comprises attaching the polynucleotide adaptor sequence of any one of Statements 33-56 to the 5' termini of the target polynucleotide by a process of ligation, transposition, or primer extension.
Statement 71. The method of Statement 70, which comprises the use of a ligase enzyme or a transposase enzyme.
Statement 72. A method of preparing a sample comprising a target polynucleotide for a sequencing or quantification process, the method comprising contacting the target polynucleotide with the polynucleotide adaptor sequence of Statement 37 or 38, or any one of Statements 39-56 when dependent on Statement 37 or 38, under conditions suitable to allow the primer sequence in the polynucleotide adaptor sequence to bind to the complementary portion of the target polynucleotide.
Statement 73. The method of any one of Statements 68-72, wherein the target polynucleotide is a DNA polynucleotide.
Statement 74. Use of the polynucleotide adaptor sequence of any one of Statements 33-56 or the composition of any one of Statements 56-62 to calibrate a polynucleotide sequencing and/or quantitation process.
Statement 75. A method of calibrating a polynucleotide sequencing process, comprising:
   i) adding an adaptor sequence according to any one of Statements 33-56 or a composition according to any one of Statements 57-62 to a sample comprising a target polynucleotide whose sequence is to be determined, under conditions allowing attachment of the polynucleotide adaptor sequence to the 5' and/or 3' termini of the target polynucleotide;
   ii) amplifying the target polynucleotide;
   iii) determining the sequence of the target polynucleotide;
   iv) determining the sequence of the calibration sequence in the adaptor sequence; and
   v) comparing the sequence determined in iv) to the original calibration sequence;
   wherein the accuracy of the sequence determination in iv) is used to calibrate the sequence determination in iii).
Statement 76. A method of calibrating a polynucleotide sequencing process, comprising:
   i) adding an adaptor sequence according to Statement 37 or 38 or any one of Statements 39-56 when dependent on Statement 37 or 38, or a composition comprising same, to a sample comprising a target polynucleotide whose sequence is to be determined, under conditions allowing the primer sequence in the polynucleotide adaptor sequence to bind to the complementary portion of the target polynucleotide;
   ii) amplifying the target polynucleotide;
   iii) determining the sequence of the target polynucleotide;
   iv) determining the sequence of the calibration sequence in the adaptor sequence; and
   v) comparing the sequence determined in iv) to the original calibration sequence;
   wherein the accuracy of the sequence determination in iv) is used to calibrate the sequence determination in iii).
Statement 77. A method of calibrating a polynucleotide quantitation process, comprising:
   i) adding a known amount of an adaptor sequence according to any one of Statements 33-56 or a composition according to any one of Statements 57-62 to a sample comprising a target polynucleotide whose quantity is to be determined, under conditions allowing attachment of the adaptor sequence to the 5' and/or 3' termini of the target polynucleotide;
   ii) amplifying the target polynucleotide;
   iii) determining the quantity of the target polynucleotide;
   iv) determining the quantity of the calibration sequence in the adaptor sequence; and
   v) comparing the quantity determined in iv) to the known amount of the calibration sequence in the adaptor sequence in i);
   wherein the accuracy of the quantity determination in iv) is used to calibrate the quantity determination in iii).
Statement 78. A method of calibrating a polynucleotide quantitation process, comprising:
   i) adding a known amount of an adaptor sequence according to Statement 37 or 38 or any one of Statements 39-56 when dependent on Statement 37 or 38, or a composition comprising same, to a sample comprising a target polynucleotide whose quantity is to be determined, under conditions allowing the primer sequence in the polynucleotide adaptor sequence to bind to the complementary portion of the target polynucleotide;
   ii) amplifying the target polynucleotide;
   iii) determining the quantity of the target polynucleotide;
   iv) determining the quantity of the calibration sequence in the adaptor sequence; and
   v) comparing the quantity determined in iv) to the known amount of the calibration sequence in the adaptor sequence in i);
   wherein the accuracy of the quantity determination in iv) is used to calibrate the quantity determination in iii).
Statement 79. The method of Statement 77 or 78, wherein at least one adaptor sequence added in i) comprises two or more calibration sequences and wherein iv) comprises determining the quantity of each calibration sequence.
Statement 80. The method of any one of Statements 75-79, which comprises any one or more of polymerase chain reaction amplification, DNA synthesis, DNA sequencing, RNA sequencing, reverse transcription and complementary cDNA sequencing.
Statement 81. The method of any one of Statements 75-80, wherein the method comprises any one or more of single-end, paired-end, short-read, long-read, sequence-by-synthesis, and nanopore-sequencing.
Statement 82. The method of any one of Statements 75-81, wherein the target polynucleotide and/or the adaptor sequence is a DNA or RNA polynucleotide.
Statement 83. The method of any one of Statements 75-81, wherein the methods comprise the identification of genetic sequences that cause or indicate the presence of, or predisposition to, a disease or infection.
Statement 84. The method of any one of Statements 75-83, wherein the methods comprise the diagnosis of a disease or condition or infection in a subject from whom the sample was taken.
Statement 85. The method of Statement 84, wherein the disease is cancer, and/or the infection is infection by a microorganism, and/or the subject is a human.

### References:

- Baker, S.C. et al. The External RNA Controls Consortium: a progress report. Nat Methods 2, 731-4 (2005).
- Bentley, D.R. et al. Accurate whole human genome sequencing using reversible terminator chemistry. Nature 456, 53-9 (2008).
- Bernstein, B.E. et al. Genomic maps and comparative analysis of histone modifications in human and mouse. Cell 120, 169-81 (2005).
- Bray, N., Pimentel, H., Melsted, P. et al. Near-optimal probabilistic RNA-seq quantification. Nat Biotechnol 34, 525-527 (2016).
- Clarke, J. et al. Continuous base identification for single-molecule nanopore DNA sequencing. Nat Nanotechnol 4, 265-70 (2009).
- Consortium, E. (2005). "Proposed methods for testing and selecting the ERCC external RNA controls." BMC Genomics 6: 150.
- Edwards, R.A. et al. Using pyrosequencing to shed light on deep mine microbial ecology. BMC Genomics 7, 57 (2006).
- Eid, J. et al. Real-time DNA sequencing from single polymerase molecules. Science 323, 133-8 (2009).
- Jiang, L. et al. Synthetic spike-in standards for RNA-seq experiments. Genome Res 21, 1543-51 (2011).
- Johnson, D.S., Mortazavi, A., Myers, R.M. & Wold, B. Genome-wide mapping of in vivo protein-DNA interactions. Science 316, 1497-502 (2007).
- Koboldt DC, Zhang Q, Larson DE, et al. VarScan 2: somatic mutation and copy number alteration discovery in cancer by exome sequencing. Genome Res. (2012);22(3):568-576.
- Lapin, Morten et al. "Fragment size and level of cell-free DNA provide prognostic information in patients with advanced pancreatic cancer." Journal of translational medicine vol. 16,1 300; 6 Nov. 2018.
- Li, H. and R. Durbin (2009). "Fast and accurate short read alignment with Burrows-Wheeler transform." Bioinformatics 25(14): 1754-1760.
- Li H. (2013) Aligning sequence reads, clone sequences and assembly contigs with BWA-MEM. arXiv:1303.3997v1 [q-bio.GN].
- Lieberman-Aiden, E. et al. Comprehensive mapping of long-range interactions reveals folding principles of the human genome. Science 326, 289-93 (2009).
- Mortazavi, A., Williams, B.A., McCue, K., Schaeffer, L. & Wold, B. Mapping and quantifying mammalian transcriptomes by RNA-Seq. Nat Methods 5, 621-8 (2008).
- Ronaghi, M., Uhlen, M. & Nyren, P. A sequencing method based on real-time pyrophosphate. Science 281, 363, 365 (1998).
- Rothberg, J.M. et al. An integrated semiconductor device enabling non-optical genome sequencing. Nature 475, 348-52 (2011).
- Zook, J.M. et al. Integrating human sequence data sets provides a resource of benchmark SNP and indel genotype calls. Nat Biotechnol 32, 246-51 (2014).

## Claims

1. A polynucleotide adaptor for use in sequencing, the polynucleotide adaptor comprising a primer sequence and a calibration sequence.

2. The polynucleotide adaptor of claim 1, wherein the primer sequence comprises one or more of:
a sequence complementary to a flow-cell DNA sequence;
an index sequence that indicates the identity of a sample containing a polynucleotide to be replicated; and
a unique molecular identifier (UMI) sequence that indicates the identity of an individual adaptor.

3. The polynucleotide adaptor of claim 1 or claim 2, wherein the primer sequence comprises a sequence of more than 3 contiguous thymine or uracil nucleotides and/or wherein the final 3' terminal nucleotide of the adaptor sequence and/or of the calibration sequence is thymine

4. The polynucloeitde adaptor of any one of claims 1-3, wherein the polynucleotide adaptor comprises one or more calibration sequences each of length between 8 and 10,000 nucleotides.

5. The polynucleotide sequencing adaptor of any one of claims 1-4, wherein the calibration sequence comprises a sequence of contiguous nucleotides of a naturally occurring genetic sequence that has been modified by one or more of reversing, shuffling, inserting, deleting or substituting one or more nucleotides of the contiguous nucleotides to reduce the sequence identity with the naturally occurring sequence from 100%.

6. The polynucleotide adaptor of any one of claims 1-5, wherein the calibration sequence comprises a sequence of contiguous nucleotides from an exon, intron, coding, noncoding, gene, chromosome, allele, genome, transcriptome, repeat, and/or variant thereof.

7. The polynucleotide adaptor of any one of claims 1-6, which comprises two or more calibration sequences, or wherein the adaptor comprises between 2 and 1,000 calibration sequences.

8. The adaptor sequence of any one of claims 1-7, wherein the calibration sequence is a first calibration sequence and the adaptor sequence comprises a second calibration sequence, wherein
a) the second calibration sequence is the reverse complement of the first calibration sequence, and/or
b) the second calibration sequence is a variant of the first calibration sequence.

9. The polynucleotide adaptor of any one of claims 1-7, wherein the calibration sequences constitute at least 10% and/or no more than 50% of the polynucleotide adaptor length.

10. The polynucleotide adaptor of any one of claims 1-9, wherein less than 5 contiguous nucleic acids of the calibration sequence are complementary to other sequences within the polynucleotide adaptor.

11. The adaptor sequence of any one of claims 1-10, wherein the calibration sequence is an artificial polynucleotide sequence having less than 100% sequence identity with sequences selected from natural genome or transcriptome sequences across its entire length.

12. A Y-shaped adaptor comprising:
a first polynucleotide adaptor that is a polynucleotide adaptor of any one of claims 1-11; and
a second polynucleotide adaptor that is a polynucleotide adaptor of any one of claims 1-11, wherein the 3' end of the first polynucleotide adaptor is complementary to the 5' end of the second polynucleotide adaptor, and the 5' end of the first polynucleotide adaptor is non-complementary to the 3' end of the second polynucleotide adaptor.

13. A method of preparing a sample comprising a target polynucleotide for a sequencing or quantification process, the method comprising
a) a step of attaching the polynucleotide adaptor of any one of claims 1-11, or the Y-shaped adaptor of claim 12, to one or both of the termini of the target polynucleotide; or
b) contacting the target polynucleotide with the polynucleotide adaptor of any one of claims 1-11, or the Y-shaped adaptor of claim 12, under conditions suitable to allow the primer sequence in the polynucleotide adaptor sequence to bind to a complementary portion of the target polynucleotide.

14. A method of calibrating a polynucleotide sequencing process, comprising:
i) adding the polynucleotide adaptor of any one of claims 1-11, or the Y-shaped adaptor of claim 12, to a sample comprising a target polynucleotide whose sequence is to be determined, under conditions allowing attachment of the polynucleotide adaptor to the 5' and/or 3' termini of the target polynucleotide; or under conditions allowing the primer sequence in the polynucleotide adaptor to bind to the complementary portion of the target polynucleotide;
ii) amplifying the target polynucleotide;
iii) determining the sequence of the target polynucleotide;
iv) determining the sequence of the calibration sequence in the adaptor; and
v) comparing the sequence determined in iv) to the original calibration sequence;
wherein the accuracy of the sequence determination in iv) is used to calibrate the sequence determination in iii).

15. A method of calibrating a polynucleotide quantitation process, comprising:
i) adding a known amount of the adaptor of any one of claims 1-11, or the Y-shaped adaptor of claim 12 to a sample comprising a target polynucleotide whose quantity is to be determined, under conditions allowing attachment of the adaptor sequence to the 5' and/or 3' termini of the target polynucleotide; or under conditions allowing the primer sequence in the polynucleotide adaptor to bind to the complementary portion of the target polynucleotide;
ii) amplifying the target polynucleotide;
iii) determining the quantity of the target polynucleotide;
iv) determining the quantity of the calibration sequence in the adaptor sequence; and
v) comparing the quantity determined in iv) to the known amount of the calibration sequence in the adaptor sequence in i);
wherein the accuracy of the quantity determination in iv) is used to calibrate the quantity determination in iii).
